# EUROPEAN PATENT SPECIFICATION

(11) **EP 2 970 449 B1**
(45) Date of publication and mention of the grant of the patent: **25.09.2019**
(21) Application number: 14718334.7
(22) Date of filing: 17.03.2014
(51) Int. Cl.: C07K 16/18, C07K 16/28, C07K 16/30, C07K 16/46

(54) **SINGLE CHAIN BINDING MOLECULES COMPRISING N-TERMINAL ABP**
EINKETTIGE BINDEMOLEKÜLE MIT N-TERMINALEN AB
MOLÉCULES DE LIAISON À CHAÎNE SIMPLE COMPRENANT L'ABP À L'EXTRÉMITÉ N-TERMINALE

(30) Priority: 15.03.2013 US 201361792073 P
(43) Date of publication of application: 20.01.2016
(73) Proprietor: Amgen Research (Munich) GmbH, 81477 München (DE)
(72) Inventor: KUFER, Peter, 81477 Munich (DE); LUTTERBUESE, Ralf, 81477 Munich (DE); HOFFMANN, Patrick, 81477 Munich (DE); NAHRWOLD, Elisabeth, 81477 Munich (DE)
(74) Representative: Schiweck Weinzierl Koch Patentanwälte Partnerschaft mbB
(86) International application number: PCT/EP2014/055223
(87) International publication number: WO 2014/140358

(56) References cited:
- EP-A1- 2 361 936
- WO-A1-2010/035012
- WO-A1-2013/128027
- WO-A2-2007/106120
- WO-A2-2008/119567
- WO-A2-2009/147248
- WO-A2-2010/037838
- WOLF E ET AL: "BiTEs: bispecific antibody constructs with unique anti-tumor activity", DRUG DISCOVERY TODAY, ELSEVIER, RAHWAY, NJ, US, vol. 10, no. 18, 15 September 2005 (2005-09-15), pages 1237-1244, XP027684780, ISSN: 1359-6446 [retrieved on 2005-09-15]
- KONTERMANN R E: "Strategies to extend plasma half-lives of recombinant antibodies", BIODRUGS: CLINICAL IMMUNOTHERAPEUTICS, BIOPHARMACEUTICALS AND GENE THERAPY, ADIS INTERNATIONAL, FR, vol. 23, no. 2, 1 April 2009 (2009-04-01), pages 93-109, XP008124089, ISSN: 1173-8804, DOI: 10.2165/00063030-200923020-00003
- LUCY J HOLT ET AL: "Anti-serum albumin domain antibodies for extending the half-lives of short lived drugs", PROTEIN ENGINEERING, DESIGN AND SELECTION, OXFORD JOURNAL, LONDON, GB, vol. 21, 1 January 2008 (2008-01-01), pages 283-288, XP007911765, ISSN: 1741-0126, DOI: 10.1093/PROTEIN/GZM067 [retrieved on 2008-04-02]

## Description

### Field of the Invention

The present invention provides a single chain bispecific antibody comprising at least three binding domains, wherein the first binding domain binds to serum albumin and is positioned at the N-terminus of the second binding domain, the second binding domain binds to a cell surface molecule on a target cell wherein the surface molecule is a tumor antigen, and the third binding domain binds to the T cell CD3 receptor complex. Moreover, the invention provides methods for the production of such binding molecule, a nucleic acid sequence encoding it, a vector comprising said nucleic acid sequence and a host cell expressing the binding molecule of the invention. Furthermore, the invention provides a pharmaceutical composition comprising a binding molecule of the invention, methods of treatment comprising the step of administering a binding molecule of the invention and the medical use of a binding molecule of the invention.

### Background of the invention

An increased half-life is generally useful in in vivo applications of immunoglobulins, especially antibodies and most especially antibody fragments of small size. Such fragments (Fvs, disulphide bonded Fvs, Fabs, scFvs, dAbs) are likely to suffer from rapid clearance from the body; thus, whilst they are able to reach most parts of the body rapidly, and are quick to produce and easier to handle, their in vivo applications may be limited by their brief persistence in vivo.

Bispecific molecules are known in the art, such as dimeric, bispecific antigen-binding molecule of the tandem diabody format being specific for CD3 and CD19 (see EP 2 361 936 A1). Further bispecific molecules such as BiTE® (Bispecific T-cell engager) antibodies are recombinant protein constructs made from two flexibly linked, single-chain antibodies (scFv). One scFv of BiTE antibodies is specific for a selected tumor-associated surface antigen on target cells; the second scFv is specific for CD3, a subunit of the T-cell receptor complex on T-cells. By their particular design and bivalent binding, BiTE antibodies are uniquely suited to transiently bind T-cells to target cells and, at the same time, potently activate the inherent cytolytic potential of T-cells against target cells. BiTE molecules are small proteins with a molecular weight below the renal cut off that could likely result in a shorter half-life, a feature that is shared by BiTEs with many other antibody formats (see e.g. WOLF E ET AL: "BiTEs: bispecific antibody constructs with unique anti-tumor activity", DRUG DISCOVERY TODAY, ELSEVIER, RAHWAY, NJ, US, vol. 10, no. 18, 15 September 2005 (2005-09-15), pages 1237-1244 or WO 2010/037838 A2. In fact, while it is one the one hand desirable to have a small binding molecule, since, for example, it can quickly reach its designated location in the body and can also reach most parts of the body, the "size" of such a binding molecule is not favorable as regards, in particular, renal clearance as they are quickly cleared from the blood (see e.g. KONTERMANN R E: "Strategies to extend plasma half-lives of recombinant antibodies", BIODRUGS: CLINICAL IMMUNOTHERAPEUTICS, BIOPHARMACEUTICALS AND GENE THERAPY, ADIS INTERNATIONAL, FR, vol. 23, no. 2, 1 April 2009 (2009-04-01), pages 93-109 or WO 2009/147248 A2. It may also happen that such a binding molecule is faster degraded, since it has, so to say, no good natural protection, unless it was stabilized before, for example, by amino acid changes. Thus, it is a balancing act between small size and stability/renal clearance. It is therefore desirable to have available a binding molecule, in particular, a bispecific binding molecule that is improved in its stability and/or retarded in its renal clearance, thereby having an overall increased serum half-life, but advantageously still so small that it can be manufactured in good yield and/or conveniently handled.

### Summary of the invention

The present invention provides a single chain bispecific antibody comprising at least three binding domains, wherein
(a) the first binding domain binds to serum albumin, comprises SEQ ID Nos. 2, 4 or 6, and is positioned at the N-terminus of the second binding domain;
(b) said second binding domain binds to a cell surface molecule on a target cell wherein the surface molecule is a tumor antigen; and
(c) the third binding domain binds to the T cell CD3 receptor complex,
wherein the three domains are consecutively on one polypeptide chain in the order from the N-terminus to the C-terminus
- the first binding domain;
- the second binding domain; and
- the third binding domain.

The invention also provides a single chain bispecific antibody comprising at least three binding domains comprised in one polypeptide chain, wherein
(a) the first domain is capable of binding to serum albumin and is positioned at the N-terminus of the second binding domain;
(b) said second domain is capable of binding to a cell surface molecule on a target cell; and
(c) the third domain is capable of binding to the T cell CD3 receptor complex,
wherein the percentage of expressible monomeric binding molecule [in relation to the total amount of binding molecule] depends on the order of the first and second binding domain in said binding molecule.

In one embodiment the bispecific antibody of the invention is characterized in a way that at least one of the binding domains, preferably the second and/or third binding domain, is an scFv or single domain antibody.

Also in one embodiment of the bispecific antibody of the invention the molecule comprises one or more further heterologous polypeptide.

A bispecific antibody of the invention may also comprise a His-tag as a heterologus poypeptide. It is preferred for the binding molecule of the invention that the His-tag is positioned at the C-terminus of the third binding domain.

The invention also provides a bispecific antibody, wherein
(a) the first binding domain is capable of binding to human and non-human primate serum albumin;
(b) the second binding domain is capable of binding to the cell surface molecule on a human and a non-human primate cell, and
(c) the third binding domain is capable of binding to the T cell CD3 receptor complex on a human and a non-human primate cell.

In one embodiment the bispecific antibody according to the invention is characterized that the first binding domain capable of binding to serum albumin is derived from a combinatorial library or an antibody binding domain.

In one embodiment of the bispecific antibody of the invention
- the second binding domain is binding to the cell surface molecule on a target cell with an affinity (KD) of ≤ 100 nM; and
- the third binding domain is binding to the T cell CD3 receptor complex with an affinity (KD) of ≤ 100nM.

In one embodiment of the bispecific antibody of the invention the binding molecule shows cytotoxic activity in an in vitro assay measuring the lysis of target cells by effector cells in the presence of 10% human serum albumin.

In a preferred embodiment of the bispecific antibody of the invention the molecule consists of a single polypeptide chain.

In one embodiment of the bispecific antibody of the invention
(a) the second binding domain comprises an antibody derived VL and VH chain; and/or
(b) the second binding domain comprises an antibody derived VL and VH chain.

In a preferred embodiment of the bispecific antibody of the invention the second binding domain capable of binding to the T cell CD3 receptor complex is capable of binding to an epitope of human and Callithrix jacchus, Saguinus oedipus or Saimiri sciureus CD3ε chain, wherein the epitope is part of an amino acid sequence comprised in the group consisting of SEQ ID NOs: 2, 4, 6, or 8 of WO 2008/119567 and comprises at least the amino acid sequence Gln-Asp-Gly-Asn-Glu (SEQ ID NO:37).

In a preferred embodiment of the invention the second binding domain is capable of binding CD33 or CEA.

In one embodiment the bispecific antibody of the invention is characterized by an amino acid sequence as depicted in SEQ ID NOs: 8, 12, 16, 20, 24, 26, 30, or 34.

An alternative embodiment of the invention provides a method for the production of bispecific antibody of the invention, the method comprising the step of:
- selecting for bispecific antibodies comprising a binding domain, which is capable of binding to a cell surface molecule on a target cell, comprising at the N-terminus a binding domain which is capable of binding to serum albumin.

In one embodiment the method of the invention further comprises the step of:
- adding to the antibody an additional binding domain, which is capable of binding to the T cell CD3 receptor complex.

With one embodiment the invention provides a nucleic acid molecule having a sequence encoding a binding molecule of the invention.

The invention also provides a vector comprising a nucleic acid sequence of the invention. The invention provides host cell transformed or transfected with the nucleic acid sequence of the invention or with the vector of the invention.

In one embodiment the invention provides a process for the production of a bispecific antibody of the invention or produced by a method of the invention, said process comprising culturing a host cell of the invention under conditions allowing the expression of the binding molecule of
the invention or produced by a method of the invention and recovering the produced binding molecule from the culture.

The invention also provides a pharmaceutical composition comprising a bispecific antibody of the invention, or produced according to the process of the invention.

According to one embodiment of the invention the bispecific antibody of the invention, or the bispecific antibody produced according to a method of the invention is for use in the prevention, treatment or amelioration of a disease selected from the group consisting of a proliferative disease, an inflammatory disease, an infectious disease and an autoimmune disease.

Also in one embodiment the invention provides a kit comprising a bispecific antibody of the invention or produced according to a method of the invention, a nucleic acid molecule of the invention, a vector of the invention, or a host cell of the invention.

### Brief description of the drawings

**Figure 1****:**
   Graph of an elution profile from the IMAC purification of a SA-21-CEAxCD3 bispecific antibody. 1: Capture, 2: Wash out unbound sample, 3: Pre-Elution 50 mM Imidazole, 4: Elution 500 mM Imidazole. Blue line: Optical absorption at 280 nm, Red line: Optical absorption at 254 nm.
**Figure 2****:**
   Graph of an SEC profile for a SA-21-CEAxCD3 bispecific antibody. Peak 1: Aggregates (Non binding molecules) in void volume. Peak 2: bispecific antibody dimer. Peak 3: bispecific antibody monomer. Peak 4: Low molecular weight contaminants and salts. Optical absorption at 280 nm, Red line: Optical absorption at 254 nm.
**Figure 3****:**
   Cytotoxic activity of CD33 bispecific antibodies as measured in an 18-hour ⁵¹chromium release assay in presence of 10% HSA. Effector cells: stimulated enriched human CD8 T cells. Target cells: Human CD33 transfected CHO cells. Effector to target cell (E:T) ratio: 10:1.

### Detailed description of the invention

### Definitions:

It must be noted that as used herein, the singular forms "a", "an", and "the", include plural references unless the context clearly indicates otherwise. Thus, for example, reference to "a reagent" includes one or more of such different reagents and reference to "the method" includes reference to equivalent steps and methods known to those of ordinary skill in the art that could be modified or substituted for the methods described herein.

Unless otherwise indicated, the term "at least" preceding a series of elements is to be understood to refer to every element in the series. Those skilled in the art will recognize, or be able to ascertain using no more than routine experimentation, many equivalents to the specific embodiments of the invention described herein. Such equivalents are intended to be encompassed by the present invention.

The term "and/or" wherever used herein includes the meaning of "and", "or" and "all or any other combination of the elements connected by said term".

The term "about" or "approximately" as used herein means within ±20%, preferably within ±15%, more preferably within ±10%, and most preferably within ±5% of a given value or range.

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step. When used herein the term "comprising" can be substituted with the term "containing" or "including" or sometimes when used herein with the term "having".

When used herein "consisting of" excludes any element, step, or ingredient not specified in the claim element. When used herein, "consisting essentially of" does not exclude materials or steps that do not materially affect the basic and novel characteristics of the claim.

In each instance herein any of the terms "comprising", "consisting essentially of" and "consisting of" may be replaced with either of the other two terms.

The term "binding molecule" or "antibody construct" in the sense of the present disclosure indicates any molecule capable of (specifically) binding to, interacting with or recognizing the target molecule. For the second binding domain such target molecule is a cell surface molecule on a target cell. For the third binding domain such target molecule is the T cell CD3 receptor complex.

The term "single chain binding molecule" defines in connection with the present invention that the disclosed binding molecules in its simplest form are monomers. The molecules or constructs may include proteinaceous parts and non-proteinaceous parts (e.g. chemical linkers or chemical cross-linking agents such as glutaraldehyde). Thus, the single chain binding molecule may comprising accordance with the invention non-peptide linkers preferably to link at least two of the binding domains. Also in line with this invention are herein defined peptide linkers.

A binding molecule, so to say, provides the scaffold for said one or more binding domains so that said binding domains can bind/interact with the surface molecule on a target cell and CD3 receptor complex on a T cell. For example, such a scaffold could be provided by protein A, in particular, the Z-domain thereof (affibodies), lmmE7 (immunity proteins), BPTI/APPI (Kunitz domains), Ras-binding protein AF-6 (PDZ-domains), charybdotoxin (Scorpion toxin), CTLA-4, Min-23 (knottins), lipocalins (anticalins), neokarzinostatin, a fibronectin domain, an ankyrin consensus repeat domain (Stumpp et al., Curr Opin Drug Discov Devel. 10(2), 153-159 (2007)) or thioredoxin (Skerra, Curr. Opin. Biotechnol. 18, 295-304 (2005); Hosse et al., Protein Sci. 15, 14-27 (2006); Nicaise et al., Protein Sci. 13, 1882-1891 (2004); Nygren and Uhlen, Curr. Opin. Struc. Biol. 7, 463-469 (1997)). A preferred binding molecule is an antibody, more preferably a bispecific antibody.

The definition of the term "antibody" includes embodiments such as monoclonal, chimeric, single chain, humanized and human antibodies, as well as antibody fragments, like, inter alia, Fab fragments. Antibody fragments or derivatives further comprise F(ab')₂, Fv, scFv fragments or single domain antibodies such as domain antibodies or nanobodies, single variable domain antibodies or immunoglobulin single variable domain comprising merely one variable domain, which might be VHH, VH or VL, that specifically bind an antigen or epitope independently of other V regions or domains; see, for example, Harlow and Lane (1988) and (1999), loc. cit.; Kontermann and Dübel, Antibody Engineering, Springer, 2nd ed. 2010 and Little, Recombinant Antibodies for Immunotherapy, Cambridge University Press 2009. Such immunoglobulin single variable domain encompasses not only an isolated antibody single variable domain polypeptide, but also larger polypeptides that comprise one or more monomers of an antibody single variable domain polypeptide sequence.

Monovalent antibody fragments in line with the above definition describe an embodiment of a binding domain in connection with this invention. Such monovalent antibody fragments bind to a specific antigen and can be also designated "antigen-binding domain", "antigen-binding fragment" or "antibody binding region".

In line with this definition all above described embodiments of the term antibody can be subsumed under the term "antibody construct". Said term also includes diabodies or Dual-Affinity Re-Targeting (DART) antibodies. Further envisaged are (bispecific) single chain diabodies, tandem diabodies (Tandab's), "minibodies" exemplified by a structure which is as follows: (VH-VL-CH3)₂, (scFv-CH3)₂ or (scFv-CH3-scFv)₂, "Fc DART" antibodies and "lgG DART" antibodies, and multibodies such as triabodies. Immunoglobulin single variable domains encompass not only an isolated antibody single variable domain polypeptide, but also larger polypeptides that comprise one or more monomers of an antibody single variable domain polypeptide sequence.

Various procedures are known in the art and may be used for the production of such antibody constructs (antibodies and/or fragments). Thus, (antibody) derivatives can be produced by peptidomimetics. Further, techniques described for the production of single chain antibodies (see, inter alia, US Patent 4,946,778, Kontermann and Dübel (2010), loc. cit. and Little(2009), loc. cit.) can be adapted to produce single chain antibodies specific for elected polypeptide(s). Also, transgenic animals may be used to express humanized antibodies specific for polypeptides and fusion proteins of this invention. For the preparation of monoclonal antibodies, any technique, providing antibodies produced by continuous cell line cultures can be used. Examples for such techniques include the hybridoma technique (Kohler and Milstein Nature 256 (1975), 495-497), the trioma technique, the human B-cell hybridoma technique (Kozbor, Immunology Today 4 (1983), 72) and the EBV-hybridoma technique to produce human monoclonal antibodies (Cole et al., Monoclonal Antibodies and Cancer Therapy, Alan R. Liss, Inc. (1985), 77-96). Surface plasmon resonance as employed in the BIAcore system can be used to increase the efficiency of phage antibodies which bind to an epitope of a target polypeptide, such as CD3 epsilon (Schier, Human Antibodies Hybridomas 7 (1996), 97-105; Malmborg, J. Immunol. Methods 183 (1995), 7-13). It is also envisaged in the context of this invention that the term "antibody" comprises antibody constructs, which may be expressed in a host as described herein below, e.g. antibody constructs which may be transfected and/or transduced via, inter alia, viruses or plasmid vectors.

Furthermore, the term "antibody" as employed in the invention also relates to derivatives or variants of the antibodies described herein which display the same specificity as the described antibodies.

The terms "antigen-binding domain", "antigen-binding fragment" and "antibody binding region" when used herein refer to a part of an antibody molecule that comprises amino acids responsible for the specific binding between antibody and antigen. The part of the antigen that is specifically recognized and bound by the antibody is referred to as the "epitope" as described herein above. As mentioned above, an antigen-binding domain may typically comprise an antibody light chain variable region (VL) and an antibody heavy chain variable region (VH); however, it does not have to comprise both. Fd fragments, for example, have two VH regions and often retain some antigen-binding function of the intact antigen-binding domain. Examples of antigen-binding fragments of an antibody include (1) a Fab fragment, a monovalent fragment having the VL, VH, CL and CH1 domains; (2) a F(ab')2 fragment, a bivalent fragment having two Fab fragments linked by a disulfide bridge at the hinge region; (3) a Fd fragment having the two VH and CH1 domains; (4) a Fv fragment having the VL and VH domains of a single arm of an antibody, (5) a dAb fragment (Ward et al., (1989) Nature 341 :544-546), which has a VH domain; (6) an isolated complementarity determining region (CDR), and (7) a single chain Fv (scFv). Although the two domains of the Fv fragment, VL and VH are coded for by separate genes, they can be joined, using recombinant methods, by a synthetic linker that enables them to be made as a single protein chain in which the VL and VH regions pair to form monovalent molecules (known as single chain Fv (scFv); see e.g., Huston et al. (1988) Proc. Natl. Acad. Sci USA 85:5879-5883). These antibody fragments are obtained using conventional techniques known to those with skill in the art, and the fragments are evaluated for function in the same manner as are intact antibodies.

The term "monoclonal antibody" as used herein refers to an antibody obtained from a population of substantially homogeneous antibodies, i.e., the individual antibodies comprising the population are identical except for possible naturally occurring mutations and/or post- translation modifications (e.g., isomerizations, amidations) that may be present in minor amounts. Monoclonal antibodies are highly specific, being directed against a single antigenic site. Furthermore, in contrast to conventional (polyclonal) antibody preparations which typically include different antibodies directed against different determinants (epitopes), each monoclonal antibody is directed against a single determinant on the antigen. In addition to their specificity, the monoclonal antibodies are advantageous in that they are synthesized by the hybridoma culture, uncontaminated by other immunoglobulins. The modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by the hybridoma method first described by Kohler et al., Nature, 256: 495 (1975), or may be made by recombinant DNA methods (see, e.g., U. S. Patent No. 4,816,567). The "monoclonal antibodies" may also be isolated from phage antibody libraries using the techniques described in Clackson et al., Nature, 352: 624-628 (1991) and Marks et al., J. Mol. Biol., 222: 581-597 (1991), for example.

The term "human antibody" includes antibodies having variable and constant regions corresponding substantially to human germline immunoglobulin sequences known in the art, including, for example, those described by Kabat *et al.* (See Kabat *et al.* (1991) loc. cit.). The human antibodies of the invention may include amino acid residues not encoded by human germline immunoglobulin sequences (e.g., mutations introduced by random or site-specific mutagenesis *in vitro* or by somatic mutation *in vivo*)*,* for example in the CDRs, and in particular, CDR3. The human antibody can have at least one, two, three, four, five, or more positions replaced with an amino acid residue that is not encoded by the human germline immunoglobulin sequence. It is emphasized that the definition of human antibodies as used herein also contemplates fully human antibodies, which include only non-artificially and/or genetically altered human sequences of antibodies as those can be derived by using technologies using systems such as the Xenomice.

Examples of "antibody variants" include humanized variants of non- human antibodies, "affinity matured" antibodies (see, e.g. Hawkins et al. J. Mol. Biol. 254, 889-896 (1992) and Lowman et al., Biochemistry 30, 10832- 10837 (1991)) and antibody mutants with altered effector function (s) (see, e.g., US Patent 5, 648, 260, Kontermann and Dübel (2010), loc. cit. and Little(2009), loc. cit.).

As used herein, *"in vitro* generated antibody" refers to an antibody where all or part of the variable region (e.g., at least one CDR) is generated in a non-immune cell selection (e.g., an in vitro phage display, protein chip or any other method in which candidate sequences can be tested for their ability to bind to an antigen). This term thus preferably excludes sequences generated by genomic rearrangement in an immune cell.

The pairing of a VH and VL together forms a single antigen-binding site. The CH domain most proximal to VH is designated as CH1. Each L chain is linked to an H chain by one covalent disulfide bond, while the two H chains are linked to each other by one or more disulfide bonds depending on the H chain isotype. The VH and VL domains consist of four regions of relatively conserved sequences called framework regions (FR1, FR2, FR3, and FR4), which form a scaffold for three regions of hypervariable sequences (complementarity determining regions, CDRs). The CDRs contain most of the residues responsible for specific interactions of the antibody with the antigen. CDRs are referred to as CDR 1, CDR2, and CDR3. Accordingly, CDR constituents on the heavy chain are referred to as H1, H2, and H3, while CDR constituents on the light chain are referred to as L1, L2, and L3.

The term "variable" refers to the portions of the immunoglobulin domains that exhibit variability in their sequence and that are involved in determining the specificity and binding affinity of a particular antibody (i.e., the "variable domain(s)"). Variability is not evenly distributed throughout the variable domains of antibodies; it is concentrated in sub-domains of each of the heavy and light chain variable regions. These sub-domains are called "hypervariable" regions or "complementarity determining regions" (CDRs). The more conserved (i.e., non-hypervariable) portions of the variable domains are called the "framework" regions (FRM). The variable domains of naturally occurring heavy and light chains each comprise four FRM regions, largely adopting a β-sheet configuration, connected by three hypervariable regions, which form loops connecting, and in some cases forming part of, the β-sheet structure. The hypervariable regions in each chain are held together in close proximity by the FRM and, with the hypervariable regions from the other chain, contribute to the formation of the antigen-binding site (see Kabat *et al.,* loc. cit.). The constant domains are not directly involved in antigen binding, but exhibit various effector functions, such as, for example, antibody-dependent, cell-mediated cytotoxicity and complement activation.

The terms "CDR", and its plural "CDRs", refer to a complementarity determining region (CDR) of which three make up the binding character of a light chain variable region (CDRL1, CDRL2 and CDRL3) and three make up the binding character of a heavy chain variable region (CDRH1, CDRH2 and CDRH3). CDRs contribute to the functional activity of an antibody molecule and are separated by amino acid sequences that comprise scaffolding or framework regions. The exact definitional CDR boundaries and lengths are subject to different classification and numbering systems. CDRs may therefore be referred to by Kabat, Chothia, contact or any other boundary definitions, including the numbering system described herein. Despite differing boundaries, each of these systems has some degree of overlap in what constitutes the so called "hypervariable regions" within the variable sequences. CDR definitions according to these systems may therefore differ in length and boundary areas with respect to the adjacent framework region. See for example Kabat, Chothia, and/or MacCallum (Kabat *et al.,* loc. cit.; Chothia et al., J. Mol. Biol, 1987, 196: 901; and MacCallum et al., J. Mol. Biol, 1996, 262: 732). However, the numbering in accordance with the so-called Kabat system is preferred. The CDR3 of the light chain and, particularly, CDR3 of the heavy chain may constitute the most important determinants in antigen binding within the light and heavy chain variable regions. In some antibody constructs, the heavy chain CDR3 appears to constitute the major area of contact between the antigen and the antibody. In vitro selection schemes in which CDR3 alone is varied can be used to vary the binding properties of an antibody or determine which residues contribute to the binding of an antigen.

"Consisting essentially of" means that the amino acid sequence can vary by about 1, 2, 3, 4, 5, 6, 7, 8, 9, 10, 11, 12, 13, 14, or 15% relative to the recited SEQ ID NO: sequence and still retain biological activity, as described herein.

In some embodiments, the bispecific antibodies of the invention are isolated proteins or substantially pure proteins. An "isolated" protein is unaccompanied by at least some of the material with which it is normally associated in its natural state, for example constituting at least about 5%, or at least about 50% by weight of the total protein in a given sample. It is understood that the isolated protein may constitute from 5 to 99.9% by weight of the total protein content depending on the circumstances. For example, the protein may be made at a significantly higher concentration through the use of an inducible promoter or high expression promoter, such that the protein is made at increased concentration levels. The definition includes the production of an antigen binding protein in a wide variety of organisms and/or host cells that are known in the art.

For amino acid sequences, sequence identity and/or similarity is determined by using standard techniques known in the art, including, but not limited to, the local sequence identity algorithm of Smith and Waterman, 1981, Adv. Appl. Math. 2:482, the sequence identity alignment algorithm of Needleman and Wunsch, 1970, J. Mol. Biol. 48:443, the search for similarity method of Pearson and Lipman, 1988, Proc. Nat. Acad. Sci. U.S.A. 85:2444, computerized implementations of these algorithms (GAP, BESTFIT, FASTA, and TFASTA in the Wisconsin Genetics Software Package, Genetics Computer Group, 575 Science Drive, Madison, Wis.), the Best Fit sequence program described by Devereux et al., 1984, Nucl. Acid Res. 12:387-395, preferably using the default settings, or by inspection. Preferably, percent identity is calculated by FastDB based upon the following parameters: mismatch penalty of 1; gap penalty of 1; gap size penalty of 0.33; and joining penalty of 30, "Current Methods in Sequence Comparison and Analysis," Macromolecule Sequencing and Synthesis, Selected Methods and Applications, pp 127-149 (1988), Alan R. Liss, Inc.

An example of a useful algorithm is PILEUP. PILEUP creates a multiple sequence alignment from a group of related sequences using progressive, pairwise alignments. It can also plot a tree showing the clustering relationships used to create the alignment. PILEUP uses a simplification of the progressive alignment method of Feng & Doolittle, 1987, J. Mol. Evol. 35:351-360; the method is similar to that described by Higgins and Sharp, 1989, CABIOS 5:151-153. Useful PILEUP parameters including a default gap weight of 3.00, a default gap length weight of 0.10, and weighted end gaps.

Another example of a useful algorithm is the BLAST algorithm, described in: Altschul et al., 1990, J. Mol. Biol. 215:403-410; Altschul et al., 1997, Nucleic Acids Res. 25:3389-3402; and Karin et al., 1993, Proc. Natl. Acad. Sci. U.S.A. 90:5873-5787. A particularly useful BLAST program is the WU-BLAST-2 program which was obtained from Altschul et al., 1996, Methods in Enzymology 266:460-480. WU-BLAST-2 uses several search parameters, most of which are set to the default values. The adjustable parameters are set with the following values: overlap span=1, overlap fraction=0.125, word threshold (T)=II. The HSP S and HSP S2 parameters are dynamic values and are established by the program itself depending upon the composition of the particular sequence and composition of the particular database against which the sequence of interest is being searched; however, the values may be adjusted to increase sensitivity.

An additional useful algorithm is gapped BLAST as reported by Altschul et al., 1993, Nucl. Acids Res. 25:3389-3402. Gapped BLAST uses BLOSUM-62 substitution scores; threshold T parameter set to 9; the two-hit method to trigger ungapped extensions, charges gap lengths of k a cost of 10+k; Xu set to 16, and Xg set to 40 for database search stage and to 67 for the output stage of the algorithms. Gapped alignments are triggered by a score corresponding to about 22 bits.

Generally, the amino acid homology, similarity, or identity between individual variant CDRs are at least 80% to the sequences depicted herein, and more typically with preferably increasing homologies or identities of at least 85%, 90%, 91%, 92%, 93%, 94%, 95%, 96%, 97%, 98%, 99%, and almost 100%. In a similar manner, "percent (%) nucleic acid sequence identity" with respect to the nucleic acid sequence of the binding proteins identified herein is defined as the percentage of nucleotide residues in a candidate sequence that are identical with the nucleotide residues in the coding sequence of the antigen binding protein. A specific method utilizes the BLASTN module of WU-BLAST-2 set to the default parameters, with overlap span and overlap fraction set to 1 and 0.125, respectively.

Generally, the nucleic acid sequence homology, similarity, or identity between the nucleotide sequences encoding individual variant CDRs and the nucleotide sequences depicted herein are at least 80%, and more typically with preferably increasing homologies or identities of at least 80%, 81%, 82%, 83%, 84%, 85%, 86%, 87%, 88%, 89%, 90%, 91%, 92%, 93%, 94%, 95%, 96%, 97%, 98%, or 99%, and almost 100%.

Thus, a "variant CDR" is one with the specified homology, similarity, or identity to the parent CDR of the invention, and shares biological function, including, but not limited to, at least 80%, 81%, 82%, 83%, 84%, 85%, 86%, 87%, 88%, 89%, 90%, 91%, 92%, 93%, 94%, 95%, 96%, 97%, 98%, or 99% of the specificity and/or activity of the parent CDR.

While the site or region for introducing an amino acid sequence variation is predetermined, the mutation *per se* need not be predetermined. For example, in order to optimize the performance of a mutation at a given site, random mutagenesis may be conducted at the target codon or region and the expressed antigen binding protein CDR variants screened for the optimal combination of desired activity. Techniques for making substitution mutations at predetermined sites in DNA having a known sequence are well known, for example, M13 primer mutagenesis and PCR mutagenesis. Screening of the mutants is done using assays of antigen binding protein activities, such as binding to an elected a cell surface molecule on a target cell.

The term "amino acid" or "amino acid residue" typically refers to an amino acid having its art recognized definition such as an amino acid selected from the group consisting of: alanine (Ala or A); arginine (Arg or R); asparagine (Asn or N); aspartic acid (Asp or D); cysteine (Cys or C); glutamine (Gin or Q); glutamic acid (Glu or E); glycine (Gly or G); histidine (His or H); isoleucine (He or I): leucine (Leu or L); lysine (Lys or K); methionine (Met or M); phenylalanine (Phe or F); pro line (Pro or P); serine (Ser or S); threonine (Thr or T); tryptophan (Trp or W); tyrosine (Tyr or Y); and valine (Val or V), although modified, synthetic, or rare amino acids may be used as desired. Generally, amino acids can be grouped as having a nonpolar side chain (e.g., Ala, Cys, He, Leu, Met, Phe, Pro, Val); a negatively charged side chain (e.g., Asp, Glu); a positively charged sidechain (e.g., Arg, His, Lys); or an uncharged polar side chain (e.g., Asn, Cys, Gin, Gly, His, Met, Phe, Ser, Thr, Trp, and Tyr).

The term "hypervariable region" (also known as "complementarity determining regions" or CDRs) when used herein refers to the amino acid residues of an antibody which are (usually three or four short regions of extreme sequence variability) within the V-region domain of an immunoglobulin which form the antigen-binding site and are the main determinants of antigen specificity. There are at least two methods for identifying the CDR residues: (1) An approach based on cross-species sequence variability (i. e., Kabat *et al.,* loc. cit.); and (2) An approach based on crystallographic studies of antigen-antibody complexes (Chothia, C. et al., J. Mol. Biol. 196: 901-917 (1987)). However, to the extent that two residue identification techniques define regions of overlapping, but not identical regions, they can be combined to define a hybrid CDR. However, in general, the CDR residues are preferably identified in accordance with the so-called Kabat (numbering) system.

The term "framework region" refers to the art-recognized portions of an antibody variable region that exist between the more divergent (i.e., hypervariable) CDRs. Such framework regions are typically referred to as frameworks 1 through 4 (FR1, FR2, FR3, and FR4) and provide a scaffold for the presentation of the six CDRs (three from the heavy chain and three from the light chain) in three dimensional space, to form an antigen-binding surface.

Typically, CDRs form a loop structure that can be classified as a canonical structure. The term "canonical structure" refers to the main chain conformation that is adopted by the antigen binding (CDR) loops. From comparative structural studies, it has been found that five of the six antigen binding loops have only a limited repertoire of available conformations. Each canonical structure can be characterized by the torsion angles of the polypeptide backbone. Correspondent loops between antibodies may, therefore, have very similar three dimensional structures, despite high amino acid sequence variability in most parts of the loops (Chothia and Lesk, J. Mol. Biol., 1987, 196: 901; Chothia et al., Nature, 1989, 342: 877; Martin and Thornton, J. Mol. Biol, 1996, 263: 800). Furthermore, there is a relationship between the adopted loop structure and the amino acid sequences surrounding it. The conformation of a particular canonical class is determined by the length of the loop and the amino acid residues residing at key positions within the loop, as well as within the conserved framework (i.e., outside of the loop). Assignment to a particular canonical class can therefore be made based on the presence of these key amino acid residues. The term "canonical structure" may also include considerations as to the linear sequence of the antibody, for example, as catalogued by Kabat (Kabat *et al.,* loc. cit.). The Kabat numbering scheme (system) is a widely adopted standard for numbering the amino acid residues of an antibody variable domain in a consistent manner and is the preferred scheme applied in the present invention as also mentioned elsewhere herein. Additional structural considerations can also be used to determine the canonical structure of an antibody. For example, those differences not fully reflected by Kabat numbering can be described by the numbering system of Chothia et al and/or revealed by other techniques, for example, crystallography and two or three-dimensional computational modeling. Accordingly, a given antibody sequence may be placed into a canonical class which allows for, among other things, identifying appropriate chassis sequences (e.g., based on a desire to include a variety of canonical structures in a library). Kabat numbering of antibody amino acid sequences and structural considerations as described by Chothia *et al.,* loc. cit. and their implications for construing canonical aspects of antibody structure, are described in the literature.

CDR3 is typically the greatest source of molecular diversity within the antibody-binding site. H3, for example, can be as short as two amino acid residues or greater than 26 amino acids. The subunit structures and three-dimensional configurations of different classes of immunoglobulins are well known in the art. For a review of the antibody structure, see Antibodies: A Laboratory Manual, Cold Spring Harbor Laboratory, eds. Harlow et al., 1988. One of skill in the art will recognize that each subunit structure, e.g., a CH, VH, CL, VL, CDR, FR structure, comprises active fragments, e.g., the portion of the VH, VL, or CDR subunit the binds to the antigen, i.e., the antigen-binding fragment, or, e.g., the portion of the CH subunit that binds to and/or activates, e.g., an Fc receptor and/or complement. The CDRs typically refer to the Kabat CDRs, as described in Sequences of Proteins of immunological Interest, US Department of Health and Human Services (1991), eds. Kabat *et al.* Another standard for characterizing the antigen binding site is to refer to the hypervariable loops as described by Chothia. See, e.g., Chothia, et al. (1987; J. Mol. Biol. 227:799-817); and Tomlinson et al. (1995) EMBO J. 14: 4628-4638. Still another standard is the AbM definition used by Oxford Molecular's AbM antibody modeling software. See, generally, e.g., Protein Sequence and Structure Analysis of Antibody Variable Domains. In: Antibody Engineering Lab Manual (Ed.: Duebel, S. and Kontermann, R., Springer-Verlag, Heidelberg). Embodiments described with respect to Kabat CDRs can alternatively be implemented using similar described relationships with respect to Chothia hypervariable loops or to the AbM-defined loops.

The sequence of antibody genes after assembly and somatic mutation is highly varied, and these varied genes are estimated to encode 10¹⁰ different antibody molecules (Immunoglobulin Genes, 2nd ed., eds. Jonio et al., Academic Press, San Diego, CA, 1995). Accordingly, the immune system provides a repertoire of immunoglobulins. The term "repertoire" refers to at least one nucleotide sequence derived wholly or partially from at least one sequence encoding at least one immunoglobulin. The sequence(s) may be generated by rearrangement in vivo of the V, D, and J segments of heavy chains, and the V and J segments of light chains. Alternatively, the sequence(s) can be generated from a cell in response to which rearrangement occurs, e.g., in vitro stimulation. Alternatively, part or all of the sequence(s) may be obtained by DNA splicing, nucleotide synthesis, mutagenesis, and other methods, see, e.g., U.S. Patent 5,565,332. A repertoire may include only one sequence or may include a plurality of sequences, including ones in a genetically diverse collection.

The term "binding molecule" or "antibody construct" in the sense of the present disclosure indicates any molecule capable of (specifically) binding to, interacting with or recognizing the target molecules a cell surface molecule on a target cell and CD3. Such molecules or constructs may include proteinaceous parts and non-proteinaceous parts (e.g. chemical linkers or chemical cross-linking agents such as glutaraldehyde).

In the event that a linker is used, this linker is preferably of a length and sequence sufficient to ensure that each of the first, second and third domains can, independently from one another, retain their differential binding specificities. Most preferably and as documented in the appended examples, the binding molecule of the invention is a "bispecific single chain binding molecule", more preferably a bispecific single chain Fv (scFv). Bispecific single chain molecules are known in the art and are described in WO 99/54440, Mack, J. Immunol. (1997), 158, 3965-3970, Mack, PNAS, (1995), 92, 7021-7025, Kufer, Cancer Immunol. Immunother., (1997), 45, 193-197, Löffler, Blood, (2000), 95, 6, 2098-2103, Bruhl, Immunol., (2001), 166, 2420-2426, Kipriyanov, J. Mol. Biol., (1999), 293, 41-56.

The said variable domains comprised in the herein described bispecific antibodies may be connected by additional linker sequences. The term "peptide linker" defines in accordance with the present invention an amino acid sequence by which the amino acid sequences of the first domain, the second domain and the third domain of the bispecific antibody of the invention are linked with each other. An essential technical feature of such peptide linker is that said peptide linker does not comprise any polymerization activity. Among the suitable peptide linkers are those described in U.S. Patents 4,751,180 and 4,935,233 or WO 88/09344. A preferred embodiment of a peptide linker is characterized by the amino acid sequence Gly-Gly-Gly-Gly-Ser, i.e. Gly₄Ser, or polymers thereof, i.e. (Gly₄Ser)x, where x is an integer 1 or greater. The characteristics of said peptide linker, which comprise the absence of the promotion of secondary structures are known in the art and described e.g. in Dall'Acqua et al. (Biochem. (1998) 37, 9266-9273), Cheadle et al. (Mol Immunol (1992) 29, 21-30) and Raag and Whitlow (FASEB (1995) 9(1), 73-80). Peptide linkers which also do not promote any secondary structures are preferred. The linkage of said domains to each other can be provided by, e.g. genetic engineering, as described in the examples. Methods for preparing fused and operatively linked bispecific single chain constructs and expressing them in mammalian cells or bacteria are well-known in the art (e.g. WO 99/54440 or Sambrook et al., Molecular Cloning: A Laboratory Manual, Cold Spring Harbor Laboratory Press, Cold Spring Harbor, New York, 2001).

For peptide linkers, which connect the binding domains in the bispecific antibody of the invention peptide linkers are preferred which comprise only a few number of amino acid residues, e.g. 12 amino acid residues or less. Thus, peptide linker of 12, 11, 10, 9, 8, 7, 6 or 5 amino acid residues are preferred. An envisaged peptide linker with less than 5 amino acids comprises 4, 3, 2 or one amino acid(s) wherein Gly-rich linkers are preferred. A particularly preferred "single" amino acid in context of said "peptide linker" is Gly. Accordingly, said peptide linker may consist of the single amino acid Gly.

The term "multispecific" as used herein refers to a bispecific antibody of the invention which comprises at least is capable of binding to at least one further antigen or target.

It is also envisaged that the bispecific antibody of the invention has, apart from addition to the specificity for serum albumin and in addition to its function to bind to a cell surface molecule on a target cell and CD3, a further function. In this format, the antibody construct is a multifunctional antibody construct by targeting cells through binding to a cell surface molecule on a target cell, mediating cytotoxic T cell activity through CD3 binding and providing a further function such as a fully functional Fc constant domain mediating antibody-dependent cellular cytotoxicity through recruitment of effector cells like NK cells, a label (fluorescent etc.), a therapeutic agent such as, e.g. a toxin or radionuclide, and/or means to enhance serum half-life, etc.

The term "binding domain" characterizes in connection with the present invention a domain which is capable of specifically binding to / interacting with a given target epitope or a given target site on the target cell surface molecule on a target cell and CD3.

Binding domains can be derived from a binding domain donor such as for example an antibody. It is envisaged that a binding domain of the present invention comprises at least said part of any of the aforementioned binding domains that is required for binding to/interacting with a given target epitope or a given target site on the cell surface molecule on a target cell and CD3.

It is envisaged that the binding domain of the aforementioned binding domain donors is characterized by that part of these donors that is responsible for binding the respective target, i.e. when that part is removed from the binding domain donor, said donor loses its binding capability. "Loses" means a reduction of at least 50% of the binding capability when compared with the binding donor. Methods to map these binding sites are well known in the art - it is therefore within the standard knowledge of the skilled person to locate/map the binding site of a binding domain donor and, thereby, to "derive" said binding domain from the respective binding domain donors.

The term "epitope" refers to a site on an antigen to which a binding domain, such as an antibody or immunoglobulin or derivative or fragment of an antibody or of an immunoglobulin, specifically binds. An "epitope" is antigenic and thus the term epitope is sometimes also referred to herein as "antigenic structure" or "antigenic determinant". Thus, the binding domain is an "antigen-interaction-site". Said binding/interaction is also understood to define a "specific recognition". In one example, said binding domain which (specifically) binds to / interacts with a given target epitope or a given target site on a cell surface molecule on a target cell and CD3 is an antibody or immunoglobulin, and said binding domain is a VH and/or VL region of an antibody or of an immunoglobulin.

"Epitopes" can be formed both by contiguous amino acids or non-contiguous amino acids juxtaposed by tertiary folding of a protein. A "linear epitope" is an epitope where an amino acid primary sequence comprises the recognized epitope. A linear epitope typically includes at least 3 or at least 4, and more usually, at least 5 or at least 6 or at least 7, for example, about 8 to about 10 amino acids in a unique sequence.

A "conformational epitope", in contrast to a linear epitope, is an epitope wherein the primary sequence of the amino acids comprising the epitope is not the sole defining component of the epitope recognized (e.g., an epitope wherein the primary sequence of amino acids is not necessarily recognized by the binding domain). Typically a conformational epitope comprises an increased number of amino acids relative to a linear epitope. With regard to recognition of conformational epitopes, the binding domain recognizes a three-dimensional structure of the antigen, preferably a peptide or protein or fragment thereof (in the context of the present invention, the antigen for one of the binding domains is comprised within a cell surface molecule on a target cell). For example, when a protein molecule folds to form a three-dimensional structure, certain amino acids and/or the polypeptide backbone forming the conformational epitope become juxtaposed enabling the antibody to recognize the epitope. Methods of determining the conformation of epitopes include, but are not limited to, x-ray crystallography, two-dimensional nuclear magnetic resonance (2D-NMR) spectroscopy and site-directed spin labelling and electron paramagnetic resonance (EPR) spectroscopy. Moreover, the provided examples describe a further method to characterize a given binding domain by way of binning, which includes a test whether the given binding domain binds to one or more epitope cluster(s) of a given protein, in particular a cell surface molecule on a target cell.

As used herein, the term "epitope cluster" denotes the entirety of epitopes lying in a defined contiguous stretch of an antigen. An epitope cluster can comprise one, two or more epitopes. The concept of epitope cluster is also used in the characterization of the features of the bispecific antibodies of the invention.

The terms "(capable of) binding to", "specifically recognizing", "directed to" and "reacting with" mean in accordance with this invention that a binding domain is capable of specifically interacting with one or more, preferably at least two, more preferably at least three and most preferably at least four amino acids of an epitope.

As used herein, the terms "specifically interacting", "specifically binding" or "specifically bind(s)" mean that a binding domain exhibits appreciable affinity for a particular protein or antigen and, generally, does not exhibit significant reactivity with proteins or antigens other than the cell surface molecule on a target cell or CD3. "Appreciable affinity" includes binding with an affinity of about 10⁻⁶M (KD) or stronger. Preferably, binding is considered specific when binding affinity is about 10⁻¹² to 10⁻⁸ M, 10⁻¹² to 10⁻⁹ M, 10⁻¹² to 10⁻¹⁰ M, 10⁻¹¹ to 10⁻⁸ M, preferably of about 10⁻¹¹ to 10⁻⁹ M. Whether a binding domain specifically reacts with or binds to a target can be tested readily by, *inter alia,* comparing the reaction of said binding domain with a target protein or antigen with the reaction of said binding domain with proteins or antigens other than the cell surface molecule on a target cell or CD3. Preferably, a binding domain of the invention does not essentially bind or is not capable of binding to proteins or antigens other than the cell surface molecule on a target cell or CD3 (i.e. the second binding domain is not capable of binding to proteins other than the cell surface molecule on a target cell and the third binding domain is not capable of binding to proteins other than CD3).

The term "does not essentially bind", or "is not capable of binding" means that a binding domain of the present invention does not bind another protein or antigen other than the elected cell surface molecule on a target cell or CD3, i.e., does not show reactivity of more than 30%, preferably not more than 20%, more preferably not more than 10%, particularly preferably not more than 9%, 8%, 7%, 6% or 5% with proteins or antigens other than the cell surface molecule on a target cell or CD3, whereby binding to the cell surface molecule on a target cell or CD3, respectively, is set to be 100%.

Specific binding is believed to be effected by specific motifs in the amino acid sequence of the binding domain and the antigen. Thus, binding is achieved as a result of their primary, secondary and/or tertiary structure as well as the result of secondary modifications of said structures. The specific interaction of the antigen-interaction-site with its specific antigen may result in a simple binding of said site to the antigen. Moreover, the specific interaction of the antigen-interaction-site with its specific antigen may alternatively or additionally result in the initiation of a signal, e.g. due to the induction of a change of the conformation of the antigen, an oligomerization of the antigen, etc.

Proteins (including fragments thereof, preferably biologically active fragments, and peptides, usually having less than 30 amino acids) comprise one or more amino acids coupled to each other via a covalent peptide bond (resulting in a chain of amino acids). The term "polypeptide" as used herein describes a group of molecules, which consist of more than 30 amino acids. Polypeptides may further form multimers such as dimers, trimers and higher oligomers, i.e. consisting of more than one polypeptide molecule. Polypeptide molecules forming such dimers, trimers etc. may be identical or non-identical. The corresponding higher order structures of such multimers are, consequently, termed homo- or heterodimers, homo- or heterotrimers etc. An example for a hereteromultimer is an antibody molecule, which, in its naturally occurring form, consists of two identical light polypeptide chains and two identical heavy polypeptide chains. The terms "polypeptide" and "protein" also refer to naturally modified polypeptides/proteins wherein the modification is effected e.g. by post-translational modifications like glycosylation, acetylation, phosphorylation and the like. A "polypeptide" when referred to herein may also be chemically modified such as pegylated. Such modifications are well known in the art.

"Isolated" when used to describe the bispecific antibody disclosed herein, means a bispecific antibody that has been identified, separated and/or recovered from a component of its production environment. Preferably, the isolated bispecific antibody is free of association with all other components from its production environment. Contaminant components of its production environment, such as that resulting from recombinant transfected cells, are materials that would typically interfere with diagnostic or therapeutic uses for the polypeptide, and may include enzymes, hormones, and other proteinaceous or non-proteinaceous solutes. In preferred embodiments, the bispecific antibody will be purified (1) to a degree sufficient to obtain at least 15 residues of N-terminal or internal amino acid sequence by use of a spinning cup sequenator, or (2) to homogeneity by SDS-PAGE under non-reducing or reducing conditions using Coomassie blue or, preferably, silver stain. Ordinarily, however, an isolated antibody will be prepared by at least one purification step.

Amino acid sequence modifications of the bispecific antibodies described herein are contemplated. For example, it may be desirable to improve the binding affinity and/or other biological properties of the antibody. Amino acid sequence variants of the bispecific antibodies are prepared by introducing appropriate nucleotide changes into the bispecific antibodies nucleic acid, or by peptide synthesis.

Such modifications include, for example, deletions from, and/or insertions into, and/or substitutions of, residues within the amino acid sequences of the bispecific antibodies. Any combination of deletion, insertion, and substitution is made to arrive at the final construct, provided that the final construct possesses the desired characteristics. The amino acid changes also may alter post-translational processes of the bispecific antibodies, such as changing the number or position of glycosylation sites. Preferably, 1, 2, 3, 4, 5, 6, 7, 8, 9, or 10 amino acids may be substituted in a CDR, while 1, 2, 3, 4, 5, 6, 7, 8, 9, 10, 11, 12, 13, 14, 15, 16, 17, 18, 19, 20, or 25 amino acids may be substituted in the framework regions (FRs). The substitutions are preferably conservative substitutions as described herein. Additionally or alternatively, 1, 2, 3, 4, 5, or 6 amino acids may be inserted or deleted in each of the CDRs (of course, dependent on their length), while 1, 2, 3, 4, 5, 6, 7, 8, 9, 10, 11, 12, 13, 14, 15, 16, 17, 18, 19, 20, or 25 amino acids may be inserted or deleted in each of the FRs.

A useful method for identification of certain residues or regions of the bispecific antibodies that are preferred locations for mutagenesis is called "alanine scanning mutagenesis" as described by Cunningham and Wells in Science, 244: 1081-1085 (1989). Here, a residue or group of target residues within the bispecific antibody is/are identified (e.g. charged residues such as arg, asp, his, lys, and glu) and replaced by a neutral or negatively charged amino acid (most preferably alanine or polyalanine) to affect the interaction of the amino acids with the epitope.

Those amino acid locations demonstrating functional sensitivity to the substitutions then are refined by introducing further or other variants at, or for, the sites of substitution. Thus, while the site for introducing an amino acid sequence variation is predetermined, the nature of the mutation *per se* needs not to be predetermined. For example, to analyze the performance of a mutation at a given site, ala scanning or random mutagenesis is conducted at a target codon or region and the expressed bispecific antibody variants are screened for the desired activity.

Preferably, amino acid sequence insertions include amino- and/or carboxyl-terminal fusions ranging in length from 1, 2, 3, 4, 5, 6, 7, 8, 9 or 10 residues to polypeptides containing a hundred or more residues, as well as intrasequence insertions of single or multiple amino acid residues. An insertional variant of the bispecific antibody includes the fusion to the N-or C-terminus of the antibody to an enzyme or a fusion to a polypeptide which increases the serum half-life of the antibody.

Another type of variant is an amino acid substitution variant. These variants have preferably at least 1, 2, 3, 4, 5, 6, 7, 8, 9 or 10 amino acid residues in the bispecific antibody replaced by a different residue. The sites of greatest interest for substitutional mutagenesis include the CDRs of the heavy and/or light chain, in particular the hypervariable regions, but FR alterations in the heavy and/or light chain are also contemplated.

For example, if a CDR sequence encompasses 6 amino acids, it is envisaged that one, two or three of these amino acids are substituted. Similarly, if a CDR sequence encompasses 15 amino acids it is envisaged that one, two, three, four, five or six of these amino acids are substituted.

Generally, if amino acids are substituted in one or more or all of the CDRs of the heavy and/or light chain, it is preferred that the then-obtained "substituted" sequence is at least 60%, more preferably 65%, even more preferably 70%, particularly preferably 75%, more particularly preferably 80% identical to the "original" CDR sequence. This means that it is dependent of the length of the CDR to which degree it is identical to the "substituted" sequence. For example, a CDR having 5 amino acids is preferably 80% identical to its substituted sequence in order to have at least one amino acid substituted. Accordingly, the CDRs of the bispecific antibody may have different degrees of identity to their substituted sequences, e.g., CDRL1 may have 80%, while CDRL3 may have 90%.

Preferred substitutions (or replacements) are conservative substitutions. However, any substitution (including non-conservative substitution or one or more from the "exemplary substitutions" listed in Table 1, below) is envisaged as long as the bispecific antibody retains its capability to bind to the cell surface molecule on a target cell via the second binding domain and to CD3 epsilon via the third binding domain and/or its CDRs have an identity to the then substituted sequence (at least 60%, more preferably 65%, even more preferably 70%, particularly preferably 75%, more particularly preferably 80% identical to the "original" CDR sequence).

Conservative substitutions are shown in Table 1 under the heading of "preferred substitutions". If such substitutions result in a change in biological activity, then more substantial changes, denominated "exemplary substitutions" in Table 1, or as further described below in reference to amino acid classes, may be introduced and the products screened for a desired characteristic.

**Table 1: Amino Acid Substitutions**

| Original | Exemplary Substitutions | Preferred Substitutions |
|---|---|---|
| Ala (A) | val, leu, ile | val |
| Arg (R) | lys, gln, asn | lys |
| Asn (N) | gln, his, asp, lys, arg | gln |
| Asp (D) | glu, asn | glu |
| Cys (C) | ser, ala | ser |
| Gln (Q) | asn, glu | asn |
| Glu (E) | asp, gln | asp |
| Gly (G) | ala | ala |
| His (H) | asn, gln, lys, arg | arg |
| Ile (I) | leu, val, met, ala, phe | leu |
| Leu (L) | norleucine, ile, val, met, ala | ile |
| Lys (K) | arg, gln, asn | arg |
| Met (M) | leu, phe, ile | leu |
| Phe (F) | leu, val, ile, ala, tyr | tyr |
| Pro (P) | ala | ala |
| Ser (S) | thr | thr |
| Thr (T) | ser | ser |
| Trp (W) | tyr, phe | tyr |
| Tyr (Y) | trp, phe, thr, ser | phe |
| Val (V) | ile, leu, met, phe, ala | leu |

Substantial modifications in the biological properties of the bispecific antibody of the present invention are accomplished by selecting substitutions that differ significantly in their effect on maintaining (a) the structure of the polypeptide backbone in the area of the substitution, for example, as a sheet or helical conformation, (b) the charge or hydrophobicity of the molecule at the target site, or (c) the bulk of the side chain. Naturally occurring residues are divided into groups based on common side-chain properties: (1) hydrophobic: norleucine, met, ala, val, leu, ile; (2) neutral hydrophilic: cys, ser, thr; (3) acidic: asp, glu; (4) basic: asn, gin, his, lys, arg; (5) residues that influence chain orientation: gly, pro; and (6) aromatic : trp, tyr, phe.

Non-conservative substitutions will entail exchanging a member of one of these classes for another class. Any cysteine residue not involved in maintaining the proper conformation of the bispecific antibody may be substituted, generally with serine, to improve the oxidative stability of the molecule and prevent aberrant crosslinking. Conversely, cysteine bond(s) may be added to the antibody to improve its stability (particularly where the antibody is an antibody fragment such as an Fv fragment).

A particularly preferred type of substitutional variant involves substituting one or more hypervariable region residues of a parent antibody (e. g. a humanized or human antibody). Generally, the resulting variant(s) selected for further development will have improved biological properties relative to the parent antibody from which they are generated. A convenient way for generating such substitutional variants involves affinity maturation using phage display. Briefly, several hypervariable region sites (e. g. 6-7 sites) are mutated to generate all possible amino acid substitutions at each site. The antibody variants thus generated are displayed in a monovalent fashion from filamentous phage particles as fusions to the gene III product of M13 packaged within each particle. The phage-displayed variants are then screened for their biological activity (e. g. binding affinity) as herein disclosed. In order to identify candidate hypervariable region sites for modification, alanine scanning mutagenesis can be performed to identify hypervariable region residues contributing significantly to antigen binding. Alternatively, or additionally, it may be beneficial to analyze a crystal structure of the antigen-antibody complex to identify contact points between the binding domain and, e.g., a human cell surface molecule on a target cell. Such contact residues and neighbouring residues are candidates for substitution according to the techniques elaborated herein. Once such variants are generated, the panel of variants is subjected to screening as described herein and antibodies with superior properties in one or more relevant assays may be selected for further development.

Other modifications of the bispecific antibody are contemplated herein. For example, the bispecific antibody may be linked to one of a variety of non-proteinaceous polymers, e.g., polyethylene glycol, polypropylene glycol, polyoxyalkylenes, or copolymers of polyethylene glycol and polypropylene glycol. The bispecific antibody may also be entrapped in microcapsules prepared, for example, by coacervation techniques or by interfacial polymerization (for example, hydroxymethylcellulose or gelatine-microcapsules and poly (methylmethacylate) microcapsules, respectively), in colloidal drug delivery systems (for example, liposomes, albumin microspheres, microemulsions, nanoparticles and nanocapsules), or in macroemulsions. Such techniques are disclosed in Remington's Pharmaceutical Sciences, 16th edition, Oslo, A., Ed., (1980).

The bispecific antibodies disclosed herein may also be formulated as immuno-liposomes. A "liposome" is a small vesicle composed of various types of lipids, phospholipids and/or surfactant which is useful for delivery of a drug to a mammal. The components of the liposome are commonly arranged in a bilayer formation, similar to the lipid arrangement of biological membranes. Liposomes containing the antibody are prepared by methods known in the art, such as described in Epstein et al., Proc. Natl. Acad. Sci. USA, 82: 3688 (1985); Hwang et al. , Proc. Natl Acad. Sci. USA, 77: 4030 (1980); US Pat. Nos. 4,485,045 and 4,544,545; and WO 97/38731 published October 23, 1997. Liposomes with enhanced circulation time are disclosed in US Patent No. 5,013, 556. Particularly useful liposomes can be generated by the reverse phase evaporation method with a lipid composition comprising phosphatidylcholine, cholesterol and PEG-derivatized phosphatidylethanolamine (PEG-PE). Liposomes are extruded through filters of defined pore size to yield liposomes with the desired diameter. Fab' fragments of the antibody of the present invention can be conjugated to the liposomes as described in Martin et al. J. Biol. Chem. 257: 286-288 (1982) via a disulfide interchange reaction. A chemotherapeutic agent is optionally contained within the liposome. See Gabizon et al. J. National Cancer Inst. 81 (19) 1484 (1989).

When using recombinant techniques, the bispecific antibody can be produced intracellularly, in the periplasmic space, or directly secreted into the medium. If the bispecific antibody is produced intracellularly, as a first step, the particulate debris, either host cells or lysed fragments, are removed, for example, by centrifugation or ultrafiltration. Carter et al., Bio/Technology 10: 163-167 (1992) describe a procedure for isolating antibodies which are secreted to the periplasmic space of *E. coli.*

The bispecific antibody composition prepared from the cells can be purified using, for example, hydroxylapatite chromatography, gel electrophoresis, dialysis, and affinity chromatography, with affinity chromatography being the preferred purification technique.

The term "nucleic acid" is well known to the skilled person and encompasses DNA (such as cDNA) and RNA (such as mRNA). The nucleic acid can be double stranded and single stranded, linear and circular. Said nucleic acid molecule is preferably comprised in a vector which is preferably comprised in a host cell. Said host cell is, e.g. after transformation or transfection with the nucleic acid sequence of the invention, capable of expressing the bispecific antibody. For that purpose the nucleic acid molecule is operatively linked with control sequences.

A vector is a nucleic acid molecule used as a vehicle to transfer (foreign) genetic material into a cell. The term "vector" encompasses - but is not restricted to - plasmids, viruses, cosmids and artificial chromosomes. In general, engineered vectors comprise an origin of replication, a multicloning site and a selectable marker. The vector itself is generally a nucleotide sequence, commonly a DNA sequence, that comprises an insert (transgene) and a larger sequence that serves as the "backbone" of the vector. Modern vectors may encompass additional features besides the transgene insert and a backbone: promoter, genetic marker, antibiotic resistance, reporter gene, targeting sequence, protein purification tag. Vectors called expression vectors (expression constructs) specifically are for the expression of the transgene in the target cell, and generally have control sequences such as a promoter sequence that drives expression of the transgene. Insertion of a vector into the target cell is usually called "transformation" for bacteria, "transfection" for eukaryotic cells, although insertion of a viral vector is also called "transduction".

As used herein, the term "host cell" is intended to refer to a cell into which a nucleic acid encoding the bispecific antibody of the invention is introduced by way of transformation, transfection and the like. It should be understood that such terms refer not only to the particular subject cell but to the progeny or potential progeny of such a cell. Because certain modifications may occur in succeeding generations due to either mutation or environmental influences, such progeny may not, in fact, be identical to the parent cell, but are still included within the scope of the term as used herein.

As used herein, the term "expression" includes any step involved in the production of a bispecific antibody of the invention including, but not limited to, transcription, post-transcriptional modification, translation, post-translational modification, and secretion.

The term "control sequences" refers to DNA sequences necessary for the expression of an operably linked coding sequence in a particular host organism. The control sequences that are suitable for prokaryotes, for example, include a promoter, optionally an operator sequence, and a ribosome binding site. Eukaryotic cells are known to utilize promoters, polyadenylation signals, and enhancers.

A nucleic acid is "operably linked" when it is placed into a functional relationship with another nucleic acid sequence. For example, DNA for a presequence or secretory leader is operably linked to DNA for a polypeptide if it is expressed as a preprotein that participates in the secretion of the polypeptide; a promoter or enhancer is operably linked to a coding sequence if it affects the transcription of the sequence; or a ribosome binding site is operably linked to a coding sequence if it is positioned so as to facilitate translation. Generally, "operably linked" means that the DNA sequences being linked are contiguous, and, in the case of a secretory leader, contiguous and in reading phase. However, enhancers do not have to be contiguous. Linking is accomplished by ligation at convenient restriction sites. If such sites do not exist, the synthetic oligonucleotide adaptors or linkers are used in accordance with conventional practice.

The terms "host cell," "target cell" or "recipient cell" are intended to include any individual cell or cell culture that can be or has/have been recipients for vectors or the incorporation of exogenous nucleic acid molecules, polynucleotides and/or proteins. It also is intended to include progeny of a single cell, and the progeny may not necessarily be completely identical (in morphology or in genomic or total DNA complement) to the original parent cell due to natural, accidental, or deliberate mutation. The cells may be prokaryotic or eukaryotic, and include but are not limited to bacteria, yeast cells, animal cells, and mammalian cells, e.g., murine, rat, macaque or human.

Suitable host cells include prokaryotes and eukaryotic host cells including yeasts, fungi, insect cells and mammalian cells.

The bispecific antibody of the invention can be produced in bacteria. After expression, the bispecific antibody of the invention, preferably the bispecific antibody is isolated from the *E. coli* cell paste in a soluble fraction and can be purified through, e.g., affinity chromatography and/or size exclusion. Final purification can be carried out similar to the process for purifying antibody expressed e. g, in CHO cells.

In addition to prokaryotes, eukaryotic microbes such as filamentous fungi or yeast are suitable cloning or expression hosts for the bispecific antibody of the invention. *Saccharomyces cerevisiae,* or common baker's yeast, is the most commonly used among lower eukaryotic host microorganisms. However, a number of other genera, species, and strains are commonly available and useful herein, such as *Schizosaccharomyces pombe,* Kluyveromyces hosts such as, e.g., *K. lactis, K. fragilis* (ATCC 12424), *K. bulgaricus* (ATCC 16045), *K. wickeramii* (ATCC 24178), *K. waltii* (ATCC 56500), *K. drosophilarum* (ATCC 36906), *K. thermotolerans,* and *K. marxianus;* yarrowia (EP 402 226); *Pichia pastoris* (EP 183 070); Candida; *Trichoderma reesia* (EP 244 234); *Neurospora crassa;* Schwanniomyces such as *Schwanniomyces occidentalis;* and filamentous fungi such as, e.g., Neurospora, Penicillium, Tolypocladium, and Aspergillus hosts such as *A. nidulans* and *A. niger.*

Suitable host cells for the expression of glycosylated bispecific antibody of the invention, preferably antibody derived bispecific antibodies are derived from multicellular organisms. Examples of invertebrate cells include plant and insect cells. Numerous baculoviral strains and variants and corresponding permissive insect host cells from hosts such as *Spodoptera frugiperda* (caterpillar), *Aedes aegypti* (mosquito), *Aedes albopictus* (mosquito), *Drosophila melanogaster* (fruit fly), and *Bombyx mori* have been identified. A variety of viral strains for transfection are publicly available, e. g. , the L-1 variant of *Autographa californica* NPV and the Bm-5 strain of *Bombyx mori* NPV, and such viruses may be used as the virus herein according to the present invention, particularly for transfection of *Spodoptera frugiperda* cells.

Plant cell cultures of cotton, corn, potato, soybean, petunia, tomato, Arabidopsis and tobacco can also be utilized as hosts. Cloning and expression vectors useful in the production of proteins in plant cell culture are known to those of skill in the art. See e.g. Hiatt et al., Nature (1989) 342: 76-78, Owen et al. (1992) Bio/Technology 10: 790-794, Artsaenko et al. (1995) The Plant J 8: 745-750, and Fecker et al. (1996) Plant Mol Biol 32: 979-986.

However, interest has been greatest in vertebrate cells, and propagation of vertebrate cells in culture (tissue culture) has become a routine procedure. Examples of useful mammalian host cell lines are monkey kidney CV1 line transformed by SV40 (COS-7, ATCC CRL 1651); human embryonic kidney line (293 or 293 cells subcloned for growth in suspension culture, Graham et al. , J. Gen Virol. 36 : 59 (1977)); baby hamster kidney cells (BHK, ATCC CCL 10); Chinese hamster ovary cells/-DHFR (CHO, Urlaub et al. , Proc. Natl. Acad. Sci. USA 77: 4216 (1980)); mouse sertoli cells (TM4, Mather, Biol. Reprod. 23: 243-251 (1980)); monkey kidney cells (CVI ATCC CCL 70); African green monkey kidney cells (VERO-76, ATCC CRL1587) ; human cervical carcinoma cells (HELA, ATCC CCL 2); canine kidney cells (MDCK, ATCC CCL 34); buffalo rat liver cells (BRL 3A, ATCC CRL 1442); human lung cells (W138, ATCC CCL 75); human liver cells (Hep G2,1413 8065); mouse mammary tumor (MMT 060562, ATCC CCL5 1); TRI cells (Mather et al., Annals N. Y Acad. Sci. 383 : 44-68 (1982)) ; MRC 5 cells; FS4 cells; and a human hepatoma line (Hep G2).

When using recombinant techniques, the bispecific antibody of the invention can be produced intracellularly, in the periplasmic space, or directly secreted into the medium. If the bispecific antibody is produced intracellularly, as a first step, the particulate debris, either host cells or lysed fragments, are removed, for example, by centrifugation or ultrafiltration. Carter et al., Bio/Technology 10: 163-167 (1992) describe a procedure for isolating antibodies which are secreted to the periplasmic space of *E. coli.* Briefly, cell paste is thawed in the presence of sodium acetate (pH 3.5), EDTA, and phenylmethylsulfonylfluoride (PMSF) over about 30 min. Cell debris can be removed by centrifugation. Where the antibody is secreted into the medium, supernatants from such expression systems are generally first concentrated using a commercially available protein concentration filter, for example, an Amicon or Millipore Pellicon ultrafiltration unit. A protease inhibitor such as PMSF may be included in any of the foregoing steps to inhibit proteolysis and antibiotics may be included to prevent the growth of adventitious contaminants.

The bispecific antibody of the invention prepared from the host cells can be purified using, for example, hydroxylapatite chromatography, gel electrophoresis, dialysis, and affinity chromatography, with affinity chromatography being the preferred purification technique.

The matrix to which the affinity ligand is attached is most often agarose, but other matrices are available. Mechanically stable matrices such as controlled pore glass or poly (styrenedivinyl) benzene allow for faster flow rates and shorter processing times than can be achieved with agarose. Where the bispecific antibody of the invention comprises a CH3 domain, the Bakerbond ABXMresin (J. T. Baker, Phillipsburg, NJ) is useful for purification. Other techniques for protein purification such as fractionation on an ion-exchange column, ethanol precipitation, Reverse Phase HPLC, chromatography on silica, chromatography on heparin SEPHAROSE™ chromatography on an anion or cation exchange resin (such as a polyaspartic acid column), chromato-focusing, SDS-PAGE, and ammonium sulfate precipitation are also available depending on the antibody to be recovered.

The term "culturing" refers to the in vitro maintenance, differentiation, growth, proliferation and/or propagation of cells under suitable conditions in a medium.

As used herein, the term "pharmaceutical composition" relates to a composition for administration to a patient, preferably a human patient. The particular preferred pharmaceutical composition of this invention comprises the bispecific antibody of the invention. Preferably, the pharmaceutical composition comprises suitable formulations of carriers, stabilizers and/or excipients. In a preferred embodiment, the pharmaceutical composition comprises a composition for parenteral, transdermal, intraluminal, intraarterial, intrathecal and/or intranasal administration or by direct injection into tissue. It is in particular envisaged that said composition is administered to a patient via infusion or injection. Administration of the suitable compositions may be effected by different ways, e.g., by intravenous, intraperitoneal, subcutaneous, intramuscular, topical or intradermal administration. In particular, the present invention provides for an uninterrupted administration of the suitable composition. As a non-limiting example, uninterrupted, i.e. continuous administration may be realized by a small pump system worn by the patient for metering the influx of therapeutic agent into the body of the patient. The pharmaceutical composition comprising the bispecific antibody of the invention can be administered by using said pump systems. Such pump systems are generally known in the art, and commonly rely on periodic exchange of cartridges containing the therapeutic agent to be infused. When exchanging the cartridge in such a pump system, a temporary interruption of the otherwise uninterrupted flow of therapeutic agent into the body of the patient may ensue. In such a case, the phase of administration prior to cartridge replacement and the phase of administration following cartridge replacement would still be considered within the meaning of the pharmaceutical means and methods of the invention together make up one "uninterrupted administration" of such therapeutic agent.

The continuous or uninterrupted administration of these bispecific antibodies of the invention may be intravenous or subcutaneous by way of a fluid delivery device or small pump system including a fluid driving mechanism for driving fluid out of a reservoir and an actuating mechanism for actuating the driving mechanism. Pump systems for subcutaneous administration may include a needle or a cannula for penetrating the skin of a patient and delivering the suitable composition into the patient's body. Said pump systems may be directly fixed or attached to the skin of the patient independently of a vein, artery or blood vessel, thereby allowing a direct contact between the pump system and the skin of the patient. The pump system can be attached to the skin of the patient for 24 hours up to several days. The pump system may be of small size with a reservoir for small volumes. As a non-limiting example, the volume of the reservoir for the suitable pharmaceutical composition to be administered can be between 0.1 and 50 ml.

The continuous administration may be transdermal by way of a patch worn on the skin and replaced at intervals. One of skill in the art is aware of patch systems for drug delivery suitable for this purpose. It is of note that transdermal administration is especially amenable to uninterrupted administration, as exchange of a first exhausted patch can advantageously be accomplished simultaneously with the placement of a new, second patch, for example on the surface of the skin immediately adjacent to the first exhausted patch and immediately prior to removal of the first exhausted patch. Issues of flow interruption or power cell failure do not arise.

The inventive compositions may further comprise a pharmaceutically acceptable carrier. Examples of suitable pharmaceutical carriers are well known in the art and include solutions, e.g. phosphate buffered saline solutions, water, emulsions, such as oil/water emulsions, various types of wetting agents, sterile solutions, liposomes, etc. Compositions comprising such carriers can be formulated by well known conventional methods. Formulations can comprise carbohydrates, buffer solutions, amino acids and/or surfactants. Carbohydrates may be non-reducing sugars, preferably trehalose, sucrose, octasulfate, sorbitol or xylitol. In general, as used herein, "pharmaceutically acceptable carrier" means any and all solvents, dispersion media, coatings, antibacterial and antifungal agents, isotonic and absorption delaying agents, compatible with pharmaceutical administration. The use of such media and agents for pharmaceutically active substances is well known in the art. Acceptable carriers, excipients, or stabilizers are nontoxic to recipients at the dosages and concentrations employed and include: additional buffering agents; preservatives; co-solvents; antioxidants, including ascorbic acid and methionine; chelating agents such as EDTA; metal complexes (e.g., Zn-protein complexes); biodegradable polymers, such as polyesters; salt-forming counter-ions, such as sodium, polyhydric sugar alcohols; amino acids, such as alanine, glycine, asparagine, 2-phenylalanine, and threonine; sugars or sugar alcohols, such as trehalose, sucrose, octasulfate, sorbitol or xylitol stachyose, mannose, sorbose, xylose, ribose, myoinisitose, galactose, lactitol, ribitol, myoinisitol, galactitol, glycerol, cyclitols (e.g., inositol), polyethylene glycol; sulfur containing reducing agents, such as glutathione, thioctic acid, sodium thioglycolate, thioglycerol, [alpha]-monothioglycerol, and sodium thio sulfate; low molecular weight proteins, such as human serum albumin, bovine serum albumin, gelatin, or other immunoglobulins; and hydrophilic polymers, such as polyvinylpyrrolidone. Such formulations may be used for continuous administrations which may be intravenuous or subcutaneous with and/or without pump systems. Amino acids may be charged amino acids, preferably lysine, lysine acetate, arginine, glutamate and/or histidine. Surfactants may be detergents, preferably with a molecular weight of >1.2 KD and/or a polyether, preferably with a molecular weight of >3 KD. Non-limiting examples for preferred detergents are Tween 20, Tween 40, Tween 60, Tween 80 or Tween 85. Non-limiting examples for preferred polyethers are PEG 3000, PEG 3350, PEG 4000 or PEG 5000. Buffer systems used in the present invention can have a preferred pH of 5-9 and may comprise citrate, succinate, phosphate, histidine and acetate.

The compositions of the present invention can be administered to the subject at a suitable dose which can be determined e.g. by dose escalating studies by administration of increasing doses of the polypeptide of the invention exhibiting cross-species specificity described herein to non-chimpanzee primates, for instance macaques. As set forth above, the bispecific antibody of the invention exhibiting cross-species specificity described herein can be advantageously used in identical form in preclinical testing in non-chimpanzee primates and as drug in humans. These compositions can also be administered in combination with other proteinaceous and non-proteinaceous drugs. These drugs may be administered simultaneously with the composition comprising the polypeptide of the invention as defined herein or separately before or after administration of said polypeptide in timely defined intervals and doses. The dosage regimen will be determined by the attending physician and clinical factors. As is well known in the medical arts, dosages for any one patient depend upon many factors, including the patient's size, body surface area, age, the particular compound to be administered, sex, time and route of administration, general health, and other drugs being administered concurrently.

Preparations for parenteral administration include sterile aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's, or fixed oils. Intravenous vehicles include fluid and nutrient replenishers, electrolyte replenishers (such as those based on Ringer's dextrose), and the like. Preservatives and other additives may also be present such as, for example, antimicrobials, anti-oxidants, chelating agents, inert gases and the like. In addition, the composition of the present invention might comprise proteinaceous carriers, like, e.g., serum albumin or immunoglobulin, preferably of human origin. It is envisaged that the composition of the invention might comprise, in addition to the polypeptide of the invention defined herein, further biologically active agents, depending on the intended use of the composition. Such agents might be drugs acting on the gastro-intestinal system, drugs acting as cytostatica, drugs preventing hyperurikemia, drugs inhibiting immunoreactions (e.g. corticosteroids), drugs modulating the inflammatory response, drugs acting on the circulatory system and/or agents such as cytokines known in the art. It is also envisaged that the bispecific antibody of the present invention is applied in a co-therapy, i.e., in combination with another anti-cancer medicament.

The biological activity of the pharmaceutical composition defined herein can be determined for instance by cytotoxicity assays, as described in the following examples, in WO 99/54440 or by Schlereth et al. (Cancer Immunol. Immunother. 20 (2005), 1-12). "Efficacy" *or "in vivo* efficacy" as used herein refers to the response to therapy by the pharmaceutical composition of the invention, using e.g. standardized NCI response criteria. The success or *in vivo* efficacy of the therapy using a pharmaceutical composition of the invention refers to the effectiveness of the composition for its intended purpose, i.e. the ability of the composition to cause its desired effect, i.e. depletion of pathologic cells, e.g. tumor cells. The *in vivo* efficacy may be monitored by established standard methods for the respective disease entities including, but not limited to white blood cell counts, differentials, Fluorescence Activated Cell Sorting, bone marrow aspiration. In addition, various disease specific clinical chemistry parameters and other established standard methods may be used. Furthermore, computer-aided tomography, X-ray, nuclear magnetic resonance tomography (e.g. for National Cancer Institute-criteria based response assessment [Cheson BD, Horning SJ, Coiffier B, Shipp MA, Fisher RI, Connors JM, Lister TA, Vose J, Grillo-Lopez A, Hagenbeek A, Cabanillas F, Klippensten D, Hiddemann W, Castellino R, Harris NL, Armitage JO, Carter W, Hoppe R, Canellos GP. Report of an international workshop to standardize response criteria for non-Hodgkin's lymphomas. NCI Sponsored International Working Group. J Clin Oncol. 1999 Apr;17(4):1244]), positron-emission tomography scanning, white blood cell counts, differentials, Fluorescence Activated Cell Sorting, bone marrow aspiration, lymph node biopsies/histologies, and various lymphoma specific clinical chemistry parameters (e.g. lactate dehydrogenase) and other established standard methods may be used.

Another major challenge in the development of drugs such as the pharmaceutical composition of the invention is the predictable modulation of pharmacokinetic properties. To this end, a pharmacokinetic profile of the drug candidate, i.e. a profile of the pharmacokinetic parameters that affect the ability of a particular drug to treat a given condition, can be established. Pharmacokinetic parameters of the drug influencing the ability of a drug for treating a certain disease entity include, but are not limited to: half-life, volume of distribution, hepatic first-pass metabolism and the degree of blood serum binding. The efficacy of a given drug agent can be influenced by each of the parameters mentioned above.

"Half-life" means the time where 50% of an administered drug are eliminated through biological processes, e.g. metabolism, excretion, etc.

By "hepatic first-pass metabolism" is meant the propensity of a drug to be metabolized upon first contact with the liver, i.e. during its first pass through the liver.

"Volume of distribution" means the degree of retention of a drug throughout the various compartments of the body, like e.g. intracellular and extracellular spaces, tissues and organs, etc. and the distribution of the drug within these compartments.

"Degree of blood serum binding" means the propensity of a drug to interact with and bind to blood serum proteins, such as albumin, leading to a reduction or loss of biological activity of the drug.

Pharmacokinetic parameters also include bioavailability, lag time (Tlag), Tmax, absorption rates, more onset and/or Cmax for a given amount of drug administered. "Bioavailability" means the amount of a drug in the blood compartment. "Lag time" means the time delay between the administration of the drug and its detection and measurability in blood or plasma.

"Tmax" is the time after which maximal blood concentration of the drug is reached, and "Cmax" is the blood concentration maximally obtained with a given drug. The time to reach a blood or tissue concentration of the drug which is required for its biological effect is influenced by all parameters. Pharmacokinetic parameters of bispecific single chain antibodies exhibiting cross-species specificity, which may be determined in preclinical animal testing in non-chimpanzee primates as outlined above, are also set forth e.g. in the publication by Schlereth et al. (Cancer Immunol. Immunother. 20 (2005), 1-12).

The term "toxicity" as used herein refers to the toxic effects of a drug manifested in adverse events or severe adverse events. These side events might refer to a lack of tolerability of the drug in general and/or a lack of local tolerance after administration. Toxicity could also include teratogenic or carcinogenic effects caused by the drug.

The term "safety", *"in vivo* safety" or "tolerability" as used herein defines the administration of a drug without inducing severe adverse events directly after administration (local tolerance) and during a longer period of application of the drug. "Safety", *"in vivo* safety" or "tolerability" can be evaluated e.g. at regular intervals during the treatment and follow-up period. Measurements include clinical evaluation, e.g. organ manifestations, and screening of laboratory abnormalities. Clinical evaluation may be carried out and deviations to normal findings recorded/coded according to NCI-CTC and/or MedDRA standards. Organ manifestations may include criteria such as allergy/immunology, blood/bone marrow, cardiac arrhythmia, coagulation and the like, as set forth e.g. in the Common Terminology Criteria for adverse events v3.0 (CTCAE). Laboratory parameters which may be tested include for instance hematology, clinical chemistry, coagulation profile and urine analysis and examination of other body fluids such as serum, plasma, lymphoid or spinal fluid, liquor and the like. Safety can thus be assessed e.g. by physical examination, imaging techniques (i.e. ultrasound, x-ray, CT scans, Magnetic Resonance Imaging (MRI), other measures with technical devices (i.e. electrocardiogram), vital signs, by measuring laboratory parameters and recording adverse events. For example, adverse events in non-chimpanzee primates in the uses and methods according to the invention may be examined by histopathological and/or histochemical methods.

The term "effective dose" or "effective dosage" is defined as an amount sufficient to achieve or at least partially achieve the desired effect. The term "therapeutically effective dose" is defined as an amount sufficient to cure or at least partially arrest the disease and its complications in a patient already suffering from the disease. Amounts effective for this use will depend upon the severity of the infection and the general state of the subject's own immune system. The term "patient" includes human and other mammalian subjects that receive either prophylactic or therapeutic treatment.

The term "effective and non-toxic dose" as used herein refers to a tolerable dose of an inventive bispecific antibody which is high enough to cause depletion of pathologic cells, tumor elimination, tumor shrinkage or stabilization of disease without or essentially without major toxic effects. Such effective and non-toxic doses may be determined e.g. by dose escalation studies described in the art and should be below the dose inducing severe adverse side events (dose limiting toxicity, DLT).

The above terms are also referred to e.g. in the Preclinical safety evaluation of biotechnology-derived pharmaceuticals S6; ICH Harmonised Tripartite Guideline; ICH Steering Committee meeting on July 16, 1997.

The appropriate dosage, or therapeutically effective amount, of the bispecific antibody of the invention will depend on the condition to be treated, the severity of the condition, prior therapy, and the patient's clinical history and response to the therapeutic agent. The proper dose can be adjusted according to the judgment of the attending physician such that it can be administered to the patient one time or over a series of administrations. The pharmaceutical composition can be administered as a sole therapeutic or in combination with additional therapies such as anti-cancer therapies as needed.

The pharmaceutical compositions of this invention are particularly useful for parenteral administration, i.e., subcutaneously, intramuscularly, intravenously, intra-articular and/or intra-synovial. Parenteral administration can be by bolus injection or continuous infusion.

If the pharmaceutical composition has been lyophilized, the lyophilized material is first reconstituted in an appropriate liquid prior to administration. The lyophilized material may be reconstituted in, e.g., bacteriostatic water for injection (BWFI), physiological saline, phosphate buffered saline (PBS), or the same formulation the protein had been in prior to lyophilization.

The mode of action of the bispecific antibodies that bind both to a cell surface molecule on a target cell such as a tumor antigen and to the T cell CD3 receptor complex is commonly known. Bringing a T cell in close vicinity to a target cell, i.e., engaging said T cell results under the circumstances in killing of the target cell by the T cell. This process can be exploited in fighting against proliferative disease, inflammatory disease, infectious disease and autoimmune disease. Thus, fusing anything such as additional amino acid sequences to the CD3 binding domain, i.e., the "effector domain" of a bispecific antibody or to the target binding domain influences the properties of the bispecific antibody such that it would no longer exert its function in properly engaging a T cell and/or binding to its target. Indeed, T-cells are equipped with granules containing a deadly combination of pore-forming proteins, called perforins, and cell death-inducing proteases, called granzymes. These proteins are delivered into target cells via a cytolytic synapse that can only form if T-cells are in closest vicinity with a target cell that is aimed to be killed. Normally, closest vicinity between a T cell and a target cell is achieved by the T cell binding to an MHC class I/peptide complex using its matching T-cell receptor. Yet, it is the
function of the bispecific antibodies of the present invention to bring a T cell into such close vicinity to a target cell in the absence of T cell receptor/MHC interaction. Hence, one can imagine that fusing anything such as additional amino acid sequences to either or all of the first, second and/or third binding domain of the bispecific antibodies of the present invention could negatively influence the function thereof, i.e., bringing together a target cell and a T cell in order to kill the target cell.

That being so and bearing in mind that it is highly desirable to increase the serum half-life of the bispecific antibodies in order to stabilize it or prevent it from fast renal clearance and the like, the skilled person seems to be in a dilemma. Indeed, while an increase of the half-life could be achieved by having a bispecific antibody binding to, e.g., serum albumin which requires equipping said bispecific antibody with a domain which is capable of binding to serum albumin, the addition of such a domain could probably adversely affect the properties of said bispecific antibody, e.g., it might lose its function or become at least less effective.

Notwithstanding this potential dilemma and bearing in mind that a bispecific antibody of the present invention could at least be weakened or even inactivated by the addition of a further binding which is capable of binding to serum albumin, the present inventors generated bispecific antibodies that have, in addition a binding domain which is capable of binding to a cell surface molecule on a target cell and a binding domain which is capable of binding to the T cell CD3 receptor complex, an additional binding domain which is capable of binding to serum albumin.

Albumin binding domains are generally known in the art (see e.g. WO 2007/106120 A2)

WO 01/45746 which provides serum albumin binding domains leaves it completely up to the skilled person to which terminus of a protein, in particular an antibody, a serum albumin binding domain should be fused - it can either be the N-or C-terminus and may depend on the circumstances. Thus, the prior art does not provide guidance.

It has been surprisingly found that the albumin binding domain positioned at the C-terminus of the CD3 receptor specific domain relates to poor yields of monomeric molecules isolated from the supernatant of host cells producing the binding domain. In contrast, when the albumin binding domain was positioned at the N-terminus of the molecule, a significant increase in expression productivity and yield was observed in comparison with the above mentioned bispecific antibodies having the albumin binding domain positioned at the C-terminus of the CD3 receptor.

This increase of the yield of monomeric molecules is indeed a surprise, since one could more likely have expected that adding the identical short albumin binding domain to either the N-terminus or the C-terminus of a given binging molecule with two different specificities for cell surface antigens should have similar effects.

The observed higher yield of monomeric molecules is especially relevant in view of a commercial production of bispecific antibodies of the invention. This optimization in the product quality allows for higher compound concentration in working solutions as well in final pharmaceutical formulations of the bispecific antibodies. It further allows for smaller fermenter volumes resulting in the same production yield and for lesser cost for purification columns and other equipment for manufacturing the product and, consequently for preferred cost of goods calculation. Moreover, the higher production concentration/concentration of product in the production cell supernatant allows for more stringent purification/isolation procedures with higher acceptable loss of product during the production, which leads to a clear separation of the product from undesired components and, thus, to a preferred higher product purity.

All the more, prior art bispecific antibodies, such as diabodies with two binding domains, one for CD3 and a second for a target molecule such as CEA, and equipped with a serum albumin binding domain are constructed in a way that the serum albumin binding domain is fused to the C-terminus of the domain binding to the target cell; see Stork et al., Prot Eng Des Sel 20(11), 569-576 (2007) and Mueller et al., J Biol Chem 282(17), 12650-12660 (2007). Thus, also from the prior one would have concluded that a serum albumin binding domain should be added to the C-terminus of the domain binding to a cell surface molecule of target cell. A similar approach was done in the construction of DART antibodies such as an ABD-DART (see WO 2010/080538, e.g. Figure 45).

That being so, the present inventors, despite the teaching of the prior art and the expectations grasped from the teaching of the prior art, added a serum albumin binding domain to the N-terminus of the binding domain that engages a target cell via binding to a cell surface molecule with a T cell via binding to the T cell receptor complex and were successful in the generation of a bispecific antibody that has an increased serum half-life in a desired yield, while it is still capable of binding to a cell surface molecule on a target cell and binding to the T cell CD3 receptor complex. Thereby it is still feasible engaging the T cell in a way that it exerts its killing functions on the target cell. Thus, a bispecific antibody of the present invention in which the binding domains are in the order as described herein (see, e.g. claim 1) is capable of mediating cytotoxicity on a target cell that is effected by T cell engaged by said bispecific antibody.

Due to the albumin binding domain of the bispecific antibody of the present invention, a bispecific antibody of the present invention has preferably an increased half-life and/or longer persistence times in the body, thereby also providing a longer functional activity of the bispecific antibody while it is still producible in an amount desirable for commercial production scale.

In addition, the present inventors have observed that a bispecific antibody of the present invention is also capable of mediating cytotoxicity in vitro in the presence of 10% (v/v) serum albumin, in particular human serum albumin. This is an important feature, since in human blood serum albumin is present at about 10-20% (v/v). In fact, a bispecific antibody of the present invention is not-naturally occurring in a mammal, in particular in a human and, thus, it could well have been that the presence of serum albumin could somehow disturb or interfere with the action of a bispecific antibody of the present invention.

By way of example, the in vitro cytotoxicity assay in the presence of serum albumin, in particular human serum albumin can be used to test a bispecific antibody of the present invention for its capability of mediating cytotoxicity.

Another surprising property of a bispecific antibody of the present invention having the order (set-up/arrangement) of the domains as described herein, e.g. in claim 1, can mainly be produced in high yields as monomer. In particular, the present inventors found that more than 80, 85, 90 or even 95% of the bispecific antibody obtainable from host cells expressing said bispecific antibody are in the form of a monomer. This is an important feature, since dimers or even multimers are not desirable since they are assumed to have lost most of their binding capabilities to a target cell (via a cell surface molecule) and/or a T cell (via the T cell receptor complex).

In view of the above, the present invention provides an isolated single chain bispecific antibody comprising at least three binding domains, wherein
(a) the first binding domain binds to serum albumin and is positioned at the N-terminus of the second binding domain;
(b) said second binding domain binds to a cell surface molecule on a target cell wherein the surface molecule is a tumor antigen; and
(c) the third binding domain binds to the T cell CD3 receptor complex on a human and a non-human primate cell, wherein the three domains are consecutively on one polypeptide chain in the order from the N-terminus to the C-terminus
   - the first binding domain;
   - the second binding domain; and
   - the third binding domain.

The term "cell surface molecule on a target cell" or "cell surface antigen" as used herein denotes a molecule, which is displayed on the surface of a cell. In most cases, this molecule will be located in or on the plasma membrane of the cell such that at least part of this molecule remains accessible from outside the cell in tertiary form. A non-limiting example of a cell surface molecule, which is located in the plasma membrane is a transmembrane protein comprising, in its tertiary conformation, regions of hydrophilicity and hydrophobicity. Here, at least one hydrophobic region allows the cell surface molecule to be embedded, or inserted in the hydrophobic plasma membrane of the cell while the hydrophilic regions extend on either side of the plasma membrane into the cytoplasm and extracellular space, respectively. Non-limiting examples of cell surface molecules which are located on the plasma membrane are proteins which have been modified at a cysteine residue to bear a palmitoyl group, proteins modified at a C-terminal cysteine residue to bear a farnesyl group or proteins which have been modified at the C-terminus to bear a glycosyl phosphatidyl inositol ("GPI") anchor. These groups allow covalent attachment of proteins to the outer surface of the plasma membrane, where they remain accessible for recognition by extracellular molecules such as antibodies. As apparent from the appended examples, non-limiting examples for cell surface molecules on a target cell are the CD33 molecule and the CEA molecule. Binding domains for CD33 suitable for the herein described format of a bispecific antibody are described in detail e.g. in WO 2008/119567. Corresponding binding domains for CEA suitable for the herein described format of a bispecific antibody are described in detail e.g. in WO 2007/071426.

The T cell CD3 receptor complex is a protein complex and is composed of four distinct chains. In mammals, the complex contains a CD3γ chain, a CD3δ chain, and two CD3ε (epsilon) chains. These chains associate with a molecule known as the T cell receptor (TCR) and the ζ chain to generate an activation signal in T lymphocytes.

The redirected lysis of target cells via the recruitment of T cells by a multispecific, at least bispecific, bispecific antibody involves cytolytic synapse formation and delivery of perforin and granzymes. The engaged T cells are capable of serial target cell lysis, and are not affected by immune escape mechanisms interfering with peptide antigen processing and presentation, or clonal T cell differentiation; see, for example, WO 2007/042261.

The affinity of the second binding domain for a human cell surface molecule on a target cell is preferably ≤100 nM and more preferably ≤50 nM. In a preferred embodiment of the invention the affinity is ≤15 nM, more preferably ≤10 nM, even more preferably ≤5 nM, even more preferably ≤1 nM, even more preferably ≤0.5 nM, even more preferably ≤0.1 nM, and most preferably ≤0.05 nM. The affinity of the second binding domain for a macaque cell surface molecule on a target cell is preferably ≤100 nM and more preferably ≤50 nM. In a preferred embodiment of the invention the affinity is ≤15 nM, more preferably ≤10 nM, even more preferably ≤5 nM, even more preferably ≤1 nM, even more preferably ≤0.5 nM, even more preferably ≤0.1 nM, and most preferably ≤0.05 nM or even ≤0.01 nM. The affinity can be measured for example in a Biacore assay or in a Scatchard assay, e.g. as described in the Examples. The affinity gap for binding to macaque cell surface molecule on a target cell versus human cell surface molecule on a target cell is preferably [1:10-1:5] or [5:1-10:1], more preferably [1:5-5:1], and most preferably [1:2-3:1] or even [1:1-3:1]. Other methods of determining the affinity are well-known to the skilled person.

Human antibodies, respectively human bispecific antibodies, avoid some of the problems associated with antibodies/bispecific antibodies that possess murine or rat variable and/or constant regions. The presence of such murine or rat derived proteins can lead to the rapid clearance of the antibodies/bispecific antibodies or can lead to the generation of an immune response against the antibody/bispecific antibody by a patient. In order to avoid the utilization of murine or rat derived antibodies/bispecific antibodies, human or fully human antibodies can be generated through the introduction of human antibody function into a rodent so that the rodent produces fully human antibodies.

The ability to clone and reconstruct megabase-sized human loci in YACs and to introduce them into the mouse germline provides a powerful approach to elucidating the functional components of very large or crudely mapped loci as well as generating useful models of human disease. Furthermore, the utilization of such technology for substitution of mouse loci with their human equivalents could provide unique insights into the expression and regulation of human gene products during development, their communication with other systems, and their involvement in disease induction and progression.

An important practical application of such a strategy is the "humanization" of the mouse humoral immune system. Introduction of human immunoglobulin (Ig) loci into mice in which the endogenous Ig genes have been inactivated offers the opportunity to study the mechanisms underlying programmed expression and assembly of antibodies as well as their role in B-cell development. Furthermore, such a strategy could provide an ideal source for production of fully human monoclonal antibodies (mAbs)--an important milestone towards fulfilling the promise of antibody therapy in human disease. Fully human antibodies/bispecific antibodies are expected to minimize the immunogenic and allergic responses intrinsic to mouse or mouse-derivatized mAbs and thus to increase the efficacy and safety of the administered antibodies/bispecific antibodies. The use of fully human antibodies/bispecific antibodies can be expected to provide a substantial advantage in the treatment of chronic and recurring human diseases, such as inflammation, autoimmunity, and cancer, which require repeated compound administrations. One approach towards this goal was to engineer mouse strains deficient in mouse antibody production with large fragments of the human Ig loci in anticipation that such mice would produce a large repertoire of human antibodies in the absence of mouse antibodies. Large human Ig fragments would preserve the large variable gene diversity as well as the proper regulation of antibody production and expression. By exploiting the mouse machinery for antibody diversification and selection and the lack of immunological tolerance to human proteins, the reproduced human antibody repertoire in these mouse strains should yield high affinity antibodies against any antigen of interest, including human antigens. Using the hybridoma technology, antigen-specific human mAbs with the desired specificity could be readily produced and selected. This general strategy was demonstrated in connection with our generation of the first XenoMouse mouse strains, as published in 1994. (See Green et al. Nature Genetics 7:13-21 (1994)) The XenoMouse strains were engineered with yeast artificial chromosomes (YACs) containing 245 kb and 190 kb-sized germline configuration fragments of the human heavy chain locus and kappa light chain locus, respectively, which contained core variable and constant region sequences. Id. The human Ig containing YACs proved to be compatible with the mouse system for both rearrangement and expression of antibodies and were capable of substituting for the inactivated mouse Ig genes. This was demonstrated by their ability to induce B-cell development, to produce an adult-like human repertoire of fully human antibodies, and to generate antigen-specific human mAbs. These results also suggested that introduction of larger portions of the human Ig loci containing greater numbers of V genes, additional regulatory elements, and human Ig constant regions might recapitulate substantially the full repertoire that is characteristic of the human humoral response to infection and immunization. The work of Green et al. was recently extended to the introduction of greater than approximately 80% of the human antibody repertoire through introduction of megabase sized, germline configuration YAC fragments of the human heavy chain loci and kappa light chain loci, respectively. See Mendez et al. Nature Genetics 15:146-156 (1997) and U.S. patent application Ser. No. 08/759,620, filed Dec. 3, 1996.

The production of the XenoMouse mice is further discussed and delineated in U.S. patent application Ser. No. 07/466,008, filed Jan. 12, 1990, Ser. No. 07/610,515, filed Nov. 8, 1990, Ser. No. 07/919,297, filed Jul. 24, 1992, Ser. No. 07/922,649, filed Jul. 30, 1992, filed Ser. No. 08/031,801, filed Mar. 15, 1993, Ser. No. 08/112,848, filed Aug. 27, 1993, Ser. No. 08/234,145, filed Apr. 28, 1994, Ser. No. 08/376,279, filed Jan. 20, 1995, Ser. No. 08/430,938, Apr. 27, 1995, Ser. No. 08/464,584, filed Jun. 5, 1995, Ser. No. 08/464,582, filed Jun. 5, 1995, Ser. No. 08/463,191, filed Jun. 5, 1995, Ser. No. 08/462,837, filed Jun. 5, 1995, Ser. No. 08/486,853, filed Jun. 5, 1995, Ser. No. 08/486,857, filed Jun. 5, 1995, Ser. No. 08/486,859, filed Jun. 5, 1995, Ser. No. 08/462,513, filed Jun. 5, 1995, Ser. No. 08/724,752, filed Oct. 2, 1996, and Ser. No. 08/759,620, filed Dec. 3, 1996 and U.S. Pat. Nos. 6,162,963, 6,150,584, 6,114,598, 6,075,181, and 5,939,598 and Japanese Patent Nos. 3 068 180 B2, 3 068 506 B2, and 3 068 507 B2. See also Mendez et al. Nature Genetics 15:146-156 (1997) and Green and Jakobovits J. Exp. Med. 188:483-495 (1998). See also European Patent No., EP 0 463151 B1, grant published Jun. 12, 1996, International Patent Application No., WO 94/02602, published Feb. 3, 1994, International Patent Application No., WO 96/34096, published Oct. 31, 1996, WO 98/24893, published Jun. 11, 1998, WO 00/76310, published Dec. 21, 2000, WO 03/47336. In an alternative approach, others, including GenPharm International, Inc., have utilized a "minilocus" approach. In the minilocus approach, an exogenous Ig locus is mimicked through the inclusion of pieces (individual genes) from the Ig locus. Thus, one or more V.sub.H genes, one or more D.sub.H genes, one or more J.sub.H genes, a mu constant region, and a second constant region (preferably a gamma constant region) are formed into a construct for insertion into an animal. This approach is described in U.S. Pat. No. 5,545,807 to Surani et al. and U.S. Pat. Nos. 5,545,806, 5,625,825, 5,625,126, 5,633,425, 5,661,016, 5,770,429, 5,789,650, 5,814,318, 5,877,397, 5,874,299, and 6,255,458 each to Lonberg and Kay, U.S. Pat. Nos. 5,591,669 and 6,023.010 to Krimpenfort and Berns, U.S. Pat. Nos. 5,612,205, 5,721,367, and 5,789,215 to Berns et al., and U.S. Pat. No. 5,643,763 to Choi and Dunn, and GenPharm International U.S. patent application Ser. No. 07/574,748, filed Aug. 29, 1990, Ser. No. 07/575,962, filed Aug. 31, 1990, Ser. No. 07/810,279, filed Dec. 17, 1991, Ser. No. 07/853,408, filed Mar. 18, 1992, Ser. No. 07/904,068, filed Jun. 23, 1992, Ser. No. 07/990,860, filed Dec. 16, 1992, Ser. No. 08/053,131, filed Apr. 26, 1993, Ser. No. 08/096,762, filed Jul. 22, 1993, Ser. No. 08/155,301, filed Nov. 18, 1993, Ser. No. 08/161,739, filed Dec. 3, 1993, Ser. No. 08/165,699, filed Dec. 10, 1993, Ser. No. 08/209,741, filed Mar. 9, 1994,. See also European Patent No. 0 546 073 B1, International Patent Application Nos. WO 92/03918, WO 92/22645, WO 92/22647, WO 92/22670, WO 93/12227, WO 94/00569, WO 94/25585, WO 96/14436, WO 97/13852, and WO 98/24884 and U.S. Pat. No. 5,981,175. See further Taylor et al., 1992, Chen et al., 1993, Tuaillon et al., 1993, Choi et al., 1993, Lonberg et al., (1994), Taylor et al., (1994), and Tuaillon et al., (1995), Fishwild et al., (1996).

Kirin has also demonstrated the generation of human antibodies from mice in which, through microcell fusion, large pieces of chromosomes, or entire chromosomes, have been introduced. See European Patent Application Nos. 773 288 and 843 961. Xenerex Biosciences is developing a technology for the potential generation of human antibodies. In this technology, SCID mice are reconstituted with human lymphatic cells, e.g., B and/or T cells. Mice are then immunized with an antigen and can generate an immune response against the antigen. See U.S. Pat. Nos. 5,476,996, 5,698,767, and 5,958,765.

Human anti-mouse antibody (HAMA) responses have led the industry to prepare chimeric or otherwise humanized antibodies. While chimeric antibodies have a human constant region and a murine variable region, it is expected that certain human anti-chimeric antibody (HACA) responses will be observed, particularly in chronic or multi-dose utilizations of the antibody. Thus, it would be desirable to provide fully human antibodies against EGFRvIII in order to vitiate concerns and/or effects of HAMA or HACA response.

Cytotoxicity mediated by anti-cell surface molecule/CD3 bispecific bispecific antibodies can be measured in various ways. Effector cells can be e.g. stimulated enriched (human) CD8 positive T cells or unstimulated (human) peripheral blood mononuclear cells (PBMC). If the target cells are of macaque origin or express or are transfected with macaque cell surface molecule, the effector cells should also be of macaque origin such as a macaque T cell line, e.g. 4119LnPx. The target cells should express (at least the extracellular domain of) cell surface molecule, e.g. the human or macaque cell surface molecule. Target cells can be a cell line (such as CHO) which is stably or transiently transfected with the cell surface molecule, e.g. the human or the macaque cell surface molecule. Alternatively, the target cells can be an elected cell surface molecule positive natural expresser cell line. Thus, if e.g. the elected cell surface molecule is CD33, the target cells must express CD33, either as target cells naturally expressing the CD33 molecule or, as described, after transfection of the correseponding gene in the format of an expression vector. Usually EC₅₀-values are expected to be lower with target cell lines expressing higher levels of the elected cell surface molecule on the cell surface. The effector to target cell (E:T) ratio is usually about 10:1, but can also vary. Cytotoxic activity of anti-cell surface molecule/CD3 bispecific bispecific antibodies can be measured in a ⁵¹-chromium release assay (incubation time of about 18 hours) or in a in a FACS-based cytotoxicity assay (incubation time of about 48 hours). Modifications of the assay incubation time (cytotoxic reaction) are also possible. Other methods of measuring cytotoxicity are well-known to the skilled person and comprise MTT or MTS assays, ATP-based assays including bioluminescent
assays, the sulforhodamine B (SRB) assay, WST assay, clonogenic assay and the ECIS technology.

The cytotoxic activity mediated by anti-cell surface molecule/CD3 bispecific bispecific antibodies of the present invention is preferably measured in a cell-based cytotoxicity assay. It is represented by the EC₅₀ value, which corresponds to the half maximal effective concentration (concentration of the bispecific antibody which induces a cytotoxic response halfway between the baseline and maximum). Preferably, the EC₅₀ value of the anti-cell surface molecule/CD3 bispecific bispecific antibodies is ≤20.000 pg/ml, more preferably ≤5000 pg/ml, even more preferably ≤1000 pg/ml, even more preferably ≤500 pg/ml, even more preferably ≤350 pg/ml, even more preferably ≤320 pg/ml, even more preferably ≤250 pg/ml, even more preferably ≤100 pg/ml, even more preferably ≤50 pg/ml, even more preferably ≤10 pg/ml, and most preferably ≤5 pg/ml.

Any of the above given EC₅₀ values can be combined with any one of the indicated scenarios of a cell-based cytotoxicity assay. For example, when (human) CD8 positive T cells or a macaque T cell line are used as effector cells, the EC₅₀ value of the anti-cell surface molecule/CD3 bispecific bispecific antibody is preferably ≤1000 pg/ml, more preferably ≤500 pg/ml, even more preferably ≤250 pg/ml, even more preferably ≤100 pg/ml, even more preferably ≤50 pg/ml, even more preferably ≤10 pg/ml, and most preferably ≤5 pg/ml. If in this assay the target cells are (human or macaque) elected cell surface molecule transfected cells such as CHO cells, the EC₅₀ value of the anti-cell surface molecule/CD3 bispecific bispecific antibody is preferably ≤150 pg/ml, more preferably ≤100 pg/ml, even more preferably ≤50 pg/ml, even more preferably ≤30 pg/ml, even more preferably ≤10 pg/ml, and most preferably ≤5 pg/ml.

If the target cells are elected cell surface molecule positive natural expresser cell line, then the EC₅₀ value is preferably ≤350 pg/ml, more preferably ≤320 pg/ml, even more preferably ≤250 pg/ml, even more preferably ≤200 pg/ml, even more preferably ≤100 pg/ml, even more preferably ≤150 pg/ml, even more preferably ≤100 pg/ml, and most preferably ≤50 pg/ml, or lower.

When (human) PBMCs are used as effector cells, the EC₅₀ value of the anti-cell surface molecule/CD3 bispecific bispecific antibody is preferably ≤1000 pg/ml, more preferably ≤750 pg/ml, more preferably ≤500 pg/ml, even more preferably ≤350 pg/ml, even more preferably ≤320 pg/ml, even more preferably ≤250 pg/ml, even more preferably ≤100 pg/ml, and most preferably ≤50 pg/ml, or lower.

The difference in cytotoxic activity between the monomeric and the dimeric isoform of individual anti-cell surface molecule/CD3 bispecific antibodies is referred to as "potency gap". This potency gap can e.g. be calculated as ratio between EC₅₀ values of the molecule's monomeric and dimeric form. Potency gaps of the anti-cell surface molecule/CD3 bispecific antibodies of the present invention are preferably ≤5, more preferably ≤4, even more preferably ≤3, even more preferably ≤2 and most preferably ≤1.

The binding molecule of the invention is a fusion protein comprising at least two binding domains, with or without peptide linkers (spacer peptides). Among the suitable peptide linkers are those described in U.S. Patents 4,751,180 and 4,935,233 or WO 88/09344. Another method for preparing oligomeric antibody constuct derivatives involves use of a leucine zipper. Leucine zipper domains are peptides that promote oligomerization of the proteins in which they are found. Leucine zippers were originally identified in several DNA-binding proteins (Landschulz et al., 1988, Science 240:1759), and have since been found in a variety of different proteins. Among the known leucine zippers are naturally occurring peptides and derivatives thereof that dimerize or trimerize. Examples of leucine zipper domains suitable for producing soluble oligomeric proteins are described in PCT application WO 94/10308, and the leucine zipper derived from lung surfactant protein D (SPD) described in Hoppe et al., 1994, FEBS Letters 344:191. The use of a modified leucine zipper that allows for stable trimerization of a heterologous protein fused thereto is described in Fanslow et al., 1994, Semin. Immunol. 6:267-78. In one approach, recombinant fusion proteins comprising elected cell surface molecule antibody fragment or derivative fused to a leucine zipper peptide are expressed in suitable host cells, and the soluble oligomeric elected cell surface molecule antibody fragments or derivatives that form are recovered from the culture supernatant.

Covalent modifications of antigen binding proteins are included within the scope of this invention, and are generally, but not always, done post-translationally. For example, several types of covalent modifications of the antigen binding protein are introduced into the molecule by reacting specific amino acid residues of the antigen binding protein with an organic derivatizing agent that is capable of reacting with selected side chains or the N- or C-terminal residues.

Cysteinyl residues most commonly are reacted with α-haloacetates (and corresponding amines), such as chloroacetic acid or chloroacetamide, to give carboxymethyl or carboxyamidomethyl derivatives. Cysteinyl residues also are derivatized by reaction with bromotrifluoroacetone, α-bromo-β-(5-imidozoyl)propionic acid, chloroacetyl phosphate, N-alkylmaleimides, 3-nitro-2-pyridyl disulfide, methyl 2-pyridyl disulfide, p-chloromercuribenzoate, 2-chloromercuri-4-nitrophenol, or chloro-7-nitrobenzo-2-oxa-1,3-diazole.

Histidyl residues are derivatized by reaction with diethylpyrocarbonate at pH 5.5-7.0 because this agent is relatively specific for the histidyl side chain. Para-bromophenacyl bromide also is useful; the reaction is preferably performed in 0.1 M sodium cacodylate at pH 6.0.

Lysinyl and amino terminal residues are reacted with succinic or other carboxylic acid anhydrides. Derivatization with these agents has the effect of reversing the charge of the lysinyl residues. Other suitable reagents for derivatizing alpha-amino-containing residues include imidoesters such as methyl picolinimidate; pyridoxal phosphate; pyridoxal; chloroborohydride; trinitrobenzenesulfonic acid; O-methylisourea; 2,4-pentanedione; and transaminase-catalyzed reaction with glyoxylate.

Arginyl residues are modified by reaction with one or several conventional reagents, among them phenylglyoxal, 2,3-butanedione, 1,2-cyclohexanedione, and ninhydrin. Derivatization of arginine residues requires that the reaction be performed in alkaline conditions because of the high pKa of the guanidine functional group. Furthermore, these reagents may react with the groups of lysine as well as the arginine epsilon-amino group.

The specific modification of tyrosyl residues may be made, with particular interest in introducing spectral labels into tyrosyl residues by reaction with aromatic diazonium compounds or tetranitromethane. Most commonly, N-acetylimidizole and tetranitromethane are used to form O-acetyl tyrosyl species and 3-nitro derivatives, respectively. Tyrosyl residues are iodinated using ¹²⁵I or ¹³¹I to prepare labeled proteins for use in radioimmunoassay, the chloramine T method described above being suitable.

Carboxyl side groups (aspartyl or glutamyl) are selectively modified by reaction with carbodiimides (R'-N=C=N--R'), where R and R' are optionally different alkyl groups, such as 1-cyclohexyl-3-(2-morpholinyl-4-ethyl) carbodiimide or 1-ethyl-3-(4-azonia-4,4-dimethylpentyl) carbodiimide. Furthermore, aspartyl and glutamyl residues are converted to asparaginyl and glutaminyl residues by reaction with ammonium ions.

Derivatization with bifunctional agents is useful for crosslinking antigen binding proteins to a water-insoluble support matrix or surface for use in a variety of methods. Commonly used crosslinking agents include, e.g., 1,1-bis(diazoacetyl)-2-phenylethane, glutaraldehyde, N-hydroxysuccinimide esters, for example, esters with 4-azidosalicylic acid, homobifunctional imidoesters, including disuccinimidyl esters such as 3,3'-dithiobis(succinimidylpropionate), and bifunctional maleimides such as bis-N-maleimido-1,8-octane. Derivatizing agents such as methyl-3-[(p-azidophenyl)dithio]propioimidate yield photoactivatable intermediates that are capable of forming crosslinks in the presence of light. Alternatively, reactive water-insoluble matrices such as cyanogen bromide-activated carbohydrates and the reactive substrates described in U.S. Pat. Nos. 3,969,287; 3,691,016; 4,195,128; 4,247,642; 4,229,537; and 4,330,440 are employed for protein immobilization.

Glutaminyl and asparaginyl residues are frequently deamidated to the corresponding glutamyl and aspartyl residues, respectively. Alternatively, these residues are deamidated under mildly acidic conditions. Either form of these residues falls within the scope of this invention.

Other modifications include hydroxylation of proline and lysine, phosphorylation of hydroxyl groups of seryl or threonyl residues, methylation of the α-amino groups of lysine, arginine, and histidine side chains (T. E. Creighton, Proteins: Structure and Molecular Properties, W. H. Freeman & Co., San Francisco, 1983, pp. 79-86), acetylation of the N-terminal amine, and amidation of any C-terminal carboxyl group.

Another type of covalent modification of the antigen binding protein included within the scope of this invention comprises altering the glycosylation pattern of the protein. As is known in the art, glycosylation patterns can depend on both the sequence of the protein (e.g., the presence or absence of particular glycosylation amino acid residues, discussed below), or the host cell or organism in which the protein is produced. Particular expression systems are discussed below. Glycosylation of polypeptides is typically either N-linked or O-linked. N-linked refers to the attachment of the carbohydrate moiety to the side chain of an asparagine residue. The tripeptide sequences asparagine-X-serine and asparagine-X-threonine, where X is any amino acid except proline, are the recognition sequences for enzymatic attachment of the carbohydrate moiety to the asparagine side chain. Thus, the presence of either of these tripeptide sequences in a polypeptide creates a potential glycosylation site. O-linked glycosylation refers to the attachment of one of the sugars N-acetylgalactosamine, galactose, or xylose, to a hydroxyamino acid, most commonly serine or threonine, although 5-hydroxyproline or 5-hydroxylysine may also be used.

Addition of glycosylation sites to the antigen binding protein is conveniently accomplished by altering the amino acid sequence such that it contains one or more of the above-described tripeptide sequences (for N-linked glycosylation sites). The alteration may also be made by the addition of, or substitution by, one or more serine or threonine residues to the starting sequence (for O-linked glycosylation sites). For ease, the antigen binding protein amino acid sequence is preferably altered through changes at the DNA level, particularly by mutating the DNA encoding the target polypeptide at preselected bases such that codons are generated that will translate into the desired amino acids.

Another means of increasing the number of carbohydrate moieties on the antigen binding protein is by chemical or enzymatic coupling of glycosides to the protein. These procedures are advantageous in that they do not require production of the protein in a host cell that has glycosylation capabilities for N- and O-linked glycosylation. Depending on the coupling mode used, the sugar(s) may be attached to (a) arginine and histidine, (b) free carboxyl groups, (c) free sulfhydryl groups such as those of cysteine, (d) free hydroxyl groups such as those of serine, threonine, or hydroxyproline, (e) aromatic residues such as those of phenylalanine, tyrosine, or tryptophan, or (f) the amide group of glutamine. These methods are described in WO 87/05330 published Sep. 11, 1987, and in Aplin and Wriston, 1981, CRC Crit. Rev. Biochem., pp. 259-306.

Removal of carbohydrate moieties present on the starting antigen binding protein may be accomplished chemically or enzymatically. Chemical deglycosylation requires exposure of the protein to the compound trifluoromethanesulfonic acid, or an equivalent compound. This treatment results in the cleavage of most or all sugars except the linking sugar (N-acetylglucosamine or N-acetylgalactosamine), while leaving the polypeptide intact. Chemical deglycosylation is described by Hakimuddin et al., 1987, Arch. Biochem. Biophys. 259:52 and by Edge et al., 1981, Anal. Biochem. 118:131. Enzymatic cleavage of carbohydrate moieties on polypeptides can be achieved by the use of a variety of endo- and exo-glycosidases as described by Thotakura et al., 1987, Meth. Enzymol. 138:350. Glycosylation at potential glycosylation sites may be prevented by the use of the compound tunicamycin as described by Duskin et al., 1982, J. Biol. Chem. 257:3105. Tunicamycin blocks the formation of protein-N-glycoside linkages.

Another type of covalent modification of the antigen binding protein comprises linking the antigen binding protein to various non-proteinaceous polymers, including, but not limited to, various polyols such as polyethylene glycol, polypropylene glycol or polyoxyalkylenes, in the manner set forth in U.S. Pat. Nos. 4,640,835; 4,496,689; 4,301,144; 4,670,417; 4,791,192 or 4,179,337. In addition, as is known in the art, amino acid substitutions may be made in various positions within the antigen binding protein to facilitate the addition of polymers such as PEG.

In some embodiments, the covalent modification of the antigen binding proteins of the invention comprises the addition of one or more labels.

The term "labelling group" means any detectable label. Examples of suitable labelling groups include, but are not limited to, the following: radioisotopes or radionuclides (e.g., ³H, ¹⁴C, ¹⁵N, ³⁵S, ⁹⁰Y, ⁹⁹Tc, ¹¹¹In, ¹²⁵I, ¹³¹I), fluorescent groups (e.g., FITC, rhodamine, lanthanide phosphors), enzymatic groups (e.g., horseradish peroxidase, β-galactosidase, luciferase, alkaline phosphatase), chemiluminescent groups, biotinyl groups, or predetermined polypeptide epitopes recognized by a secondary reporter (e.g., leucine zipper pair sequences, binding sites for secondary antibodies, metal binding domains, epitope tags). In some embodiments, the labelling group is coupled to the antigen binding protein *via* spacer arms of various lengths to reduce potential steric hindrance. Various methods for labelling proteins are known in the art and may be used in performing the present invention.

In general, labels fall into a variety of classes, depending on the assay in which they are to be detected: a) isotopic labels, which may be radioactive or heavy isotopes; b) magnetic labels (e.g., magnetic particles); c) redox active moieties; d) optical dyes; enzymatic groups (e.g. horseradish peroxidase, β-galactosidase, luciferase, alkaline phosphatase); e) biotinylated groups; and f) predetermined polypeptide epitopes recognized by a secondary reporter (e.g., leucine zipper pair sequences, binding sites for secondary antibodies, metal binding domains, epitope tags, etc.). In some embodiments, the labelling group is coupled to the antigen binding protein *via* spacer arms of various lengths to reduce potential steric hindrance. Various methods for labelling proteins are known in the art and may be used in performing the present invention. Specific labels include optical dyes, including, but not limited to, chromophores, phosphors and fluorophores, with the latter being specific in many instances. Fluorophores can be either "small molecule" fluores, or proteinaceous fluores.

By "fluorescent label" is meant any molecule that may be detected *via* its inherent fluorescent properties. Suitable fluorescent labels include, but are not limited to, fluorescein, rhodamine, tetramethylrhodamine, eosin, erythrosin, coumarin, methyl-coumarins, pyrene, Malacite green, stilbene, Lucifer Yellow, Cascade BlueJ, Texas Red, IAEDANS, EDANS, BODIPY FL, LC Red 640, Cy 5, Cy 5.5, LC Red 705, Oregon green, the Alexa-Fluor dyes (Alexa Fluor 350, Alexa Fluor 430, Alexa Fluor 488, Alexa Fluor 546, Alexa Fluor 568, Alexa Fluor 594, Alexa Fluor 633, Alexa Fluor 660, Alexa Fluor 680), Cascade Blue, Cascade Yellow and R-phycoerythrin (PE) (Molecular Probes, Eugene, OR), FITC, Rhodamine, and Texas Red (Pierce, Rockford, IL), Cy5, Cy5.5, Cy7 (Amersham Life Science, Pittsburgh, PA). Suitable optical dyes, including fluorophores, are described in Molecular Probes Handbook by Richard P. Haugland.

Suitable proteinaceous fluorescent labels also include, but are not limited to, green fluorescent protein, including a Renilla, Ptilosarcus, or Aequorea species of GFP (Chalfie et al., 1994, Science 263:802-805), EGFP (Clontech Laboratories, Inc., Genbank Accession Number U55762), blue fluorescent protein (BFP, Quantum Biotechnologies, Inc. 1801 de Maisonneuve Blvd. West, 8th Floor, Montreal, Quebec, Canada H3H 1J9; Stauber, 1998, Biotechniques 24:462-471; Heim et al., 1996, Curr. Biol. 6:178-182), enhanced yellow fluorescent protein (EYFP, Clontech Laboratories, Inc.), luciferase (Ichiki et al., 1993, J. Immunol. 150:5408-5417), β galactosidase (Nolan et al., 1988, Proc. Natl. Acad. Sci. U.S.A. 85:2603-2607) and Renilla (WO92/15673, WO95/07463, WO98/14605, WO98/26277, WO99/49019, U.S. Patent Nos. 5292658, 5418155, 5683888, 5741668, 5777079, 5804387, 5874304, 5876995, 5925558).

In one embodiment the bispecific antibody of the invention is characterized in a way that the three domains are consecutively on one polypeptide chain in the order from the N-terminus to the C-terminus
- the first binding domain;
- the second binding domain; and
- the third binding domain.

The invention also provides a single chain bispecific antibody comprising at least three binding domains comprised in one polypeptide chain, wherein
(a) the first domain is capable of binding to serum albumin and is positioned at the N-terminus of the second binding domain;
(b) said second domain is capable of binding to a cell surface molecule on a target cell; and
(c) the third domain is capable of binding to the T cell CD3 receptor complex,
wherein the yield of expressible monomeric bispecific antibody in relation to the total amount of bispecific antibody isolated from the culture supernatant of host cells producing the bispecific antibody depends on the order of the first and second binding domain in said bispecific antibody.

The invention further provides a single chain bispecific antibody comprising at least three binding domains comprised in one polypeptide chain in the order first domain, second domain and third domain, wherein
(a) the first domain is capable of binding to serum albumin and is positioned at the N-terminus of the second binding domain;
(b) said second domain is capable of binding to a cell surface molecule on a target cell; and
(c) the third domain is capable of binding to the T cell CD3 receptor complex;
wherein the yield of monomeric bispecific antibody isolated from the culture supernatant of host cells producing the bispecific antibody is at least 1.5 times higher than the yield of monomeric bispecific antibody isolated from the culture supernatant of host cells producing a bispecific antibody comprising the binding domain capable of binding to serum albumin is at the C-terminus of the molecule.

It is further preferred that this yield of monomeric bispecific antibodies is 2 fold higher, more preferably, 2.5 fold higher, even more preferred 3 fold, 4 fold or 5 fold higher.

In one embodiment the bispecific antibody of the invention is characterized in a way that at least one of the binding domains, preferably the second and/or third binding domain, is an scFv or single domain antibody.

Also in one embodiment of the bispecific antibody of the invention the molecule comprises one or more further heterologous polypeptide.

A bispecific antibody of the invention may also comprise a His-tag as a heterologus poypeptide. It is preferred for the bispecific antibody of the invention that the His-tag is positioned at the C-terminus of the third binding domain.

The bispecific antibody of the invention may also comprise additional domains, which e.g. are helpful in the isolation of the molecule or relate to an adapted pharmacokinetic profile of the molecule.

Domains helpful for the isolation of a bispecific antibody may be elected from peptide motives or secondarily introduced moieties, which can be captured in an isolation method, e.g. an isolation column. A non-limiting embodiments of such additional domains comprise peptide motives known as Myc-tag, HAT-tag, HA-tag, TAP-tag, GST-tag, chitin binding domain (CBD-tag), maltose binding protein (MBP-tag), Flag-tag, Strep-tag and variants thereof (e.g. Strepll-tag) and His-tag. All herein disclosed bispecific antibodies characterized by the identified CDRs are preferred to comprise a His-tag domain, which is generally known as a repeat of consecutive His residues in the amino acid sequence of a molecule, preferably of six His residues.

As apparent from the appended examples, it appears that the yield of monomeric bispecific antibody of the invention isolated from the cell supernatant of host cells expressing the bispecific antibody may be further increased by using bispecific antibodies, which do not comprise a His-tag. Thus, without being bound by theory, the electing a bispecific antibody of the invention, which does not comprise a His-tag domain can result in further increasing the yield of monomeric bispecific antibodies from the cell supernatant of host cells expressing the bispecific antibody. Accordingly, bispecific antibodies which do not comprise a His-tag are alternatively preferred.

The invention also provides a bispecific antibody, wherein
(a) the first binding domain binds to human and non-human primate serum albumin comprises SEQ ID Nos. 2, 4 or 6, and is positioned at the N-terminus of the second binding domain;
(b) the second binding domain binds to the cell surface molecule on a human and a non-human primate cell wherein the surface molecule is a tumor antigen, and
(c) the third binding domain is binds to the T cell CD3 receptor complex on a human and a non-human primate cell,
wherein the three domains are consecutively on one polypeptide chain in the order from the N-terminus to the C-terminus
- the first binding domain;
- the second binding domain; and
- the third binding domain.

In one embodiment the bispecific antibody according to the invention is characterized that the first binding domain capable of binding to serum albumin is derived from a combinatorial library or an antibody binding domain.

In one embodiment of the bispecific antibody of the invention the first binding domain capable of binding to serum albumin is derived from a CDR of a single domain antibody.

In one embodiment of the bispecific antibody of the invention
- the second binding domain is binding to the cell surface molecule on a target cell with an affinity (KD) of ≤ 100 nM; and
- the third binding domain is binding to the T cell CD3 receptor complex with an affinity (KD) of ≤ 100nM.

In one embodiment of the bispecific antibody of the invention the bispecific antibody shows cytotoxic activity in an in vitro assay measuring the lysis of target cells by effector cells in the presence of 10% human serum albumin.

In a preferred embodiment of the bispecific antibody of the invention the molecule consists of a single polypeptide chain.

In one embodiment of the bispecific antibody of the invention
(a) the second binding domain comprises an antibody derived VL and VH chain; and/or
(b) the third binding domain comprises an antibody derived VL and VH chain.

Also in one embodiment of the bispecific antibody of the invention the molecule comprises one or more further heterologous polypeptide.

In one embodiment of the bispecific antibody of the invention the first binding domain capable of binding to a cell surface molecule is binding to a tumor antigen.

In a preferred embodiment of the bispecific antibody of the invention the third binding domain capable of binding to the T cell CD3 receptor complex is capable of binding to an epitope of human and Callithrix jacchus, Saguinus oedipus or Saimiri sciureus CD3ε chain, wherein the epitope is part of an amino acid sequence comprised in the group consisting of SEQ ID NOs: 2, 4, 6, or 8 WO 2008/119567 and comprises at least the amino acid sequence Gln-Asp-Gly-Asn-Glu.

In one aspect of the invention, the third binding domain is capable of binding to to human CD3 and to macaque CD3, preferably to human CD3 epsilon and to macaque CD3 epsilon. Additionally or alternatively, the third binding domain is capable of binding to *Callithrix jacchus, Saguinus oedipus* and/or *Saimiri sciureus* CD3 epsilon. According to these embodiments, one or both binding domains of the bispecific antibody of the invention are preferably cross-species specific for members of the mammalian order of primates. Cross-species specific CD3 binding domains are, for example, described in WO 2008/119567.

It is particularly preferred for the bispecific antibody of the present invention that the third binding domain capable of binding to the T cell CD3 receptor complex comprises a VL region comprising CDR-L1, CDR-L2 and CDR-L3 selected from:
(a) CDR-L1 as depicted in SEQ ID NO: 27 of WO 2008/119567, CDR-L2 as depicted in SEQ ID NO: 28 of WO 2008/119567 and CDR-L3 as depicted in SEQ ID NO: 29 of WO 2008/119567;
(b) CDR-L1 as depicted in SEQ ID NO: 117 of WO 2008/119567, CDR-L2 as depicted in SEQ ID NO: 118 of WO 2008/119567 and CDR-L3 as depicted in SEQ ID NO: 119 of WO 2008/119567; and
(c) CDR-L1 as depicted in SEQ ID NO: 153 of WO 2008/119567, CDR-L2 as depicted in SEQ ID NO: 154 of WO 2008/119567 and CDR-L3 as depicted in SEQ ID NO: 155 of WO 2008/119567.

In an alternatively preferred embodiment of the bispecific antibody of the present invention, the third binding domain capable of binding to the T cell CD3 receptor complex comprises a VH region comprising CDR-H 1, CDR-H2 and CDR-H3 selected from:
(a) CDR-H1 as depicted in SEQ ID NO: 12 of WO 2008/119567, CDR-H2 as depicted in SEQ ID NO: 13 of WO 2008/119567 and CDR-H3 as depicted in SEQ ID NO: 14 of WO 2008/119567;
(b) CDR-H1 as depicted in SEQ ID NO: 30 of WO 2008/119567, CDR-H2 as depicted in SEQ ID NO: 31 of WO 2008/119567 and CDR-H3 as depicted in SEQ ID NO: 32 of WO 2008/119567;
(c) CDR-H1 as depicted in SEQ ID NO: 48 of WO 2008/119567, CDR-H2 as depicted in SEQ ID NO: 49 of WO 2008/119567 and CDR-H3 as depicted in SEQ ID NO: 50 of WO 2008/119567;
(d) CDR-H1 as depicted in SEQ ID NO: 66 of WO 2008/119567, CDR-H2 as depicted in SEQ ID NO: 67 of WO 2008/119567 and CDR-H3 as depicted in SEQ ID NO: 68 of WO 2008/119567;
(e) CDR-H1 as depicted in SEQ ID NO: 84 of WO 2008/119567, CDR-H2 as depicted in SEQ ID NO: 85 of WO 2008/119567 and CDR-H3 as depicted in SEQ ID NO: 86 of WO 2008/119567;
(f) CDR-H1 as depicted in SEQ ID NO: 102 of WO 2008/119567, CDR-H2 as depicted in SEQ ID NO: 103 of WO 2008/119567 and CDR-H3 as depicted in SEQ ID NO: 104 of WO 2008/119567;
(g) CDR-H1 as depicted in SEQ ID NO: 120 of WO 2008/119567, CDR-H2 as depicted in SEQ ID NO: 121 of WO 2008/119567 and CDR-H3 as depicted in SEQ ID NO: 122 of WO 2008/119567;
(h) CDR-H1 as depicted in SEQ ID NO: 138 of WO 2008/119567, CDR-H2 as depicted in SEQ ID NO: 139 of WO 2008/119567 and CDR-H3 as depicted in SEQ ID NO: 140 of WO 2008/119567;
(i) CDR-H1 as depicted in SEQ ID NO: 156 of WO 2008/119567, CDR-H2 as depicted in SEQ ID NO: 157 of WO 2008/119567 and CDR-H3 as depicted in SEQ ID NO: 158 of WO 2008/119567; and
(j) CDR-H1 as depicted in SEQ ID NO: 174 of WO 2008/119567, CDR-H2 as depicted in SEQ ID NO: 175 of WO 2008/119567 and CDR-H3 as depicted in SEQ ID NO: 176 of WO 2008/119567.

It is further preferred for the bispecific antibody of the present invention that the third binding domain capable of binding to the T cell CD3 receptor complex comprises a VL region selected from the group consisting of a VL region as depicted in SEQ ID NO: 35, 39, 125, 129, 161 or 165 of WO 2008/119567.

It is alternatively preferred that the third binding domain capable of binding to the T cell CD3 receptor complex comprises a VH region selected from the group consisting of a VH region as depicted in SEQ ID NO: 15, 19, 33, 37, 51, 55, 69, 73, 87, 91, 105, 109, 123, 127, 141, 145, 159, 163, 177 or 181 of WO 2008/119567.

More preferably, the bispecific antibody of the present invention is characterized by the third binding domain capable of binding to the T cell CD3 receptor complex comprising a VL region and a VH region selected from the group consisting of:
(a) a VL region as depicted in SEQ ID NO: 17 or 21 of WO 2008/119567 and a VH region as depicted in SEQ ID NO: 15 or 19 of WO 2008/119567;
(b) a VL region as depicted in SEQ ID NO: 35 or 39 of WO 2008/119567 and a VH region as depicted in SEQ ID NO: 33 or 37 of WO 2008/119567;
(c) a VL region as depicted in SEQ ID NO: 53 or 57 of WO 2008/119567 and a VH region as depicted in SEQ ID NO: 51 or 55 of WO 2008/119567;
(d) a VL region as depicted in SEQ ID NO: 71 or 75 of WO 2008/119567 and a VH region as depicted in SEQ ID NO: 69 or 73 of WO 2008/119567;
(e) a VL region as depicted in SEQ ID NO: 89 or 93 of WO 2008/119567 and a VH region as depicted in SEQ ID NO: 87 or 91 of WO 2008/119567;
(f) a VL region as depicted in SEQ ID NO: 107 or 111 of WO 2008/119567 and a VH region as depicted in SEQ ID NO: 105 or 109 of WO 2008/119567;
(g) a VL region as depicted in SEQ ID NO: 125 or 129 of WO 2008/119567 and a VH region as depicted in SEQ ID NO: 123 or 127 of WO 2008/119567;
(h) a VL region as depicted in SEQ ID NO: 143 or 147 of WO 2008/119567 and a VH region as depicted in SEQ ID NO: 141 or 145 of WO 2008/119567;
(i) a VL region as depicted in SEQ ID NO: 161 or 165 of WO 2008/119567 and a VH region as depicted in SEQ ID NO: 159 or 163 of WO 2008/119567; and
(j) a VL region as depicted in SEQ ID NO: 179 or 183 of WO 2008/119567 and a VH region as depicted in SEQ ID NO: 177 or 181 of WO 2008/119567.

According to a preferred embodiment of the bispecific antibody of the present invention, in particular the third binding domain capable of binding to the T cell CD3 receptor complex, the pairs of VH-regions and VL-regions are in the format of a single chain antibody (scFv). The VH and VL regions are arranged in the order VH-VL or VL-VH. It is preferred that the VH-region is positioned N-terminally to a linker sequence. The VL-region is positioned C-terminally of the linker sequence.

A preferred embodiment of the above described bispecific antibody of the present invention is characterized by the third binding domain capable of binding to the T cell CD3 receptor complex comprising an amino acid sequence selected from the group consisting of SEQ ID NOs: 23, 25, 41, 43, 59, 61, 77, 79, 95, 97, 113, 115, 131, 133, 149, 151, 167, 169, 185 or 187 of WO 2008/119567.

In one embodiment the bispecific antibody of the invention is characterized by an amino acid sequence as depicted in SEQ ID NOs: 8, 12, 16, 20, 24, 26, 30, or 34.

An alternative embodiment of the invention provides a method for the production of bispecific antibody of the invention, the method comprising the step of:
- selecting for bispecific antibodies comprising a binding domain, which binds to a cell surface molecule on a target cell, comprising at the N-terminus a binding domain which is capable of binding to serum albumin.

In one embodiment the method of the invention further comprises the step of:
- adding to the molecule an additional binding domain, which binds to the T cell CD3 receptor complex.

The invention further provides a nucleic acid molecule having a sequence encoding a bispecific antibody of the invention.

Furthermore, the invention provides a vector comprising a nucleic acid sequence of the invention. Moreover, the invention provides a host cell transformed or transfected with the nucleic acid sequence of the invention.

In one embodiment the invention provides a process for the production of a bispecific antibody of the invention or produced by a method of the invention, said process comprising culturing a host cell of the invention under conditions allowing the expression of the bispecific antibody a of the invention or produced by a method of the invention and recovering the produced bispecific antibody from the culture.

Moreover, the invention provides a pharmaceutical composition comprising a bispecific antibody of the invention or produced according to the process of the invention

The formulations described herein are useful as pharmaceutical compositions in the treatment, amelioration and/or prevention of the pathological medical condition as described herein in a patient in need thereof. The term "treatment" refers to both therapeutic treatment and prophylactic or preventative measures. Treatment includes the application or administration of the formulation to the body, an isolated tissue, or cell from a patient who has a disease/disorder, a symptom of a disease/disorder, or a predisposition toward a disease/disorder, with the purpose to cure, heal, alleviate, relieve, alter, remedy, ameliorate, improve, or affect the disease, the symptom of the disease, or the predisposition toward the disease.

Those "in need of treatment" include those already with the disorder, as well as those in which the disorder is to be prevented. The term "disease" is any condition that would benefit from treatment with the protein formulation described herein. This includes chronic and acute disorders or diseases including those pathological conditions that predispose the mammal to the disease in question. Non-limiting examples of diseases/disorders to be treated herein include proliferative disease, a tumorous disease, or an immunological disorder.

In some embodiments, the invention provides a pharmaceutical composition comprising a therapeutically effective amount of one or a plurality of the bispecific antibody of the invention together with a pharmaceutically effective diluents, carrier, solubilizer, emulsifier, preservative, and/or adjuvant. Pharmaceutical compositions of the invention include, but are not limited to, liquid, frozen, and lyophilized compositions.

Preferably, formulation materials are nontoxic to recipients at the dosages and concentrations employed. In specific embodiments, pharmaceutical compositions comprising a therapeutically effective amount of an bispecific antibody of the invention.

In certain embodiments, the pharmaceutical composition may contain formulation materials for modifying, maintaining or preserving, for example, the pH, osmolarity, viscosity, clarity, color, isotonicity, odor, sterility, stability, rate of dissolution or release, adsorption or penetration of the composition. In such embodiments, suitable formulation materials include, but are not limited to, amino acids (such as glycine, glutamine, asparagine, arginine, proline, or lysine); antimicrobials; antioxidants (such as ascorbic acid, sodium sulfite or sodium hydrogen-sulfite); buffers (such as borate, bicarbonate, Tris-HCI, citrates, phosphates or other organic acids); bulking agents (such as mannitol or glycine); chelating agents (such as ethylenediamine tetraacetic acid (EDTA)); complexing agents (such as caffeine, polyvinylpyrrolidone, beta-cyclodextrin or hydroxypropyl-beta-cyclodextrin); fillers; monosaccharides; disaccharides; and other carbohydrates (such as glucose, mannose or dextrins); proteins (such as serum albumin, gelatin or immunoglobulins); coloring, flavoring and diluting agents; emulsifying agents; hydrophilic polymers (such as polyvinylpyrrolidone); low molecular weight polypeptides; salt-forming counterions (such as sodium); preservatives (such as benzalkonium chloride, benzoic acid, salicylic acid, thimerosal, phenethyl alcohol, methylparaben, propylparaben, chlorhexidine, sorbic acid or hydrogen peroxide); solvents (such as glycerin, propylene glycol or polyethylene glycol); sugar alcohols (such as mannitol or sorbitol); suspending agents; surfactants or wetting agents (such as pluronics, PEG, sorbitan esters, polysorbates such as polysorbate 20, polysorbate, triton, tromethamine, lecithin, cholesterol, tyloxapal); stability enhancing agents (such as sucrose or sorbitol); tonicity enhancing agents (such as alkali metal halides, preferably sodium or potassium chloride, mannitol sorbitol); delivery vehicles; diluents; excipients and/or pharmaceutical adjuvants. See, REMINGTON'S PHARMACEUTICAL SCIENCES, 18" Edition, (A. R. Genrmo, ed.), 1990, Mack Publishing Company.

In certain embodiments, the optimal pharmaceutical composition will be determined by one skilled in the art depending upon, for example, the intended route of administration, delivery format and desired dosage. See, for example, REMINGTON'S PHARMACEUTICAL SCIENCES, supra. In certain embodiments, such compositions may influence the physical state, stability, rate of in vivo release and rate of in vivo clearance of the antigen binding proteins of the invention. In certain embodiments, the primary vehicle or carrier in a pharmaceutical composition may be either aqueous or non-aqueous in nature. For example, a suitable vehicle or carrier may be water for injection, physiological saline solution or artificial cerebrospinal fluid, possibly supplemented with other materials common in compositions for parenteral administration. Neutral buffered saline or saline mixed with serum albumin are further exemplary vehicles. In specific embodiments, pharmaceutical compositions comprise Tris buffer of about pH 7.0-8.5, or acetate buffer of about pH 4.0-5.5, and may further include sorbitol or a suitable substitute therefore. In certain embodiments of the invention, human antibody or antigen binding fragment thereof of the invention or the bispecific antibody of the invention compositions may be prepared for storage by mixing the selected composition having the desired degree of purity with optional formulation agents (REMINGTON'S PHARMACEUTICAL SCIENCES, supra) in the form of a lyophilized cake or an aqueous solution. Further, in certain embodiments, the human antibody or antigen binding fragment thereof of the invention or the bispecific antibody of the invention may be formulated as a lyophilizate using appropriate excipients such as sucrose.

The pharmaceutical compositions of the invention can be selected for parenteral delivery. Alternatively, the compositions may be selected for inhalation or for delivery through the digestive tract, such as orally. Preparation of such pharmaceutically acceptable compositions is within the skill of the art. The formulation components are present preferably in concentrations that are acceptable to the site of administration. In certain embodiments, buffers are used to maintain the composition at physiological pH or at a slightly lower pH, typically within a pH range of from about 5 to about 8.

When parenteral administration is contemplated, the therapeutic compositions for use in this invention may be provided in the form of a pyrogen-free, parenterally acceptable aqueous solution comprising the desired human antibody or antigen binding fragment thereof of the invention or the bispecific antibody of the invention in a pharmaceutically acceptable vehicle. A particularly suitable vehicle for parenteral injection is sterile distilled water in which the bispecific antibody of the invention is formulated as a sterile, isotonic solution, properly preserved. In certain embodiments, the preparation can involve the formulation of the desired molecule with an agent, such as injectable microspheres, bio-erodible particles, polymeric compounds (such as polylactic acid or polyglycolic acid), beads or liposomes, that may provide controlled or sustained release of the product which can be delivered via depot injection. In certain embodiments, hyaluronic acid may also be used, having the effect of promoting sustained duration in the circulation. In certain embodiments, implantable drug delivery devices may be used to introduce the desired antigen binding protein.

Additional pharmaceutical compositions will be evident to those skilled in the art, including formulations involving the bispecific antibody of the invention in sustained- or controlled-delivery formulations. Techniques for formulating a variety of other sustained- or controlled-delivery means, such as liposome carriers, bio-erodible microparticles or porous beads and depot injections, are also known to those skilled in the art. See, for example, International Patent Application No. PCT/US93/00829 describes controlled release of porous polymeric microparticles for delivery of pharmaceutical compositions. Sustained-release preparations may include semipermeable polymer matrices in the form of shaped articles, e.g., films, or microcapsules. Sustained release matrices may include polyesters, hydrogels, polylactides (as disclosed in U.S. Pat. No. 3,773,919 and European Patent Application Publication No. EP 058481), copolymers of L-glutamic acid and gamma ethyl-L-glutamate (Sidman et al., 1983, Biopolymers 2:547-556), poly (2-hydroxyethyl-methacrylate) (Langer et al., 1981, J. Biomed. Mater. Res. 15:167-277 and Langer, 1982, Chem. Tech. 12:98-105), ethylene vinyl acetate (Langer et al., 1981, supra) or poly-D(-)-3-hydroxybutyric acid (European Patent Application Publication No. EP 133,988). Sustained release compositions may also include liposomes that can be prepared by any of several methods known in the art. See, e.g., Eppstein et al., 1985, Proc. Natl. Acad. Sci. U.S.A. 82:3688-3692; European Patent Application Publication Nos. EP 036,676; EP 088,046 and EP 143,949.

Pharmaceutical compositions used for in vivo administration are typically provided as sterile preparations. Sterilization can be accomplished by filtration through sterile filtration membranes. When the composition is lyophilized, sterilization using this method may be conducted either prior to or following lyophilization and reconstitution. Compositions for parenteral administration can be stored in lyophilized form or in a solution. Parenteral compositions generally are placed into a container having a sterile access port, for example, an intravenous solution bag or vial having a stopper pierceable by a hypodermic injection needle.

Aspects of the invention includes self-buffering bispecific antibody of the invention formulations, which can be used as pharmaceutical compositions, as described in international patent application WO 06138181A2 (PCT/US2006/022599).

As discussed above, certain embodiments provide bispecific antibody of the invention protein compositions, particularly pharmaceutical compositions of the invention, that comprise, in addition to the bispecific antibody of the invention, one or more excipients such as those illustratively described in this section and elsewhere herein. Excipients can be used in the invention in this regard for a wide variety of purposes, such as adjusting physical, chemical, or biological properties of formulations, such as adjustment of viscosity, and or processes of the invention to improve effectiveness and or to stabilize such formulations and processes against degradation and spoilage due to, for instance, stresses that occur during manufacturing, shipping, storage, pre-use preparation, administration, and thereafter.

A variety of expositions are available on protein stabilization and formulation materials and methods useful in this regard, such as Arakawa et al., "Solvent interactions in pharmaceutical formulations," Pharm Res. 8(3): 285-91 (1991); Kendrick et al., "Physical stabilization of proteins in aqueous solution," in: RATIONAL DESIGN OF STABLE PROTEIN FORMULATIONS: THEORY AND PRACTICE, Carpenter and Manning, eds. Pharmaceutical Biotechnology. 13: 61-84 (2002), and Randolph et al., "Surfactant-protein interactions," Pharm Biotechnol. 13: 159-75 (2002), particularly in parts pertinent to excipients and processes of the same for self-buffering protein formulations in accordance with the current invention, especially as to protein pharmaceutical products and processes for veterinary and/or human medical uses.

Salts may be used in accordance with certain embodiments of the invention to, for example, adjust the ionic strength and/or the isotonicity of a formulation and/or to improve the solubility and/or physical stability of a protein or other ingredient of a composition in accordance with the invention.

As is well known, ions can stabilize the native state of proteins by binding to charged residues on the protein's surface and by shielding charged and polar groups in the protein and reducing the strength of their electrostatic interactions, attractive, and repulsive interactions. Ions also can stabilize the denatured state of a protein by binding to, in particular, the denatured peptide linkages (--CONH) of the protein. Furthermore, ionic interaction with charged and polar groups in a protein also can reduce intermolecular electrostatic interactions and, thereby, prevent or reduce protein aggregation and insolubility.

Ionic species differ significantly in their effects on proteins. A number of categorical rankings of ions and their effects on proteins have been developed that can be used in formulating pharmaceutical compositions in accordance with the invention. One example is the Hofmeister series, which ranks ionic and polar non-ionic solutes by their effect on the conformational stability of proteins in solution. Stabilizing solutes are referred to as "kosmotropic." Destabilizing solutes are referred to as "chaotropic." Kosmotropes commonly are used at high concentrations (e.g., >1 molar ammonium sulfate) to precipitate proteins from solution ("salting-out"). Chaotropes commonly are used to denture and/or to solubilize proteins ("salting-in"). The relative effectiveness of ions to "salt-in" and "salt-out" defines their position in the Hofmeister series.

Free amino acids can be used in the bispecific antibody of the invention formulations in accordance with various embodiments of the invention as bulking agents, stabilizers, and antioxidants, as well as other standard uses. Lysine, proline, serine, and alanine can be used for stabilizing proteins in a formulation. Glycine is useful in lyophilization to ensure correct cake structure and properties. Arginine may be useful to inhibit protein aggregation, in both liquid and lyophilized formulations. Methionine is useful as an antioxidant.

Polyols include sugars, e.g., mannitol, sucrose, and sorbitol and polyhydric alcohols such as, for instance, glycerol and propylene glycol, and, for purposes of discussion herein, polyethylene glycol (PEG) and related substances. Polyols are kosmotropic. They are useful stabilizing agents in both liquid and lyophilized formulations to protect proteins from physical and chemical degradation processes. Polyols also are useful for adjusting the tonicity of formulations. Among polyols useful in select embodiments of the invention is mannitol, commonly used to ensure structural stability of the cake in lyophilized formulations. It ensures structural stability to the cake. It is generally used with a lyoprotectant, e.g., sucrose. Sorbitol and sucrose are among preferred agents for adjusting tonicity and as stabilizers to protect against freeze-thaw stresses during transport or the preparation of bulks during the manufacturing process. Reducing sugars (which contain free aldehyde or ketone groups), such as glucose and lactose, can glycate surface lysine and arginine residues. Therefore, they generally are not among preferred polyols for use in accordance with the invention. In addition, sugars that form such reactive species, such as sucrose, which is hydrolyzed to fructose and glucose under acidic conditions, and consequently engenders glycation, also is not among preferred polyols of the invention in this regard. PEG is useful to stabilize proteins and as a cryoprotectant and can be used in the invention in this regard.

Embodiments of the bispecific antibody of the invention formulations further comprise surfactants. Protein molecules may be susceptible to adsorption on surfaces and to denaturation and consequent aggregation at air-liquid, solid-liquid, and liquid-liquid interfaces. These effects generally scale inversely with protein concentration. These deleterious interactions generally scale inversely with protein concentration and typically are exacerbated by physical agitation, such as that generated during the shipping and handling of a product. Surfactants routinely are used to prevent, minimize, or reduce surface adsorption. Useful surfactants in the invention in this regard include polysorbate 20, polysorbate 80, other fatty acid esters of sorbitan polyethoxylates, and poloxamer 188.

Surfactants also are commonly used to control protein conformational stability. The use of surfactants in this regard is protein-specific since, any given surfactant typically will stabilize some proteins and destabilize others.

Polysorbates are susceptible to oxidative degradation and often, as supplied, contain sufficient quantities of peroxides to cause oxidation of protein residue side-chains, especially methionine. Consequently, polysorbates should be used carefully, and when used, should be employed at their lowest effective concentration. In this regard, polysorbates exemplify the general rule that excipients should be used in their lowest effective concentrations.

Embodiments of the bispecific antibody of the invention formulations further comprise one or more antioxidants. To some extent deleterious oxidation of proteins can be prevented in pharmaceutical formulations by maintaining proper levels of ambient oxygen and temperature and by avoiding exposure to light. Antioxidant excipients can be used as well to prevent oxidative degradation of proteins. Among useful antioxidants in this regard are reducing agents, oxygen/free-radical scavengers, and chelating agents. Antioxidants for use in therapeutic protein formulations in accordance with the invention preferably are water-soluble and maintain their activity throughout the shelf life of a product. EDTA is a preferred antioxidant in accordance with the invention in this regard.

Antioxidants can damage proteins. For instance, reducing agents, such as glutathione in particular, can disrupt intramolecular disulfide linkages. Thus, antioxidants for use in the invention are selected to, among other things, eliminate or sufficiently reduce the possibility of themselves damaging proteins in the formulation.

Formulations in accordance with the invention may include metal ions that are protein co-factors and that are necessary to form protein coordination complexes, such as zinc necessary to form certain insulin suspensions. Metal ions also can inhibit some processes that degrade proteins. However, metal ions also catalyze physical and chemical processes that degrade proteins. Magnesium ions (10-120 mM) can be used to inhibit isomerization of aspartic acid to isoaspartic acid. Ca+2 ions (up to 100 mM) can increase the stability of human deoxyribonuclease. Mg+2, Mn+2, and Zn+2, however, can destabilize rhDNase. Similarly, Ca+2 and Sr+2 can stabilize Factor VIII, it can be destabilized by Mg+2, Mn+2 and Zn+2, Cu+2 and Fe+2, and its aggregation can be increased by AI+3 ions.

Embodiments of the bispecific antibody of the invention formulations further comprise one or more preservatives. Preservatives are necessary when developing multi-dose parenteral formulations that involve more than one extraction from the same container. Their primary function is to inhibit microbial growth and ensure product sterility throughout the shelf-life or term of use of the drug product. Commonly used preservatives include benzyl alcohol, phenol and m-cresol. Although preservatives have a long history of use with small-molecule parenterals, the development of protein formulations that includes preservatives can be challenging. Preservatives almost always have a destabilizing effect (aggregation) on proteins, and this has become a major factor in limiting their use in multi-dose protein formulations. To date, most protein drugs have been formulated for single-use only. However, when multi-dose formulations are possible, they have the added advantage of enabling patient convenience, and increased marketability. A good example is that of human growth hormone (hGH) where the development of preserved formulations has led to commercialization of more convenient, multi-use injection pen presentations. At least four such pen devices containing preserved formulations of hGH are currently available on the market. Norditropin (liquid, Novo Nordisk), Nutropin AQ (liquid, Genentech) & Genotropin (lyophilized--dual chamber cartridge, Pharmacia & Upjohn) contain phenol while Somatrope (Eli Lilly) is formulated with m-cresol. Several aspects need to be considered during the formulation and development of preserved dosage forms. The effective preservative concentration in the drug product must be optimized. This requires testing a given preservative in the dosage form with concentration ranges that confer anti-microbial effectiveness without compromising protein stability.

As might be expected, development of liquid formulations containing preservatives are more challenging than lyophilized formulations. Freeze-dried products can be lyophilized without the preservative and reconstituted with a preservative containing diluent at the time of use. This shortens the time for which a preservative is in contact with the protein, significantly minimizing the associated stability risks. With liquid formulations, preservative effectiveness and stability should be maintained over the entire product shelf-life (about 18 to 24 months). An important point to note is that preservative effectiveness should be demonstrated in the final formulation containing the active drug and all excipient components.

The bispecific antibody of the invention generally will be designed for specific routes and methods of administration, for specific administration dosages and frequencies of administration, for specific treatments of specific diseases, with ranges of bio-availability and persistence, among other things. Formulations thus may be designed in accordance with the invention for delivery by any suitable route, including but not limited to orally, aurally, opthalmically, rectally, and vaginally, and by parenteral routes, including intravenous and intraarterial injection, intramuscular injection, and subcutaneous injection.

Once the pharmaceutical composition has been formulated, it may be stored in sterile vials as a solution, suspension, gel, emulsion, solid, crystal, or as a dehydrated or lyophilized powder. Such formulations may be stored either in a ready-to-use form or in a form (e.g., lyophilized) that is reconstituted prior to administration. The invention also provides kits for producing a single-dose administration unit. The kits of the invention may each contain both a first container having a dried protein and a second container having an aqueous formulation. In certain embodiments of this invention, kits containing single and multi-chambered pre-filled syringes (e.g., liquid syringes and lyosyringes) are provided. The therapeutically effective amount of an bispecific antibody of the invention protein-containing pharmaceutical composition to be employed will depend, for example, upon the therapeutic context and objectives. One skilled in the art will appreciate that the appropriate dosage levels for treatment will vary depending, in part, upon the molecule delivered, the indication for which the bispecific antibody of the invention is being used, the route of administration, and the size (body weight, body surface or organ size) and/or condition (the age and general health) of the patient. In certain embodiments, the clinician may titer the dosage and modify the route of administration to obtain the optimal therapeutic effect. A typical dosage may range from about 0.1 µg/kg to up to about 30 mg/kg or more, depending on the factors mentioned above. In specific embodiments, the dosage may range from 1.0 µg/kg up to about 20 mg/kg, optionally from 10 µg/kg up to about 10 mg/kg or from 100 µg/kg up to about 5 mg/kg.

A therapeutic effective amount of an bispecific antibody of the invention preferably results in a decrease in severity of disease symptoms, in increase in frequency or duration of disease symptom-free periods or a prevention of impairment or disability due to the disease affliction. For treating specific tumors expressing the cell surface molecule bound by the second binding domain of the bispecific antibody of the invention, a therapeutically effective amount of the bispecific antibody of the invention, e.g. an anti-cell surface molecule/CD3 bispecific antibody, preferably inhibits cell growth or tumor growth by at least about 20%, at least about 40%, at least about 50%, at least about 60%, at least about 70%, at least about 80%, or at least about 90% relative to untreated patients. The ability of a compound to inhibit tumor growth may be evaluated in an animal model predictive of efficacy in human tumors.

Pharmaceutical compositions may be administered using a medical device. Examples of medical devices for administering pharmaceutical compositions are described in U.S. Patent Nos. 4,475,196; 4,439,196; 4,447,224; 4,447, 233; 4,486,194; 4,487,603; 4,596,556; 4,790,824; 4,941,880; 5,064,413; 5,312,335; 5,312,335; 5,383,851; and 5,399,163.

According to one embodiment of the invention the bispecific antibody of the invention, or the bispecific antibody produced according to a method of the invention is for use in the prevention, treatment or amelioration of a disease selected from the group consisting of a proliferative disease, an inflammatory disease, an infectious disease and an autoimmune disease.

Also in one embodiment the invention provides a kit comprising a binding molecule of the invention or produced according to a method of the invention, a nucleic acid molecule of the invention, a vector of the invention, or a host cell of the invention.

It should be understood that the inventions herein are not limited to particular methodology, protocols, or reagents, as such can vary. The discussion and examples provided herein are presented for the purpose of describing particular embodiments only and are not intended to limit the scope of the present invention, which is defined solely by the claims.

### Examples:

The following examples are provided for the purpose of illustrating specific embodiments or features of the present invention. These examples should not be construed as to limit the scope of this invention. The examples are included for purposes of illustration, and the present invention is limited only by the claims.

### Example 1 -

### BiTE production and purification:

BiTE antibodies were purified from culture supernatant of stably transfected chinese hamster ovary cells (CHO) adapted to 20 nM MTX. To generate one liter of supernatant for purification of BiTE antibody constructs, the cells were grown in roller bottles at a starting cell density of 5 x 10⁴ cells per ml with nucleoside-free HyQ PF CHO liquid soy medium (with 4.0 mM L-Glutamine and 0.1% Pluronic F-68; HyClone). After color change of the added color indicator phenol red from red to orange the cultivation was extended for two more days before harvesting. The cells were removed by centrifugation and the supernatant containing the expressed protein was stored at -20°C until protein purification.

Akta® Explorer Systems (GE Life Sciences) controlled by Unicorn® Software were used for chromatography. Immobilized metal affinity chromatography (IMAC) was performed using Fractogel EMD chelate® (Merck, Darmstadt) which was loaded with ZnCl₂ according to the protocol provided by the manufacturer. The column was equilibrated with buffer A (20 mM sodium phosphate buffer pH 7.2, 0.1 M NaCl , 10 mM imidazole) and the cell culture supernatant (1000 ml) applied to the column (10 ml) at a flow rate of 4 ml/min. The column was washed with buffer A to remove unbound sample. Bound protein was eluted using a two step gradient of buffer B (20 mM sodium phosphate buffer, 0.1 M NaCl, 0.5 M imidazole, pH 7.2) according to the following procedure:
Step 1: 10 % buffer B in 5 column volumes
Step 2: 100% buffer B in 5 column volumes

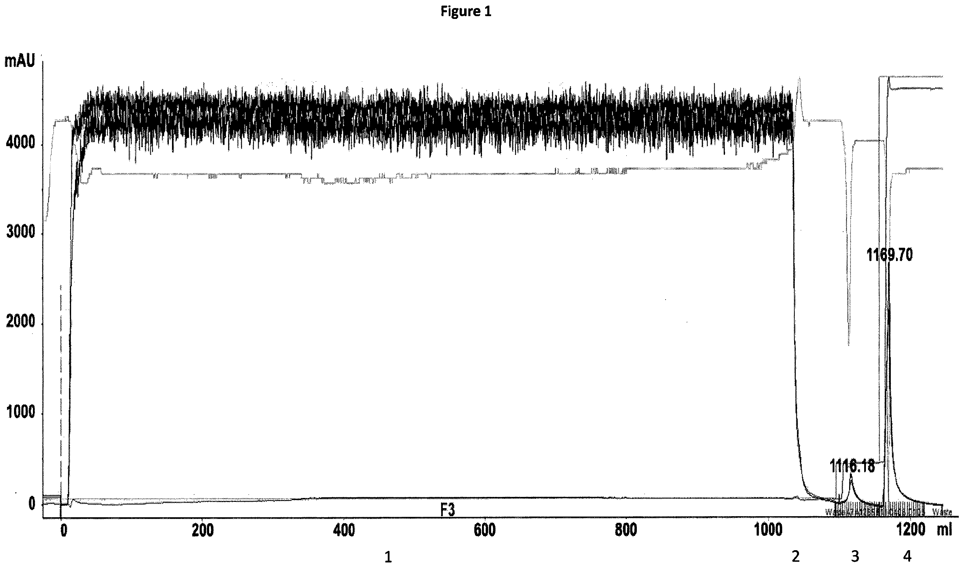

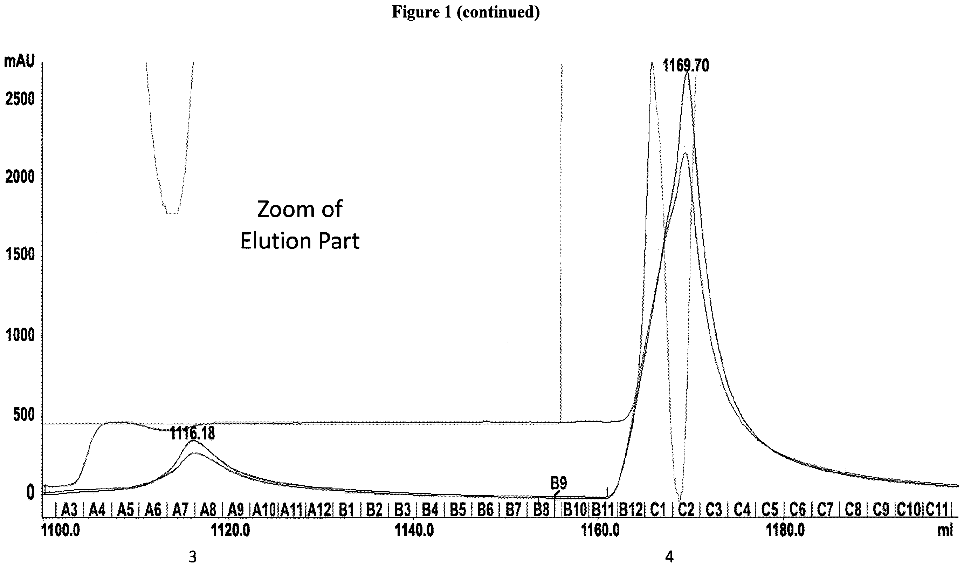

An elution profile from the IMAC purification is exemplarily shown for a SA-21-CEAxCD3 bispecific antibody in figure 1.

Eluted protein fractions from step 2 were pooled for further purification and concentrated to 3 ml final volume using Vivaspin (Sartorius-Stedim, Gottingen-Germany) centrifugation units with PES membran and a molecular weight cut-off of 10 kDa. All chemicals were of research grade and purchased from Sigma (Deisenhofen, Germany) or Merck (Darmstadt, Germany).

Size exclusion chromatography SEC was performed on a HiLoad 16/60 Superdex 200 prep grade column (GE Lifesciences) equilibrated with SEC buffer (10 mM citric acid, 75 mM lysine-HCI, pH 7.2) at a flow rate of 1 ml/min. BiTE antibody monomer and dimer fractions were pooled and a 24% trehalose stock solution was added to reach a final trehalose concentration of 4%. Eluted protein samples were subjected to reducing SDS-PAGE and Anti His TAG Western Blot for analysis.

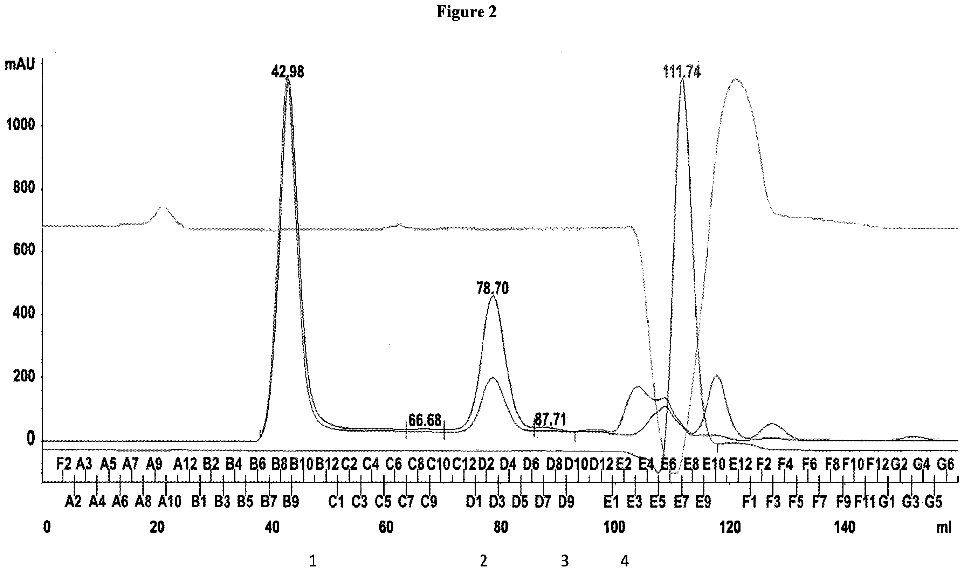

SEC Protein pools (Pool_1 = pooled SEC fractions containing the dimeric BiTE protein isoform, Pool_2 = pooled SEC fractions containing the monomeric BiTE protein) were measured for 280 nm absorption in polycarbonate cuvettes with 1 cm lightpath (Eppendorf, Hamburg-Germany) and protein concentration was calculated on the base of the Vector NTI protein analysis factor for each protein. A SEC profile is exemplarily shown for a SA-21-CEAxCD3 bispecific antibody in figure 2.

**Table 2: yield of monomeric biding molecules isolated from the culture supernatant of CHO cells stably expressing the identified bispecific antibodies**

| **Bispecific antibody** | **HSA binding domain** | **Yield [µg/l culture supernatant]** |
|---|---|---|
| CD33 x CD3 | N-term SA21 | 1277 |
| | C-term SA21 | 72 |
| | | |
| CEA x CD3 + His-tag | N-term SA21 | 719 |
| | C-term SA21 | 255 |
| | | |
| CEA x CD3 + His-tag | N-term SA25 | 660 |
| | C-term SA25 | 38 |
| | | |
| CEA x CD3 w/o His-tag | N-term SA21 | 761 |
| | C-term SA21 | 490 |
| | | |
| CEA x CD3 w/o His-tag | N-term SA08 | 1028 |
| | C-term SA08 | 216 |
| | | |
| CEA x CD3 w/o His-tag | N-term SA25 | 981 |
| | C-term SA25 | 450 |

### Example 2 -

### Cytotoxic activity: Chromium release assay with stimulated human T cells

Stimulated T cells enriched for CD8⁺ T cells were obtained as described below:
A petri dish (145 mm diameter, Greiner bio-one GmbH, Kremsmünster) was coated with a commercially available anti-CD3 specific antibody (OKT3, Orthoclone) in a final concentration of 1 µg/ml for 1 hour at 37°C. Unbound protein was removed by one washing step with PBS. 3-5 x 10⁷ human PBMC were added to the precoated petri dish in 120 ml of RPMI 1640 with stabilized glutamine / 10% FCS / IL-2 20 U/ml (Proleukin®, Chiron) and stimulated for 2 days. On the third day, the cells were collected and washed once with RPMI 1640. IL-2 was added to a final concentration of 20 U/ml and the cells were cultured again for one day in the same cell culture medium as above.

CD8⁺ cytotoxic T lymphocytes (CTLs) were enriched by depletion of CD4⁺ T cells and CD56⁺ NK cells using Dynal-Beads according to the manufacturer's protocol.

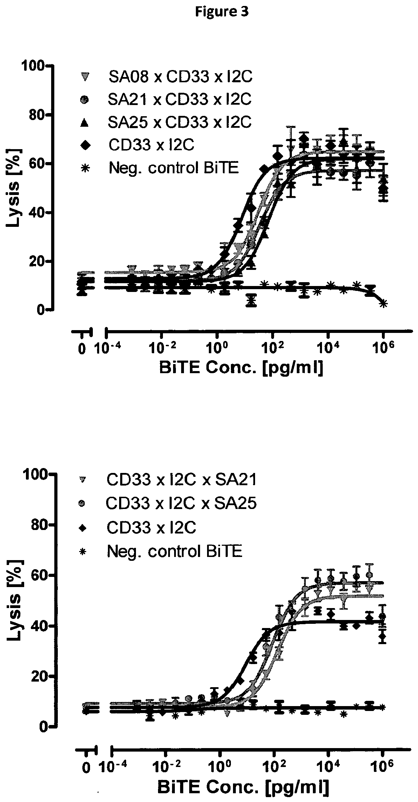

Human CD33-transfected CHO target cells were washed twice with PBS and labeled with 11.1 MBq ⁵¹Cr in a final volume of 100 µl RPMI with 50% FCS for 60 minutes at 37°C. Subsequently, the labeled target cells were washed 3 times with 5 ml RPMI and then used in the cytotoxicity assay. The assay was performed in a 96-well plate in a total volume of 200 µl supplemented RPMI with an E:T ratio of 10:1 in the presence of 10% HSA (Human-Albumin, 20%, CSL Behring GmbH: PZN-1468366). A starting concentration of 0.01 - 1 µg/ml of purified bispecific antibody and threefold dilutions thereof were used. Incubation time for the assay was 18 hours. Cytotoxicity was determined as relative values of released chromium in the supernatant relative to the difference of maximum lysis (addition of Triton-X) and spontaneous lysis (without effector cells). All measurements were carried out in quadruplicates. Measurement of chromium activity in the supernatants was performed in a Wizard 3" gamma counter (Perkin Elmer Life Sciences GmbH, Köln, Germany). Analysis of the results was carried out with Prism 5 for Windows (version 5.0, GraphPad Software Inc., San Diego, California, USA). EC50 values calculated by the analysis program from the sigmoidal dose response curves were used for comparison of cytotoxic activity (see Figure 3).

**Sequence Table**

| SEQ ID NO. | DESIGNATION | SOURCE | TYPE | SEQUENCE |
|---|---|---|---|---|
| 1 | SA08 | artificial | nt | |
| 2 | SA08 | artificial | aa | QGLIGDICLPRWGCLWGDSVK |
| 3 | SA21 | artificial | nt | |
| 4 | SA21 | artificial | aa | RLIEDICLPRWGCLWEDD |
| 5 | SA25 | artificial | nt | GAGGACATCTGCCTGCCCAGATGGGGCTGCCTGTGGGAGGAC |
| 6 | SA25 | artificial | aa | EDICLPRWGCLWED |
| 7 | SA21 x CD33 x I2C | artificial | nt | |
| 8 | SA21 x CD33 x I2C | artificial | aa | |
| 9 | CD33 x I2C x SA21 | artificial | nt | |
| | | | | |
| 10 | CD33 x I2C x SA21 | artificial | aa | |
| 11 | SA21 x EpCAM x CD3 | artificial | nt | |
| | | | | |
| 12 | SA21 x EpCAM x CD3 | artificial | aa | |
| 13 | EpCAM x CD3 x SA21 | artificial | nt | |
| 14 | EpCAM x CD3 x SA21 | artificial | aa | |
| | | | | |
| 15 | SA21 x CEA x I2C | artificial | nt | |
| 16 | SA21 x CEA x I2C | artificial | aa | |
| 17 | CEA x I2C x SA21 | artificial | nt | |
| | | | | |
| 18 | CEA x I2C x SA21 | artificial | aa | |
| 19 | SA25 x CEA x I2C | artificial | nt | |
| | | | | |
| 20 | SA25 x CEA x I2C | artificial | aa | |
| 21 | CEA x I2C x SA25 | artificial | nt | |
| 22 | CEA x I2C x SA25 | artificial | aa | |
| 23 | SA08 x CEA x I2C | artificial | nt | |
| | | | | |
| 24 | SA08 x CEA x I2C | artificial | aa | |
| 25 | SA21 x CEA x I2C H0 | artificial | nt | |
| | | | | |
| 26 | SA21 x CEA x I2C H0 | artificial | aa | |
| 27 | CEA x I2C x SA21 H0 | artificial | nt | |
| 28 | CEA x I2C x SA21 H0 | artificial | aa | |
| 29 | SA25 x CEA x I2C H0 | artificial | nt | |
| | | | | |
| 30 | SA25 x CEA x I2C H0 | artificial | aa | |
| 31 | CEA x I2C x SA25 H0 | artificial | nt | |
| | | | | |
| 32 | CEA x I2C x SA25 H0 | artificial | aa | |
| 33 | SA08 x CEA x I2C H0 | artificial | nt | |
| 34 | SA08 x CEA x I2C H0 | artificial | aa | |
| | | | | |
| 35 | CEA x I2C x SA08 H0 | artificial | nt | |
| 36 | CEA x I2C x SA08 H0 | artificial | aa | |
| 37 | N-terminus of CD3ε | human | aa | QDGNE |

## Claims

1. A single chain bispecific antibody comprising at least three binding domains, wherein
(a) the first binding domain binds to human and non-human primate serum albumin, comprises SEQ ID Nos. 2, 4 or 6, and is positioned at the N-terminus of the second binding domain;
(b) the second binding domain binds to a cell surface molecule on a human and a non-human primate cell, wherein the surface molecule is a tumor antigen; and
(c) the third binding domain binds to the T cell CD3 receptor complex on a human and a non-human primate cell,
wherein the three domains are consecutively on one polypeptide chain in the order from the N-terminus to the C-terminus
• the first binding domain;
• the second binding domain; and
• the third binding domain.

2. The bispecific antibody according to claim 1, wherein at least one of the binding domains is an scFv or single domain antibody.

3. The bispecific antibody according to any of claims 1 or 2, wherein the bispecific antibody comprises one or more further heterologous polypeptide.

4. The bispecific antibody according to any of any one of claims 1 to 3, wherein
(a) the second binding domain comprises an antibody derived VL and VH chain; and/or
(b) the third binding domain comprises an antibody derived VL and VH chain.

5. The bispecific antibody according to any of claims 1 to 4, wherein the third binding domain binding to the T cell CD3 receptor complex is binding to an epitope of human and Callithrix jacchus, Saguinus oedipus or Saimiri sciureus CD3ε chain, wherein the epitope is part of an amino acid sequence comprised in the group consisting of Gln-Asp-Gly-Asn-Glu-Glu-Met-Gly-Gly-Ile-Thr-Gln-Thr-Pro-Tyr-Lys-Val-Ser-Ile-Ser-Gly-Thr-Thr-Val-Ile-Leu-Thr, Gln-Asp-Gly-Asn-Glu-Glu-Met-Gly-Asp-Thr-Thr-Gln-Asn-Pro-Tyr-Lys-Val-Ser-Ile-Ser-Gly-Thr-Thr-Val-Thr-Leu-Thr, Gln-Asp-Gly-Asn-Glu-Glu-Met-Gly-Asp-Thr-Thr-Gln-Asn-Pro-Tyr-Lys-Val-Ser-Ile-Ser-Gly-Thr-Thr-Val-Thr-Leu-Thr, and Gln-Asp-Gly-Asn-Glu-Glu-Ile-Gly-Asp-Thr-Thr-Gln-Asn-Pro-Tyr-Lys-Val-Ser-Ile-Ser-Gly-Thr-Thr-Val-Thr-Leu-Thr, and comprises at least the amino acid sequence Gln-Asp-Gly-Asn-Glu (SEQ ID NO:37).

6. The bispecific antibody according to any of claims 1 to 5, **characterized by** an amino acid sequence as depicted in SEQ ID NOs: 8, 12, 16, 20, 24, 26, 30, or 34.

7. A nucleic acid molecule having a sequence encoding a bispecific antibody as defined in any one of claims 1 to 6.

8. A vector comprising a nucleic acid sequence as defined in claim 7.

9. A host cell transformed or transfected with the nucleic acid sequence as defined in claim 7 or with the vector as defined in claim 8.

10. A process for the production of a bispecific antibody according to any one of claims 1 to 6, said process comprising culturing a host cell as defined in claim 9 under conditions allowing the expression of the bispecific antibody as defined in any one of claims 1 to 6 and recovering the produced bispecific antibody from the culture.

11. A pharmaceutical composition comprising a bispecific antibody according to any one of claims 1 to 6, or produced according to the process of claim 6.

12. The bispecific antibody according to any one of claims 1 to 6, or produced according to a method of claim 10 for use in the prevention, treatment or amelioration of a disease selected from the group consisting of a proliferative disease, an inflammatory disease, an infectious disease and an autoimmune disease.

13. A kit comprising a bispecific antibody as defined in any one of claims 1 to 6 or produced according to a method of claim 10, a nucleic acid molecule as defined in claim 7, a vector as defined in claim 8, or a host cell as defined in claim 9.

## Patentansprüche

1. Einzelketten bispezifischer Antikörper, umfassend mindestens drei Bindungsdomänen, wobei:
(a) die erste Bindungsdomäne an menschliches und nicht-menschliches Primatenserumalbumin bindet, SEQ ID Nos. 2, 4 oder 6 umfasst und am N-Terminus der zweiten Bindungsdomäne positioniert ist;
(b) die zweite Bindungsdomäne an ein Zelloberflächenmolekül auf einer menschlichen und einer nicht-menschlichen Primatenzelle bindet, wobei das Oberflächenmolekül ein Tumorantigen ist; und
(c) die dritte Bindungsdomäne an den T-Zell CD3 Rezeptorkomplex auf einer menschlichen und einer nicht-menschlichen Primatenzelle bindet,
wobei die drei Domänen nacheinander auf einer Polypeptidkette sind in der Reihenfolge vom N-Terminus zum C-Terminus
- die erste Bindungsdomäne;
- die zweite Bindungsdomäne; und
- die dritte Bindungsdomäne.

2. Bispezifischer Antikörper nach Anspruch 1, wobei mindestens eine der Bindungsdomänen ein scFv oder Einzeldomänenantikörper ist.

3. Bispezifischer Antikörper nach einem der Ansprüche 1 oder 2, wobei der bispezifische Antikörper ein oder mehrere weitere heterologe Polypeptide umfasst.

4. Bispezifischer Antikörper nach einem der Ansprüche 1 bis 3, wobei
(a) die zweite Bindungsdomäne eine Antikörper-abgeleitete VL- und VH-Kette umfasst; und/oder
(b) die dritte Bindungsdomäne eine Antikörper-abgeleitete VL- und VH-Kette umfasst.

5. Bispezifischer Antikörper nach einem der Ansprüche 1 bis 4, wobei die dritte Bindungsdomäne, die an den T-Zell CD3 Rezeptorkomplex bindet, an ein Epitop einer menschlichen und Callithrix jacchus, Saguinus oedipus oder Saimiri sciureus CD3ε Kette bindet, wobei das Epitop Teil einer Aminosäuresequenz ist, die in der Gruppe bestehend aus Gln-Asp-Giy-Asn-Glu-Glu-Met-Giy-Gly-Gly-Ile-Thr-Gln-Thr-Pro-Tyr-Lys-Val-Ser-Ile-Ser-Gly-Thr-Thr-Val-Ile-Leu-Thr, Gln-Asp-Gly-Asn-Glu-Glu-Met-Gly-Asp-Thr-Thr-Gln-Asn-Pro-Tyr-Lys-Val-Ser-Ile-Ser-Gly-Thr-Thr-Val-Thr-Leu-Thr, Gln-Asp-Gly-Asn-Glu-Glu-Met-Gly-Asp-Thr-Thr-Gln-Asn-Pro-Tyr-Lys-Val-Ser-Ile-Ser-Gly-Thr-Thr-Val-Thr-Leu-Thr, and Gln-Asp-Gly-Asn-Glu-Glu-Ile-Gly-Asp-Thr-Thr-Gln-Asn-Pro-Tyr-Lys-Val-Ser-Ile-Ser-Gly-Thr-Thr-Val-Thr-Leu-Thr umfasst ist, und mindestens die Aminosäuresequenz Gln-Asp-GiyAsn-Giu (SEQ 10 NO:37) umfasst.

6. Bispezifischer Antikörper nach einem der Ansprüche 1 bis 5, **gekennzeichnet durch** eine Aminosäuresequenz wie in SEQ ID NOs: 8, 12, 16, 20, 24, 26, 30 oder 34 dargestellt.

7. Nukleinsäuremolekül mit einer Sequenz, die einen bispezifischen Antikörper wie in einem der Ansprüche 1 bis 6 definiert kodiert.

8. Vektor, umfassend eine Nukleinsäuresequenz wie in Anspruch 7 definiert.

9. Wirtszelle, transformiert oder transfiziert mit der Nukleinsäuresequenz wie in Anspruch 7 definiert oder mit dem Vektor wie in Anspruch 8 definiert.

10. Verfahren zur Herstellung eines bispezifischen Antikörpers nach einem der Ansprüche 1 bis 6, wobei das Verfahren das Kultivieren einer Wirtszelle wie in Anspruch 9 definiert unter Bedingungen umfasst, die die Expression des bispezifischen Antikörpers wie in einem der Ansprüche 1 bis 6 definiert und die Rückgewinnung des hergestellten bispezifischen Antikörpers aus der Kultur ermöglichen.

11. Pharmazeutische Zusammensetzung, umfassend einen bispezifischen Antikörper nach einem der Ansprüche 1 bis 6, oder hergestellt nach dem Verfahren nach Anspruch 6.

12. Bispezifischer Antikörper nach einem der Ansprüche 1 bis 6, oder hergestellt nach dem Verfahren nach Anspruch 10, zur Verwendung bei der Prävention, Behandlung oder Verbesserung einer Erkrankung ausgewählt aus der Gruppe bestehend aus einer proliferativen Erkrankung, einer entzündlichen Erkrankung, einer infektiösen Erkrankung und einer Autoimmunerkrankung.

13. Kit, umfassend einen bispezifischen Antikörper wie in einem der Ansprüche 1 bis 6 definiert oder hergestellt nach dem Verfahren nach Anspruch 10, ein Nukleinsäuremolekül wie in Anspruch 7 definiert, einen Vektor wie in Anspruch 8 definiert oder eine Wirtszelle wie in Anspruch 9 definiert.

## Revendications

1. Anticorps bispécifique à chaîne unique comprenant au moins trois domaines de liaison, dans lequel
(a) le premier domaine de liaison se lie à la sérum albumine de primate humain et non humain, comprend SEQ ID NO : 2, 4 ou 6, et est positionné au niveau de l'extrémité N-terminale du deuxième domaine de liaison ;
(b) le deuxième domaine de liaison se lie à une molécule de surface cellulaire sur une cellule de primate humain et non humain, dans lequel la molécule de surface est un antigène tumoral ; et
(c) le troisième domaine de liaison se lie au complexe récepteur CD3 des lymphocytes T sur une cellule de primate humain et non humain,
dans lequel les trois domaines sont consécutivement sur une chaîne polypeptidique dans l'ordre de l'extrémité N-terminale à l'extrémité C-terminale
- le premier domaine de liaison ;
- le deuxième domaine de liaison ; et
- le troisième domaine de liaison.

2. Anticorps bispécifique selon la revendication 1, dans lequel au moins l'un des domaines de liaison est un anticorps ScFv ou à domaine unique.

3. Anticorps bispécifique selon l'une quelconque des revendications 1 ou 2, dans lequel l'anticorps bispécifique comprend un ou plusieurs autres polypeptides hétérologues.

4. Anticorps bispécifique selon l'une quelconque des revendications 1 à 3, dans lequel
(a) le deuxième domaine de liaison comprend une chaîne VL et VH dérivée d'un anticorps ; et/ou
(b) le troisième domaine de liaison comprend une chaîne VL et VH dérivée d'un anticorps.

5. Anticorps bispécifique selon l'une quelconque des revendications 1 à 4, dans lequel le troisième domaine de liaison se liant au complexe récepteur CD3 des lymphocytes T se lie à un épitope de la chaîne CD3ε humaine et de Callithrix jacchus, Saguinus oedipus ou Saimiri sciureus, dans lequel l'épitope fait partie d'une séquence d'acides aminés comprise dans le groupe constitué de Gln-Asp-Gly-Asn-Glu-Glu-Met-Gly-Gly-Ile-Thr-Gln-Thr-Pro-Tyr-Lys-Val-Ser-Ile-Ser-Gly-Thr-Thr-Val-Ile-Leu-Thr, Gln-Asp-Gly-Asn-Glu-Glu-Met-Gly-Asp-Thr-Thr-Gln-Asn-Pro-Tyr-Lys-Val-Ser-Ile-Ser-Gly-Thr-Thr-Val-Thr-Leu-Thr, Gln-Asp-Gly-Asn-Glu-Glu-Met-Gly-Asp-Thr-Thr-Gln-Asn-Pro-Tyr-Lys-Val-Ser-Ile-Ser-Gly-Thr-Thr-Val-Thr-Leu-Thr, et Gln-Asp-Gly-Asn-Glu-Glu-Ile-Gly-Asp-Thr-Thr-Gln-Asn-Pro-Tyr-Lys-Val-Ser-Ile-Ser-Gly-Thr-Thr-Val-Thr-Leu-Thr, et comprend au moins la séquence d'acides aminés Gln-Asp-Gly-Asn-Glu (SEQ ID NO : 37).

6. Anticorps bispécifique selon l'une quelconque des revendications 1 à 5, **caractérisé par** une séquence d'acides aminés telle que représentée dans SEQ ID NO : 8, 12, 16, 20, 24, 26, 30, ou 34.

7. Molécule d'acide nucléique ayant une séquence codant pour un anticorps bispécifique tel que défini dans l'une quelconque des revendications 1 à 6.

8. Vecteur comprenant une séquence d'acide nucléique selon la revendication 7.

9. Cellule hôte transformée ou transfectée avec la séquence d'acide nucléique selon la revendication 7 ou avec le vecteur selon la revendication 8.

10. Procédé de production d'un anticorps bispécifique selon l'une quelconque des revendications 1 à 6, ledit procédé comprenant la culture d'une cellule hôte selon la revendication 9 dans des conditions permettant l'expression de l'anticorps bispécifique selon l'une quelconque des revendications 1 à 6 et la récupération de l'anticorps bispécifique produit de la culture.

11. Composition pharmaceutique comprenant un anticorps bispécifique selon l'une quelconque des revendications 1 à 6, ou produit conformément au procédé selon la revendication 6.

12. Anticorps bispécifique selon l'une quelconque des revendications 1 à 6, ou produit conformément à un procédé selon la revendication 10 pour son utilisation dans la prévention, le traitement ou l'amélioration d'une maladie sélectionnée dans le groupe constitué d'une maladie proliférative, d'une maladie inflammatoire, d'une maladie infectieuse et d'une maladie auto-immune.

13. Kit comprenant un anticorps bispécifique selon l'une quelconque des revendications 1 à 6 ou produit conformément à un procédé selon la revendication 10, une molécule d'acide nucléique selon la revendication 7, un vecteur selon la revendication 8, ou une cellule hôte selon la revendication 9.


### SEQUENCE LISTING

<110> Amgen Research (Munich) GmbH
<120> Single chain binding molecules comprising N-terminal ABP
<130> MIM14740PCT
<150> US 61/792,073
   <151> 2013-03-15
<160> 37
<170> PatentIn version 3.5
<210> 1
   <211> 63
   <212> DNA
   <213> artificial
<220>
   <223> SA08
<400> 1
   cagggcctga tcggcgacat ctgcctgccc agatggggct gcctgtgggg cgactccgtg 60
   aaa 63
<210> 2
   <211> 21
   <212> PRT
   <213> artificial
<220>
   <223> SA08
<400> 2
   Gln Gly Leu Ile Gly Asp Ile Cys Leu Pro Arg Trp Gly Cys Leu Trp
   1 5 10 15
   Gly Asp Ser Val Lys
   20
<210> 3
   <211> 54
   <212> DNA
   <213> artificial
<220>
   <223> SA21
<400> 3
   cggctgatcg aggacatctg cctgcccaga tggggctgcc tgtgggagga cgac 54
<210> 4
   <211> 18
   <212> PRT
   <213> artificial
<220>
   <223> SA21
<400> 4
   Arg Leu Ile Glu Asp Ile Cys Leu Pro Arg Trp Gly Cys Leu Trp Glu
   1 5 10 15
   Asp Asp
<210> 5
   <211> 42
   <212> DNA
   <213> artificial
<220>
   <223> SA25
<400> 5
   gaggacatct gcctgcccag atggggctgc ctgtgggagg ac 42
<210> 6
   <211> 14
   <212> PRT
   <213> artificial
<220>
   <223> SA25
<400> 6
   Glu Asp Ile Cys Leu Pro Arg Trp Gly Cys Leu Trp Glu Asp
   1 5 10
<210> 7
   <211> 1587
   <212> DNA
   <213> artificial
<220>
   <223> SA21 x CD33 x I2C
<400> 7
   cggctgatcg aggacatctg cctgcccaga tggggctgcc tgtgggagga cgaccaggtg      60
   cagctggtgc agtctggagc tgaggtgaag aagcctggag agtcagtcaa ggtctcctgc     120
   aaggctagcg ggtatacctt cacaaactat ggaatgaact gggtgaagca ggctccagga     180
   cagggtttag agtggatggg ctggataaac acctacactg gagagccaac ctatgctgat     240
   aagttccagg gacgcgttac catgactacg gatacctcta ccagcactgc ctatatggaa     300
   atccgcaacc tcggaggtga tgacacggct gtatattact gtgcgcgctg gagttggagt     360
   gatggttact acgtttactt tgactactgg ggccaaggca cttcggtcac cgtctcctca     420
   ggtggtggtg gttctggcgg cggcggctcc ggtggtggtg gttctgacat cgtgatgaca     480
   cagtctccag actccctgac tgtgtctctg ggcgagagga ccaccatcaa ctgcaagtcc     540
   agccagagtg ttttagacag ctccacgaat aagaactcct tagcttggta ccagcagaaa     600
   ccaggacagc ctcctaaatt actcctttcc tgggcatcta cgcgggaatc cgggatccct     660
   gaccgattca gtggcagcgg gtctgggaca gatttcactc tcactattga cagcccgcag     720
   cctgaagatt ctgcaactta ctattgtcaa cagtctgccc acttcccgat cacctttggc     780
   caagggacac gactggagat taaatccgga ggtggtggat ccgaggtgca gctggtcgag     840
   tctggaggag gattggtgca gcctggaggg tcattgaaac tctcatgtgc agcctctgga     900
   ttcaccttca ataagtacgc catgaactgg gtccgccagg ctccaggaaa gggtttggaa     960
   tgggttgctc gcataagaag taaatataat aattatgcaa catattatgc cgattcagtg    1020
   aaagacaggt tcaccatctc cagagatgat tcaaaaaaca ctgcctatct acaaatgaac    1080
   aacttgaaga ctgaggacac tgccgtgtac tactgtgtga gacatgggaa cttcggtaat    1140
   agctacatat cctactgggc ttactggggc caagggactc tggtcaccgt ctcctcaggt    1200
   ggtggtggtt ctggcggcgg cggctccggt ggtggtggtt ctcagactgt tgtgactcag    1260
   gaaccttcac tcaccgtatc acctggtgga acagtcacac tcacttgtgg ctcctcgact    1320
   ggggctgtta catctggcaa ctacccaaac tgggtccaac aaaaaccagg tcaggcaccc    1380
   cgtggtctaa taggtgggac taagttcctc gcccccggta ctcctgccag attctcaggc    1440
   tccctgcttg gaggcaaggc tgccctcacc ctctcagggg tacagccaga ggatgaggca    1500
   gaatattact gtgttctatg gtacagcaac cgctgggtgt tcggtggagg aaccaaactg    1560
   actgtcctac atcatcacca tcatcat                                       1587
<210> 8
   <211> 529
   <212> PRT
   <213> artificial
<220>
   <223> SA21 x CD33 x I2C
<400> 8
   Arg Leu Ile Glu Asp Ile Cys Leu Pro Arg Trp Gly Cys Leu Trp Glu
   1               5                   10                  15
   Asp Asp Gln Val Gln Leu Val Gln Ser Gly Ala Glu Val Lys Lys Pro
               20                  25                  30
   Gly Glu Ser Val Lys Val Ser Cys Lys Ala Ser Gly Tyr Thr Phe Thr
           35                  40                  45
   Asn Tyr Gly Met Asn Trp Val Lys Gln Ala Pro Gly Gln Gly Leu Glu
       50                  55                  60
   Trp Met Gly Trp Ile Asn Thr Tyr Thr Gly Glu Pro Thr Tyr Ala Asp
   65                  70                  75                  80
   Lys Phe Gln Gly Arg Val Thr Met Thr Thr Asp Thr Ser Thr Ser Thr
                   85                  90                  95
   Ala Tyr Met Glu Ile Arg Asn Leu Gly Gly Asp Asp Thr Ala Val Tyr
               100                 105                 110
   Tyr Cys Ala Arg Trp Ser Trp Ser Asp Gly Tyr Tyr Val Tyr Phe Asp
           115                 120                 125
   Tyr Trp Gly Gln Gly Thr Ser Val Thr Val Ser Ser Gly Gly Gly Gly
       130                 135                 140
   Ser Gly Gly Gly Gly Ser Gly Gly Gly Gly Ser Asp Ile Val Met Thr
   145                 150                 155                 160
   Gln Ser Pro Asp Ser Leu Thr Val Ser Leu Gly Glu Arg Thr Thr Ile
                   165                 170                 175
   Asn Cys Lys Ser Ser Gln Ser Val Leu Asp Ser Ser Thr Asn Lys Asn
               180                 185                 190
   Ser Leu Ala Trp Tyr Gln Gln Lys Pro Gly Gln Pro Pro Lys Leu Leu
           195                 200                 205
   Leu Ser Trp Ala Ser Thr Arg Glu Ser Gly Ile Pro Asp Arg Phe Ser
       210                 215                 220
   Gly Ser Gly Ser Gly Thr Asp Phe Thr Leu Thr Ile Asp Ser Pro Gln
   225                 230                 235                 240
   Pro Glu Asp Ser Ala Thr Tyr Tyr Cys Gln Gln Ser Ala His Phe Pro
                   245                 250                 255
   Ile Thr Phe Gly Gln Gly Thr Arg Leu Glu Ile Lys Ser Gly Gly Gly
               260                 265                 270
   Gly Ser Glu Val Gln Leu Val Glu Ser Gly Gly Gly Leu Val Gln Pro
           275                 280                 285
   Gly Gly Ser Leu Lys Leu Ser Cys Ala Ala Ser Gly Phe Thr Phe Asn
       290                 295                 300
   Lys Tyr Ala Met Asn Trp Val Arg Gln Ala Pro Gly Lys Gly Leu Glu
   305                 310                 315                 320
   Trp Val Ala Arg Ile Arg Ser Lys Tyr Asn Asn Tyr Ala Thr Tyr Tyr
                   325                 330                 335
   Ala Asp Ser Val Lys Asp Arg Phe Thr Ile Ser Arg Asp Asp Ser Lys
               340                 345                 350
   Asn Thr Ala Tyr Leu Gln Met Asn Asn Leu Lys Thr Glu Asp Thr Ala
           355                 360                 365
   Val Tyr Tyr Cys Val Arg His Gly Asn Phe Gly Asn Ser Tyr Ile Ser
       370                 375                 380
   Tyr Trp Ala Tyr Trp Gly Gln Gly Thr Leu Val Thr Val Ser Ser Gly
   385                 390                 395                 400
   Gly Gly Gly Ser Gly Gly Gly Gly Ser Gly Gly Gly Gly Ser Gln Thr
                   405                 410                 415
   Val Val Thr Gln Glu Pro Ser Leu Thr Val Ser Pro Gly Gly Thr Val
               420                 425                 430
   Thr Leu Thr Cys Gly Ser Ser Thr Gly Ala Val Thr Ser Gly Asn Tyr
           435                 440                 445
   Pro Asn Trp Val Gln Gln Lys Pro Gly Gln Ala Pro Arg Gly Leu Ile
       450                 455                 460
   Gly Gly Thr Lys Phe Leu Ala Pro Gly Thr Pro Ala Arg Phe Ser Gly
   465                 470                 475                 480
   Ser Leu Leu Gly Gly Lys Ala Ala Leu Thr Leu Ser Gly Val Gln Pro
                   485                 490                 495
   Glu Asp Glu Ala Glu Tyr Tyr Cys Val Leu Trp Tyr Ser Asn Arg Trp
               500                 505                 510
   Val Phe Gly Gly Gly Thr Lys Leu Thr Val Leu His His His His His
           515                 520                 525
   His
<210> 9
   <211> 1587
   <212> DNA
   <213> artificial
<220>
   <223> CD33 x I2C x SA21
<400> 9
   caggtgcagc tggtgcagtc tggagctgag gtgaagaagc ctggagagtc agtcaaggtc      60
   tcctgcaagg ctagcgggta taccttcaca aactatggaa tgaactgggt gaagcaggct     120
   ccaggacagg gtttagagtg gatgggctgg ataaacacct acactggaga gccaacctat     180
   gctgataagt tccagggacg cgttaccatg actacggata cctctaccag cactgcctat     240
   atggaaatcc gcaacctcgg aggtgatgac acggctgtat attactgtgc gcgctggagt     300
   tggagtgatg gttactacgt ttactttgac tactggggcc aaggcacttc ggtcaccgtc     360
   tcctcaggtg gtggtggttc tggcggcggc ggctccggtg gtggtggttc tgacatcgtg     420
   atgacacagt ctccagactc cctgactgtg tctctgggcg agaggaccac catcaactgc     480
   aagtccagcc agagtgtttt agacagctcc acgaataaga actccttagc ttggtaccag     540
   cagaaaccag gacagcctcc taaattactc ctttcctggg catctacgcg ggaatccggg     600
   atccctgacc gattcagtgg cagcgggtct gggacagatt tcactctcac tattgacagc     660
   ccgcagcctg aagattctgc aacttactat tgtcaacagt ctgcccactt cccgatcacc     720
   tttggccaag ggacacgact ggagattaaa tccggaggtg gtggatccga ggtgcagctg     780
   gtcgagtctg gaggaggatt ggtgcagcct ggagggtcat tgaaactctc atgtgcagcc     840
   tctggattca ccttcaataa gtacgccatg aactgggtcc gccaggctcc aggaaagggt     900
   ttggaatggg ttgctcgcat aagaagtaaa tataataatt atgcaacata ttatgccgat     960
   tcagtgaaag acaggttcac catctccaga gatgattcaa aaaacactgc ctatctacaa    1020
   atgaacaact tgaagactga ggacactgcc gtgtactact gtgtgagaca tgggaacttc    1080
   ggtaatagct acatatccta ctgggcttac tggggccaag ggactctggt caccgtctcc    1140
   tcaggtggtg gtggttctgg cggcggcggc tccggtggtg gtggttctca gactgttgtg    1200
   actcaggaac cttcactcac cgtatcacct ggtggaacag tcacactcac ttgtggctcc    1260
   tcgactgggg ctgttacatc tggcaactac ccaaactggg tccaacaaaa accaggtcag    1320
   gcaccccgtg gtctaatagg tgggactaag ttcctcgccc ccggtactcc tgccagattc    1380
   tcaggctccc tgcttggagg caaggctgcc ctcaccctct caggggtaca gccagaggat    1440
   gaggcagaat attactgtgt tctatggtac agcaaccgct gggtgttcgg tggaggaacc    1500
   aaactgactg tcctacggct gatcgaggac atctgcctgc ccagatgggg ctgcctgtgg    1560
   gaggacgacc atcatcacca tcatcat                                        1587
<210> 10
   <211> 529
   <212> PRT
   <213> artificial
<220>
   <223> CD33 x I2C x SA21
<400> 10
   Gln Val Gln Leu Val Gln Ser Gly Ala Glu Val Lys Lys Pro Gly Glu
   1               5                   10                  15
   Ser Val Lys Val Ser Cys Lys Ala Ser Gly Tyr Thr Phe Thr Asn Tyr
               20                  25                  30
   Gly Met Asn Trp Val Lys Gln Ala Pro Gly Gln Gly Leu Glu Trp Met
           35                  40                  45
   Gly Trp Ile Asn Thr Tyr Thr Gly Glu Pro Thr Tyr Ala Asp Lys Phe
       50                  55                  60
   Gln Gly Arg Val Thr Met Thr Thr Asp Thr Ser Thr Ser Thr Ala Tyr
   65                  70                  75                  80
   Met Glu Ile Arg Asn Leu Gly Gly Asp Asp Thr Ala Val Tyr Tyr Cys
                   85                  90                  95
   Ala Arg Trp Ser Trp Ser Asp Gly Tyr Tyr Val Tyr Phe Asp Tyr Trp
               100                 105                 110
   Gly Gln Gly Thr Ser Val Thr Val Ser Ser Gly Gly Gly Gly Ser Gly
           115                 120                 125
   Gly Gly Gly Ser Gly Gly Gly Gly Ser Asp Ile Val Met Thr Gln Ser
       130                 135                 140
   Pro Asp Ser Leu Thr Val Ser Leu Gly Glu Arg Thr Thr Ile Asn Cys
   145                 150                 155                 160
   Lys Ser Ser Gln Ser Val Leu Asp Ser Ser Thr Asn Lys Asn Ser Leu
                   165                 170                 175
   Ala Trp Tyr Gln Gln Lys Pro Gly Gln Pro Pro Lys Leu Leu Leu Ser
               180                 185                 190
   Trp Ala Ser Thr Arg Glu Ser Gly Ile Pro Asp Arg Phe Ser Gly Ser
           195                 200                 205
   Gly Ser Gly Thr Asp Phe Thr Leu Thr Ile Asp Ser Pro Gln Pro Glu
       210                 215                 220
   Asp Ser Ala Thr Tyr Tyr Cys Gln Gln Ser Ala His Phe Pro Ile Thr
   225 230 235 240
   Phe Gly Gln Gly Thr Arg Leu Glu Ile Lys Ser Gly Gly Gly Gly Ser
   245 250 255
   Glu Val Gln Leu Val Glu Ser Gly Gly Gly Leu Val Gln Pro Gly Gly
   260 265 270
   Ser Leu Lys Leu Ser Cys Ala Ala Ser Gly Phe Thr Phe Asn Lys Tyr
   275 280 285
   Ala Met Asn Trp Val Arg Gln Ala Pro Gly Lys Gly Leu Glu Trp Val
   290 295 300
   Ala Arg Ile Arg Ser Lys Tyr Asn Asn Tyr Ala Thr Tyr Tyr Ala Asp
   305 310 315 320
   Ser Val Lys Asp Arg Phe Thr Ile Ser Arg Asp Asp Ser Lys Asn Thr
   325 330 335
   Ala Tyr Leu Gln Met Asn Asn Leu Lys Thr Glu Asp Thr Ala Val Tyr
   340 345 350
   Tyr Cys Val Arg His Gly Asn Phe Gly Asn Ser Tyr Ile Ser Tyr Trp
   355 360 365
   Ala Tyr Trp Gly Gln Gly Thr Leu Val Thr Val Ser Ser Gly Gly Gly
   370 375 380
   Gly Ser Gly Gly Gly Gly Ser Gly Gly Gly Gly Ser Gln Thr Val Val
   385 390 395 400
   Thr Gln Glu Pro Ser Leu Thr Val Ser Pro Gly Gly Thr Val Thr Leu
   405 410 415
   Thr Cys Gly Ser Ser Thr Gly Ala Val Thr Ser Gly Asn Tyr Pro Asn
   420 425 430
   Trp Val Gln Gln Lys Pro Gly Gln Ala Pro Arg Gly Leu Ile Gly Gly
   435 440 445
   Thr Lys Phe Leu Ala Pro Gly Thr Pro Ala Arg Phe Ser Gly Ser Leu
   450 455 460
   Leu Gly Gly Lys Ala Ala Leu Thr Leu Ser Gly Val Gln Pro Glu Asp
   465                 470                 475                 480
   Glu Ala Glu Tyr Tyr Cys Val Leu Trp Tyr Ser Asn Arg Trp Val Phe
                   485                 490                 495
   Gly Gly Gly Thr Lys Leu Thr Val Leu Arg Leu Ile Glu Asp Ile Cys
               500                 505                 510
   Leu Pro Arg Trp Gly Cys Leu Trp Glu Asp Asp His His His His His
           515                 520                 525
   His
<210> 11
   <211> 1560
   <212> DNA
   <213> artificial
<220>
   <223> SA21 x EpCAM x CD3
<400> 11
   cggctgatcg aggacatctg cctgcccaga tggggctgcc tgtgggagga cgacgagctc      60
   gtgatgacac agtctccatc ctccctgact gtgacagcag gagagaaggt cactatgagc     120
   tgcaagtcca gtcagagtct gttaaacagt ggaaatcaaa agaactactt gacctggtac     180
   cagcagaaac cagggcagcc tcctaaactg ttgatctact gggcatccac tagggaatct     240
   ggggtccctg atcgcttcac aggcagtgga tctggaacag atttcactct caccatcagc     300
   agtgtgcagg ctgaagacct ggcagtttat tactgtcaga atgattatag ttatccgctc     360
   acgttcggtg ctgggaccaa gcttgagatc aaaggtggtg gtggttctgg cggcggcggc     420
   tccggtggtg gtggttctga ggtgcagctg ctcgagcagt ctggagctga gctggtaagg     480
   cctgggactt cagtgaagat atcctgcaag gcttctggat acgccttcac taactactgg     540
   ctaggttggg taaagcagag gcctggacat ggacttgagt ggattggaga tattttccct     600
   ggaagtggta atatccacta caatgagaag ttcaagggca aagccacact gactgcagac     660
   aaatcttcga gcacagccta tatgcagctc agtagcctga catttgagga ctctgctgtc     720
   tatttctgtg caagactgag gaactgggac gagcctatgg actactgggg ccaagggacc     780
   acggtcaccg tctcctccgg aggtggtggc tccgacgtcc aactggtgca gtcaggggct     840
   gaagtgaaaa aacctggggc ctcagtgaag gtgtcctgca aggcttctgg ctacaccttt     900
   actaggtaca cgatgcactg ggtaaggcag gcacctggac agggtctgga atggattgga     960
   tacattaatc ctagccgtgg ttatactaat tacgcagaca gcgtcaaggg ccgcttcaca    1020
   atcactacag acaaatccac cagcacagcc tacatggaac tgagcagcct gcgttctgag    1080
   gacactgcaa cctattactg tgcaagatat tatgatgatc attactgcct tgactactgg    1140
   ggccaaggca ccacggtcac cgtctcctca ggcgaaggta ctagtactgg ttctggtgga    1200
   agtggaggtt caggtggagc agacgacatt gtactgaccc agtctccagc aactctgtct    1260
   ctgtctccag gggagcgtgc caccctgagc tgcagagcca gtcaaagtgt aagttacatg    1320
   aactggtacc agcagaagcc gggcaaggca cccaaaagat ggatttatga cacatccaaa    1380
   gtggcttctg gagtccctgc tcgcttcagt ggcagtgggt ctgggaccga ctactctctc    1440
   acaatcaaca gcttggaggc tgaagatgct gccacttatt actgccaaca gtggagtagt    1500
   aacccgctca cgttcggtgg cgggaccaag gtggagatca aacatcatca ccatcatcat    1560
<210> 12
   <211> 520
   <212> PRT
   <213> artificial
<220>
   <223> SA21 x EpCAM x CD3
<400> 12
   Arg Leu Ile Glu Asp Ile Cys Leu Pro Arg Trp Gly Cys Leu Trp Glu
   1               5                   10                  15
   Asp Asp Glu Leu Val Met Thr Gln Ser Pro Ser Ser Leu Thr Val Thr
               20                  25                  30
   Ala Gly Glu Lys Val Thr Met Ser Cys Lys Ser Ser Gln Ser Leu Leu
           35                  40                  45
   Asn Ser Gly Asn Gln Lys Asn Tyr Leu Thr Trp Tyr Gln Gln Lys Pro
       50                  55                  60
   Gly Gln Pro Pro Lys Leu Leu Ile Tyr Trp Ala Ser Thr Arg Glu Ser
   65                  70                  75                  80
   Gly Val Pro Asp Arg Phe Thr Gly Ser Gly Ser Gly Thr Asp Phe Thr
                   85                  90                  95
   Leu Thr Ile Ser Ser Val Gln Ala Glu Asp Leu Ala Val Tyr Tyr Cys
               100                 105                 110
   Gln Asn Asp Tyr Ser Tyr Pro Leu Thr Phe Gly Ala Gly Thr Lys Leu
           115                 120                 125
   Glu Ile Lys Gly Gly Gly Gly Ser Gly Gly Gly Gly Ser Gly Gly Gly
       130                 135                 140
   Gly Ser Glu Val Gln Leu Leu Glu Gln Ser Gly Ala Glu Leu Val Arg
   145                 150                 155                 160
   Pro Gly Thr Ser Val Lys Ile Ser Cys Lys Ala Ser Gly Tyr Ala Phe
                   165                 170                 175
   Thr Asn Tyr Trp Leu Gly Trp Val Lys Gln Arg Pro Gly His Gly Leu
               180                 185                 190
   Glu Trp Ile Gly Asp Ile Phe Pro Gly Ser Gly Asn Ile His Tyr Asn
           195                 200                 205
   Glu Lys Phe Lys Gly Lys Ala Thr Leu Thr Ala Asp Lys Ser Ser Ser
       210                 215                 220
   Thr Ala Tyr Met Gln Leu Ser Ser Leu Thr Phe Glu Asp Ser Ala Val
   225                 230                 235                 240
   Tyr Phe Cys Ala Arg Leu Arg Asn Trp Asp Glu Pro Met Asp Tyr Trp
                   245                 250                 255
   Gly Gln Gly Thr Thr Val Thr Val Ser Ser Gly Gly Gly Gly Ser Asp
               260                 265                 270
   Val Gln Leu Val Gln Ser Gly Ala Glu Val Lys Lys Pro Gly Ala Ser
           275                 280                 285
   Val Lys Val Ser Cys Lys Ala Ser Gly Tyr Thr Phe Thr Arg Tyr Thr
       290                 295                 300
   Met His Trp Val Arg Gln Ala Pro Gly Gln Gly Leu Glu Trp Ile Gly
   305                 310                 315                 320
   Tyr Ile Asn Pro Ser Arg Gly Tyr Thr Asn Tyr Ala Asp Ser Val Lys
                   325                 330                 335
   Gly Arg Phe Thr Ile Thr Thr Asp Lys Ser Thr Ser Thr Ala Tyr Met
               340                 345                 350
   Glu Leu Ser Ser Leu Arg Ser Glu Asp Thr Ala Thr Tyr Tyr Cys Ala
           355                 360                 365
   Arg Tyr Tyr Asp Asp His Tyr Cys Leu Asp Tyr Trp Gly Gln Gly Thr
       370                 375                 380
   Thr Val Thr Val Ser Ser Gly Glu Gly Thr Ser Thr Gly Ser Gly Gly
   385                 390                 395                 400
   Ser Gly Gly Ser Gly Gly Ala Asp Asp Ile Val Leu Thr Gln Ser Pro
   405 410 415
   Ala Thr Leu Ser Leu Ser Pro Gly Glu Arg Ala Thr Leu Ser Cys Arg
   420 425 430
   Ala Ser Gln Ser Val Ser Tyr Met Asn Trp Tyr Gln Gln Lys Pro Gly
   435 440 445
   Lys Ala Pro Lys Arg Trp Ile Tyr Asp Thr Ser Lys Val Ala Ser Gly
   450 455 460
   Val Pro Ala Arg Phe Ser Gly Ser Gly Ser Gly Thr Asp Tyr Ser Leu
   465 470 475 480
   Thr Ile Asn Ser Leu Glu Ala Glu Asp Ala Ala Thr Tyr Tyr Cys Gln
   485 490 495
   Gln Trp Ser Ser Asn Pro Leu Thr Phe Gly Gly Gly Thr Lys Val Glu
   500 505 510
   Ile Lys His His His His His His
   515 520
<210> 13
   <211> 1560
   <212> DNA
   <213> artificial
<220>
   <223> EpCAM x CD3 x SA21
<400> 13
   gagctcgtga tgacacagtc tccatcctcc ctgactgtga cagcaggaga gaaggtcact 60
   atgagctgca agtccagtca gagtctgtta aacagtggaa atcaaaagaa ctacttgacc 120
   tggtaccagc agaaaccagg gcagcctcct aaactgttga tctactgggc atccactagg 180
   gaatctgggg tccctgatcg cttcacaggc agtggatctg gaacagattt cactctcacc 240
   atcagcagtg tgcaggctga agacctggca gtttattact gtcagaatga ttatagttat 300
   ccgctcacgt tcggtgctgg gaccaagctt gagatcaaag gtggtggtgg ttctggcggc 360
   ggcggctccg gtggtggtgg ttctgaggtg cagctgctcg agcagtctgg agctgagctg 420
   gtaaggcctg ggacttcagt gaagatatcc tgcaaggctt ctggatacgc cttcactaac 480
   tactggctag gttgggtaaa gcagaggcct ggacatggac ttgagtggat tggagatatt 540
   ttccctggaa gtggtaatat ccactacaat gagaagttca agggcaaagc cacactgact 600
   gcagacaaat cttcgagcac agcctatatg cagctcagta gcctgacatt tgaggactct 660
   gctgtctatt tctgtgcaag actgaggaac tgggacgagc ctatggacta ctggggccaa 720
   gggaccacgg tcaccgtctc ctccggaggt ggtggctccg acgtccaact ggtgcagtca 780
   ggggctgaag tgaaaaaacc tggggcctca gtgaaggtgt cctgcaaggc ttctggctac 840
   acctttacta ggtacacgat gcactgggta aggcaggcac ctggacaggg tctggaatgg 900
   attggataca ttaatcctag ccgtggttat actaattacg cagacagcgt caagggccgc 960
   ttcacaatca ctacagacaa atccaccagc acagcctaca tggaactgag cagcctgcgt 1020
   tctgaggaca ctgcaaccta ttactgtgca agatattatg atgatcatta ctgccttgac 1080
   tactggggcc aaggcaccac ggtcaccgtc tcctcaggcg aaggtactag tactggttct 1140
   ggtggaagtg gaggttcagg tggagcagac gacattgtac tgacccagtc tccagcaact 1200
   ctgtctctgt ctccagggga gcgtgccacc ctgagctgca gagccagtca aagtgtaagt 1260
   tacatgaact ggtaccagca gaagccgggc aaggcaccca aaagatggat ttatgacaca 1320
   tccaaagtgg cttctggagt ccctgctcgc ttcagtggca gtgggtctgg gaccgactac 1380
   tctctcacaa tcaacagctt ggaggctgaa gatgctgcca cttattactg ccaacagtgg 1440
   agtagtaacc cgctcacgtt cggtggcggg accaaggtgg agatcaaacg gctgatcgag 1500
   gacatctgcc tgcccagatg gggctgcctg tgggaggacg accatcatca ccatcatcat 1560
<210> 14
   <211> 520
   <212> PRT
   <213> artificial
<220>
   <223> EpCAM x CD3 x SA21
<400> 14
   Glu Leu Val Met Thr Gln Ser Pro Ser Ser Leu Thr Val Thr Ala Gly
   1               5                   10                  15
   Glu Lys Val Thr Met Ser Cys Lys Ser Ser Gln Ser Leu Leu Asn Ser
               20                  25                  30
   Gly Asn Gln Lys Asn Tyr Leu Thr Trp Tyr Gln Gln Lys Pro Gly Gln
           35                  40                  45
   Pro Pro Lys Leu Leu Ile Tyr Trp Ala Ser Thr Arg Glu Ser Gly Val
       50                  55                  60
   Pro Asp Arg Phe Thr Gly Ser Gly Ser Gly Thr Asp Phe Thr Leu Thr
   65                  70                  75                  80
   Ile Ser Ser Val Gln Ala Glu Asp Leu Ala Val Tyr Tyr Cys Gln Asn
   85 90 95
   Asp Tyr Ser Tyr Pro Leu Thr Phe Gly Ala Gly Thr Lys Leu Glu Ile
   100 105 110
   Lys Gly Gly Gly Gly Ser Gly Gly Gly Gly Ser Gly Gly Gly Gly Ser
   115 120 125
   Glu Val Gln Leu Leu Glu Gln Ser Gly Ala Glu Leu Val Arg Pro Gly
   130 135 140
   Thr Ser Val Lys Ile Ser Cys Lys Ala Ser Gly Tyr Ala Phe Thr Asn
   145 150 155 160
   Tyr Trp Leu Gly Trp Val Lys Gln Arg Pro Gly His Gly Leu Glu Trp
   165 170 175
   Ile Gly Asp Ile Phe Pro Gly Ser Gly Asn Ile His Tyr Asn Glu Lys
   180 185 190
   Phe Lys Gly Lys Ala Thr Leu Thr Ala Asp Lys Ser Ser Ser Thr Ala
   195 200 205
   Tyr Met Gln Leu Ser Ser Leu Thr Phe Glu Asp Ser Ala Val Tyr Phe
   210 215 220
   Cys Ala Arg Leu Arg Asn Trp Asp Glu Pro Met Asp Tyr Trp Gly Gln
   225 230 235 240
   Gly Thr Thr Val Thr Val Ser Ser Gly Gly Gly Gly Ser Asp Val Gln
   245 250 255
   Leu Val Gln Ser Gly Ala Glu Val Lys Lys Pro Gly Ala Ser Val Lys
   260 265 270
   Val Ser Cys Lys Ala Ser Gly Tyr Thr Phe Thr Arg Tyr Thr Met His
   275 280 285
   Trp Val Arg Gln Ala Pro Gly Gln Gly Leu Glu Trp Ile Gly Tyr Ile
   290 295 300
   Asn Pro Ser Arg Gly Tyr Thr Asn Tyr Ala Asp Ser Val Lys Gly Arg
   305 310 315 320
   Phe Thr Ile Thr Thr Asp Lys Ser Thr Ser Thr Ala Tyr Met Glu Leu
   325 330 335
   Ser Ser Leu Arg Ser Glu Asp Thr Ala Thr Tyr Tyr Cys Ala Arg Tyr
   340 345 350
   Tyr Asp Asp His Tyr Cys Leu Asp Tyr Trp Gly Gln Gly Thr Thr Val
   355 360 365
   Thr Val Ser Ser Gly Glu Gly Thr Ser Thr Gly Ser Gly Gly Ser Gly
   370 375 380
   Gly Ser Gly Gly Ala Asp Asp Ile Val Leu Thr Gln Ser Pro Ala Thr
   385 390 395 400
   Leu Ser Leu Ser Pro Gly Glu Arg Ala Thr Leu Ser Cys Arg Ala Ser
   405 410 415
   Gln Ser Val Ser Tyr Met Asn Trp Tyr Gln Gln Lys Pro Gly Lys Ala
   420 425 430
   Pro Lys Arg Trp Ile Tyr Asp Thr Ser Lys Val Ala Ser Gly Val Pro
   435 440 445
   Ala Arg Phe Ser Gly Ser Gly Ser Gly Thr Asp Tyr Ser Leu Thr Ile
   450 455 460
   Asn Ser Leu Glu Ala Glu Asp Ala Ala Thr Tyr Tyr Cys Gln Gln Trp
   465 470 475 480
   Ser Ser Asn Pro Leu Thr Phe Gly Gly Gly Thr Lys Val Glu Ile Lys
   485 490 495
   Arg Leu Ile Glu Asp Ile Cys Leu Pro Arg Trp Gly Cys Leu Trp Glu
   500 505 510
   Asp Asp His His His His His His
   515 520
<210> 15
   <211> 1593
   <212> DNA
   <213> artificial
<220>
   <223> SA21 x CEA x I2C
<400> 15
   cggctgatcg aggacatctg cctgcccaga tggggctgcc tgtgggagga cgaccaggcc 60
   gtgctgactc agccggcttc cctctctgca tctcctggag catcagccag tctcacctgc 120
   accttgcgca ggggcatcaa tgttggtgcc tacagtatat actggtacca gcagaagcca 180
   gggagtcctc cccagtatct cctgaggtac aaatcagact cagataagca gcagggctct 240
   ggagtctcca gccgcttctc tgcatccaaa gatgcttcgg ccaatgcagg gattttactc 300
   atctctgggc tccagtctga ggatgaggct gactattact gtatgatttg gcacagcggc 360
   gcttctgcgg tgttcggcgg agggaccaag ttgaccgtcc taggtggtgg tggttctggc 420
   ggcggcggct ccggtggtgg tggttctgag gtgcagctgg tcgagtctgg gggaggcttg 480
   gtccagcctg ggaggtccct gagactctcc tgtgcagcgt ctggattcac cgtcagtagc 540
   tactggatgc actgggtccg ccaagctcca gggaaggggc tggaatgggt aggtttcatt 600
   agaaacaaag ctaatggtgg gacaacagaa tacgccgcgt ctgtgaaagg cagattcacc 660
   atctcaagag atgattccaa gaacacgctg tatcttcaaa tgaacagcct gagagccgag 720
   gacacggccg tgtattactg tgcaagagat agggggctac ggttctactt tgactactgg 780
   ggccaaggga ccacggtcac cgtctcctca tccggaggtg gtggctccga ggtgcagctg 840
   gtcgagtctg gaggaggatt ggtgcagcct ggagggtcat tgaaactctc atgtgcagcc 900
   tctggattca ccttcaataa gtacgccatg aactgggtcc gccaggctcc aggaaagggt 960
   ttggaatggg ttgctcgcat aagaagtaaa tataataatt atgcaacata ttatgccgat 1020
   tcagtgaaag acaggttcac catctccaga gatgattcaa aaaacactgc ctatctacaa 1080
   atgaacaact tgaagactga ggacactgcc gtgtactact gtgtgagaca tgggaacttc 1140
   ggtaatagct acatatccta ctgggcttac tggggccaag ggactctggt caccgtctcc 1200
   tcaggtggtg gtggttctgg cggcggcggc tccggtggtg gtggttctca gactgttgtg 1260
   actcaggaac cttcactcac cgtatcacct ggtggaacag tcacactcac ttgtggctcc 1320
   tcgactgggg ctgttacatc tggcaactac ccaaactggg tccaacaaaa accaggtcag 1380
   gcaccccgtg gtctaatagg tgggactaag ttcctcgccc ccggtactcc tgccagattc 1440
   tcaggctccc tgcttggagg caaggctgcc ctcaccctct caggggtaca gccagaggat 1500
   gaggcagaat attactgtgt tctatggtac agcaaccgct gggtgttcgg tggaggaacc 1560
   aaactgactg tcctacatca tcaccatcat cat 1593
<210> 16
   <211> 531
   <212> PRT
   <213> artificial
<220>
   <223> SA21 x CEA x I2C
<400> 16
   Arg Leu Ile Glu Asp Ile Cys Leu Pro Arg Trp Gly Cys Leu Trp Glu
   1               5                   10                  15
   Asp Asp Gln Ala Val Leu Thr Gln Pro Ala Ser Leu Ser Ala Ser Pro
               20                  25                  30
   Gly Ala Ser Ala Ser Leu Thr Cys Thr Leu Arg Arg Gly Ile Asn Val
           35                  40                  45
   Gly Ala Tyr Ser Ile Tyr Trp Tyr Gln Gln Lys Pro Gly Ser Pro Pro
       50                  55                  60
   Gln Tyr Leu Leu Arg Tyr Lys Ser Asp Ser Asp Lys Gln Gln Gly Ser
   65                  70                  75                  80
   Gly Val Ser Ser Arg Phe Ser Ala Ser Lys Asp Ala Ser Ala Asn Ala
                   85                  90                  95
   Gly Ile Leu Leu Ile Ser Gly Leu Gln Ser Glu Asp Glu Ala Asp Tyr
               100                 105                 110
   Tyr Cys Met Ile Trp His Ser Gly Ala Ser Ala Val Phe Gly Gly Gly
           115                 120                 125
   Thr Lys Leu Thr Val Leu Gly Gly Gly Gly Ser Gly Gly Gly Gly Ser
       130                 135                 140
   Gly Gly Gly Gly Ser Glu Val Gln Leu Val Glu Ser Gly Gly Gly Leu
   145                 150                 155                 160
   Val Gln Pro Gly Arg Ser Leu Arg Leu Ser Cys Ala Ala Ser Gly Phe
                   165                 170                 175
   Thr Val Ser Ser Tyr Trp Met His Trp Val Arg Gln Ala Pro Gly Lys
               180                 185                 190
   Gly Leu Glu Trp Val Gly Phe Ile Arg Asn Lys Ala Asn Gly Gly Thr
           195                 200                 205
   Thr Glu Tyr Ala Ala Ser Val Lys Gly Arg Phe Thr Ile Ser Arg Asp
       210                 215                 220
   Asp Ser Lys Asn Thr Leu Tyr Leu Gln Met Asn Ser Leu Arg Ala Glu
   225                 230                 235                 240
   Asp Thr Ala Val Tyr Tyr Cys Ala Arg Asp Arg Gly Leu Arg Phe Tyr
                   245                 250                 255
   Phe Asp Tyr Trp Gly Gln Gly Thr Thr Val Thr Val Ser Ser Ser Gly
               260                 265                 270
   Gly Gly Gly Ser Glu Val Gln Leu Val Glu Ser Gly Gly Gly Leu Val
   275 280 285
   Gln Pro Gly Gly Ser Leu Lys Leu Ser Cys Ala Ala Ser Gly Phe Thr
   290 295 300
   Phe Asn Lys Tyr Ala Met Asn Trp Val Arg Gln Ala Pro Gly Lys Gly
   305 310 315 320
   Leu Glu Trp Val Ala Arg Ile Arg Ser Lys Tyr Asn Asn Tyr Ala Thr
   325 330 335
   Tyr Tyr Ala Asp Ser Val Lys Asp Arg Phe Thr Ile Ser Arg Asp Asp
   340 345 350
   Ser Lys Asn Thr Ala Tyr Leu Gln Met Asn Asn Leu Lys Thr Glu Asp
   355 360 365
   Thr Ala Val Tyr Tyr Cys Val Arg His Gly Asn Phe Gly Asn Ser Tyr
   370 375 380
   Ile Ser Tyr Trp Ala Tyr Trp Gly Gln Gly Thr Leu Val Thr Val Ser
   385 390 395 400
   Ser Gly Gly Gly Gly Ser Gly Gly Gly Gly Ser Gly Gly Gly Gly Ser
   405 410 415
   Gln Thr Val Val Thr Gln Glu Pro Ser Leu Thr Val Ser Pro Gly Gly
   420 425 430
   Thr Val Thr Leu Thr Cys Gly Ser Ser Thr Gly Ala Val Thr Ser Gly
   435 440 445
   Asn Tyr Pro Asn Trp Val Gln Gln Lys Pro Gly Gln Ala Pro Arg Gly
   450 455 460
   Leu Ile Gly Gly Thr Lys Phe Leu Ala Pro Gly Thr Pro Ala Arg Phe
   465 470 475 480
   Ser Gly Ser Leu Leu Gly Gly Lys Ala Ala Leu Thr Leu Ser Gly Val
   485 490 495
   Gln Pro Glu Asp Glu Ala Glu Tyr Tyr Cys Val Leu Trp Tyr Ser Asn
   500 505 510
   Arg Trp Val Phe Gly Gly Gly Thr Lys Leu Thr Val Leu His His His
   515 520 525
   His His His
   530
<210> 17
   <211> 1593
   <212> DNA
   <213> artificial
<220>
   <223> CEA × I2C × SA21
<400> 17
   caggccgtgc tgactcagcc ggcttccctc tctgcatctc ctggagcatc agccagtctc        60
   acctgcacct tgcgcagggg catcaatgtt ggtgcctaca gtatatactg gtaccagcag       120
   aagccaggga gtcctcccca gtatctcctg aggtacaaat cagactcaga taagcagcag       180
   ggctctggag tctccagccg cttctctgca tccaaagatg cttcggccaa tgcagggatt       240
   ttactcatct ctgggctcca gtctgaggat gaggctgact attactgtat gatttggcac       300
   agcggcgctt ctgcggtgtt cggcggaggg accaagttga ccgtcctagg tggtggtggt       360
   tctggcggcg gcggctccgg tggtggtggt tctgaggtgc agctggtcga gtctggggga       420
   ggcttggtcc agcctgggag gtccctgaga ctctcctgtg cagcgtctgg attcaccgtc       480
   agtagctact ggatgcactg ggtccgccaa gctccaggga aggggctgga atgggtaggt       540
   ttcattagaa acaaagctaa tggtgggaca acagaatacg ccgcgtctgt gaaaggcaga       600
   ttcaccatct caagagatga ttccaagaac acgctgtatc ttcaaatgaa cagcctgaga       660
   gccgaggaca cggccgtgta ttactgtgca agagataggg ggctacggtt ctactttgac       720
   tactggggcc aagggaccac ggtcaccgtc tcctcatccg gaggtggtgg ctccgaggtg       780
   cagctggtcg agtctggagg aggattggtg cagcctggag ggtcattgaa actctcatgt       840
   gcagcctctg gattcacctt caataagtac gccatgaact gggtccgcca ggctccagga       900
   aagggtttgg aatgggttgc tcgcataaga agtaaatata ataattatgc aacatattat       960
   gccgattcag tgaaagacag gttcaccatc tccagagatg attcaaaaaa cactgcctat      1020
   ctacaaatga acaacttgaa gactgaggac actgccgtgt actactgtgt gagacatggg      1080
   aacttcggta atagctacat atcctactgg gcttactggg gccaagggac tctggtcacc      1140
   gtctcctcag gtggtggtgg ttctggcggc ggcggctccg gtggtggtgg ttctcagact      1200
   gttgtgactc aggaaccttc actcaccgta tcacctggtg gaacagtcac actcacttgt      1260
   ggctcctcga ctggggctgt tacatctggc aactacccaa actgggtcca acaaaaacca      1320
   ggtcaggcac cccgtggtct aataggtggg actaagttcc tcgcccccgg tactcctgcc      1380
   agattctcag gctccctgct tggaggcaag gctgccctca ccctctcagg ggtacagcca      1440
   gaggatgagg cagaatatta ctgtgttcta tggtacagca accgctgggt gttcggtgga      1500
   ggaaccaaac tgactgtcct acggctgatc gaggacatct gcctgcccag atggggctgc      1560
   ctgtgggagg acgaccatca tcaccatcat cat                                   1593
<210> 18
   <211> 531
   <212> PRT
   <213> artificial
<220>
   <223> CEA x I2C x SA21
<400> 18
   Gln Ala Val Leu Thr Gln Pro Ala Ser Leu Ser Ala Ser Pro Gly Ala
   1               5                   10                  15
   Ser Ala Ser Leu Thr Cys Thr Leu Arg Arg Gly Ile Asn Val Gly Ala
               20                  25                  30
   Tyr Ser Ile Tyr Trp Tyr Gln Gln Lys Pro Gly Ser Pro Pro Gln Tyr
           35                  40                  45
   Leu Leu Arg Tyr Lys Ser Asp Ser Asp Lys Gln Gln Gly Ser Gly Val
       50                  55                  60
   Ser Ser Arg Phe Ser Ala Ser Lys Asp Ala Ser Ala Asn Ala Gly Ile
   65                  70                  75                  80
   Leu Leu Ile Ser Gly Leu Gln Ser Glu Asp Glu Ala Asp Tyr Tyr Cys
                   85                  90                  95
   Met Ile Trp His Ser Gly Ala Ser Ala Val Phe Gly Gly Gly Thr Lys
               100                 105                 110
   Leu Thr Val Leu Gly Gly Gly Gly Ser Gly Gly Gly Gly Ser Gly Gly
           115                 120                 125
   Gly Gly Ser Glu Val Gln Leu Val Glu Ser Gly Gly Gly Leu Val Gln
       130                 135                 140
   Pro Gly Arg Ser Leu Arg Leu Ser Cys Ala Ala Ser Gly Phe Thr Val
   145                 150                 155                 160
   Ser Ser Tyr Trp Met His Trp Val Arg Gln Ala Pro Gly Lys Gly Leu
                   165                 170                 175
   Glu Trp Val Gly Phe Ile Arg Asn Lys Ala Asn Gly Gly Thr Thr Glu
   180 185 190
   Tyr Ala Ala Ser Val Lys Gly Arg Phe Thr Ile Ser Arg Asp Asp Ser
   195 200 205
   Lys Asn Thr Leu Tyr Leu Gln Met Asn Ser Leu Arg Ala Glu Asp Thr
   210 215 220
   Ala Val Tyr Tyr Cys Ala Arg Asp Arg Gly Leu Arg Phe Tyr Phe Asp
   225 230 235 240
   Tyr Trp Gly Gln Gly Thr Thr Val Thr Val Ser Ser Ser Gly Gly Gly
   245 250 255
   Gly Ser Glu Val Gln Leu Val Glu Ser Gly Gly Gly Leu Val Gln Pro
   260 265 270
   Gly Gly Ser Leu Lys Leu Ser Cys Ala Ala Ser Gly Phe Thr Phe Asn
   275 280 285
   Lys Tyr Ala Met Asn Trp Val Arg Gln Ala Pro Gly Lys Gly Leu Glu
   290 295 300
   Trp Val Ala Arg Ile Arg Ser Lys Tyr Asn Asn Tyr Ala Thr Tyr Tyr
   305 310 315 320
   Ala Asp Ser Val Lys Asp Arg Phe Thr Ile Ser Arg Asp Asp Ser Lys
   325 330 335
   Asn Thr Ala Tyr Leu Gln Met Asn Asn Leu Lys Thr Glu Asp Thr Ala
   340 345 350
   Val Tyr Tyr Cys Val Arg His Gly Asn Phe Gly Asn Ser Tyr Ile Ser
   355 360 365
   Tyr Trp Ala Tyr Trp Gly Gln Gly Thr Leu Val Thr Val Ser Ser Gly
   370 375 380
   Gly Gly Gly Ser Gly Gly Gly Gly Ser Gly Gly Gly Gly Ser Gln Thr
   385 390 395 400
   Val Val Thr Gln Glu Pro Ser Leu Thr Val Ser Pro Gly Gly Thr Val
   405 410 415
   Thr Leu Thr Cys Gly Ser Ser Thr Gly Ala Val Thr Ser Gly Asn Tyr
   420 425 430
   Pro Asn Trp Val Gln Gln Lys Pro Gly Gln Ala Pro Arg Gly Leu Ile
           435                 440                 445
   Gly Gly Thr Lys Phe Leu Ala Pro Gly Thr Pro Ala Arg Phe Ser Gly
       450                 455                 460
   Ser Leu Leu Gly Gly Lys Ala Ala Leu Thr Leu Ser Gly Val Gln Pro
   465                 470                 475                 480
   Glu Asp Glu Ala Glu Tyr Tyr Cys Val Leu Trp Tyr Ser Asn Arg Trp
                   485                 490                 495
   Val Phe Gly Gly Gly Thr Lys Leu Thr Val Leu Arg Leu Ile Glu Asp
               500                 505                 510
   Ile Cys Leu Pro Arg Trp Gly Cys Leu Trp Glu Asp Asp His His His
           515                 520                 525
   His His His
       530
<210> 19
   <211> 1581
   <212> DNA
   <213> artificial
<220>
   <223> SA25 x CEA x I2C
<400> 19
   gaggacatct gcctgcccag atggggctgc ctgtgggagg accaggccgt gctgactcag      60
   ccggcttccc tctctgcatc tcctggagca tcagccagtc tcacctgcac cttgcgcagg     120
   ggcatcaatg ttggtgccta cagtatatac tggtaccagc agaagccagg gagtcctccc     180
   cagtatctcc tgaggtacaa atcagactca gataagcagc agggctctgg agtctccagc     240
   cgcttctctg catccaaaga tgcttcggcc aatgcaggga ttttactcat ctctgggctc     300
   cagtctgagg atgaggctga ctattactgt atgatttggc acagcggcgc ttctgcggtg     360
   ttcggcggag ggaccaagtt gaccgtccta ggtggtggtg gttctggcgg cggcggctcc     420
   ggtggtggtg gttctgaggt gcagctggtc gagtctgggg gaggcttggt ccagcctggg     480
   aggtccctga gactctcctg tgcagcgtct ggattcaccg tcagtagcta ctggatgcac     540
   tgggtccgcc aagctccagg gaaggggctg gaatgggtag gtttcattag aaacaaagct     600
   aatggtggga caacagaata cgccgcgtct gtgaaaggca gattcaccat ctcaagagat     660
   gattccaaga acacgctgta tcttcaaatg aacagcctga gagccgagga cacggccgtg     720
   tattactgtg caagagatag gggctacggt ttctactttg actactgggg ccaagggacc     780
   acggtcaccg tctcctcatc cggaggtggt ggctccgagg tgcagctggt cgagtctgga     840
   ggaggattgg tgcagcctgg agggtcattg aaactctcat gtgcagcctc tggattcacc     900
   ttcaataagt acgccatgaa ctgggtccgc caggctccag gaaagggttt ggaatgggtt     960
   gctcgcataa gaagtaaata taataattat gcaacatatt atgccgattc agtgaaagac    1020
   aggttcacca tctccagaga tgattcaaaa aacactgcct atctacaaat gaacaacttg    1080
   aagactgagg acactgccgt gtactactgt gtgagacatg ggaacttcgg taatagctac    1140
   atatcctact gggcttactg gggccaaggg actctggtca ccgtctcctc aggtggtggt    1200
   ggttctggcg gcggcggctc cggtggtggt ggttctcaga ctgttgtgac tcaggaacct    1260
   tcactcaccg tatcacctgg tggaacagtc acactcactt gtggctcctc gactggggct    1320
   gttacatctg gcaactaccc aaactgggtc caacaaaaac caggtcaggc accccgtggt    1380
   ctaataggtg ggactaagtt cctcgccccc ggtactcctg ccagattctc aggctccctg    1440
   cttggaggca aggctgccct caccctctca ggggtacagc cagaggatga ggcagaatat    1500
   tactgtgttc tatggtacag caaccgctgg gtgttcggtg gaggaaccaa actgactgtc    1560
   ctacatcatc accatcatca t                                              1581
<210> 20
   <211> 527
   <212> PRT
   <213> artificial
<220>
   <223> SA25 x CEA x I2C
<400> 20
   Glu Asp Ile Cys Leu Pro Arg Trp Gly Cys Leu Trp Glu Asp Gln Ala
   1               5                   10                  15
   Val Leu Thr Gln Pro Ala Ser Leu Ser Ala Ser Pro Gly Ala Ser Ala
               20                  25                  30
   Ser Leu Thr Cys Thr Leu Arg Arg Gly Ile Asn Val Gly Ala Tyr Ser
           35                  40                  45
   Ile Tyr Trp Tyr Gln Gln Lys Pro Gly Ser Pro Pro Gln Tyr Leu Leu
       50                  55                  60
   Arg Tyr Lys Ser Asp Ser Asp Lys Gln Gln Gly Ser Gly Val Ser Ser
   65                  70                  75                  80
   Arg Phe Ser Ala Ser Lys Asp Ala Ser Ala Asn Ala Gly Ile Leu Leu
                   85                  90                  95
   Ile Ser Gly Leu Gln Ser Glu Asp Glu Ala Asp Tyr Tyr Cys Met Ile
   100 105 110
   Trp His Ser Gly Ala Ser Ala Val Phe Gly Gly Gly Thr Lys Leu Thr
   115 120 125
   Val Leu Gly Gly Gly Gly Ser Gly Gly Gly Gly Ser Gly Gly Gly Gly
   130 135 140
   Ser Glu Val Gln Leu Val Glu Ser Gly Gly Gly Leu Val Gln Pro Gly
   145 150 155 160
   Arg Ser Leu Arg Leu Ser Cys Ala Ala Ser Gly Phe Thr Val Ser Ser
   165 170 175
   Tyr Trp Met His Trp Val Arg Gln Ala Pro Gly Lys Gly Leu Glu Trp
   180 185 190
   Val Gly Phe Ile Arg Asn Lys Ala Asn Gly Gly Thr Thr Glu Tyr Ala
   195 200 205
   Ala Ser Val Lys Gly Arg Phe Thr Ile Ser Arg Asp Asp Ser Lys Asn
   210 215 220
   Thr Leu Tyr Leu Gln Met Asn Ser Leu Arg Ala Glu Asp Thr Ala Val
   225 230 235 240
   Tyr Tyr Cys Ala Arg Asp Arg Gly Leu Arg Phe Tyr Phe Asp Tyr Trp
   245 250 255
   Gly Gln Gly Thr Thr Val Thr Val Ser Ser Ser Gly Gly Gly Gly Ser
   260 265 270
   Glu Val Gln Leu Val Glu Ser Gly Gly Gly Leu Val Gln Pro Gly Gly
   275 280 285
   Ser Leu Lys Leu Ser Cys Ala Ala Ser Gly Phe Thr Phe Asn Lys Tyr
   290 295 300
   Ala Met Asn Trp Val Arg Gln Ala Pro Gly Lys Gly Leu Glu Trp Val
   305 310 315 320
   Ala Arg Ile Arg Ser Lys Tyr Asn Asn Tyr Ala Thr Tyr Tyr Ala Asp
   325 330 335
   Ser Val Lys Asp Arg Phe Thr Ile Ser Arg Asp Asp Ser Lys Asn Thr
   340 345 350
   Ala Tyr Leu Gln Met Asn Asn Leu Lys Thr Glu Asp Thr Ala Val Tyr
           355                 360                 365
   Tyr Cys Val Arg His Gly Asn Phe Gly Asn Ser Tyr Ile Ser Tyr Trp
       370                 375                 380
   Ala Tyr Trp Gly Gln Gly Thr Leu Val Thr Val Ser Ser Gly Gly Gly
   385                 390                 395                 400
   Gly Ser Gly Gly Gly Gly Ser Gly Gly Gly Gly Ser Gln Thr Val Val
                   405                 410                 415
   Thr Gln Glu Pro Ser Leu Thr Val Ser Pro Gly Gly Thr Val Thr Leu
               420                 425                 430
   Thr Cys Gly Ser Ser Thr Gly Ala Val Thr Ser Gly Asn Tyr Pro Asn
           435                 440                 445
   Trp Val Gln Gln Lys Pro Gly Gln Ala Pro Arg Gly Leu Ile Gly Gly
       450                 455                 460
   Thr Lys Phe Leu Ala Pro Gly Thr Pro Ala Arg Phe Ser Gly Ser Leu
   465                 470                 475                 480
   Leu Gly Gly Lys Ala Ala Leu Thr Leu Ser Gly Val Gln Pro Glu Asp
                   485                 490                 495
   Glu Ala Glu Tyr Tyr Cys Val Leu Trp Tyr Ser Asn Arg Trp Val Phe
               500                 505                 510
   Gly Gly Gly Thr Lys Leu Thr Val Leu His His His His His His
           515                 520                 525
<210> 21
   <211> 1581
   <212> DNA
   <213> artificial
<220>
   <223> CEA x I2C x SA25
<400> 21
   caggccgtgc tgactcagcc ggcttccctc tctgcatctc ctggagcatc agccagtctc      60
   acctgcacct tgcgcagggg catcaatgtt ggtgcctaca gtatatactg gtaccagcag     120
   aagccaggga gtcctcccca gtatctcctg aggtacaaat cagactcaga taagcagcag     180
   ggctctggag tctccagccg cttctctgca tccaaagatg cttcggccaa tgcagggatt     240
   ttactcatct ctgggctcca gtctgaggat gaggctgact attactgtat gatttggcac     300
   agcggcgctt ctgcggtgtt cggcggaggg accaagttga ccgtcctagg tggtggtggt     360
   tctggcggcg gcggctccgg tggtggtggt tctgaggtgc agctggtcga gtctggggga     420
   ggcttggtcc agcctgggag gtccctgaga ctctcctgtg cagcgtctgg attcaccgtc     480
   agtagctact ggatgcactg ggtccgccaa gctccaggga aggggctgga atgggtaggt     540
   ttcattagaa acaaagctaa tggtgggaca acagaatacg ccgcgtctgt gaaaggcaga     600
   ttcaccatct caagagatga ttccaagaac acgctgtatc ttcaaatgaa cagcctgaga     660
   gccgaggaca cggccgtgta ttactgtgca agagataggg ggctacggtt ctactttgac     720
   tactggggcc aagggaccac ggtcaccgtc tcctcatccg gaggtggtgg ctccgaggtg     780
   cagctggtcg agtctggagg aggattggtg cagcctggag ggtcattgaa actctcatgt     840
   gcagcctctg gattcacctt caataagtac gccatgaact gggtccgcca ggctccagga     900
   aagggtttgg aatgggttgc tcgcataaga agtaaatata ataattatgc aacatattat     960
   gccgattcag tgaaagacag gttcaccatc tccagagatg attcaaaaaa cactgcctat    1020
   ctacaaatga acaacttgaa gactgaggac actgccgtgt actactgtgt gagacatggg    1080
   aacttcggta atagctacat atcctactgg gcttactggg gccaagggac tctggtcacc    1140
   gtctcctcag gtggtggtgg ttctggcggc ggcggctccg gtggtggtgg ttctcagact    1200
   gttgtgactc aggaaccttc actcaccgta tcacctggtg gaacagtcac actcacttgt    1260
   ggctcctcga ctggggctgt tacatctggc aactacccaa actgggtcca acaaaaacca    1320
   ggtcaggcac cccgtggtct aataggtggg actaagttcc tcgcccccgg tactcctgcc    1380
   agattctcag gctccctgct tggaggcaag gctgccctca ccctctcagg ggtacagcca    1440
   gaggatgagg cagaatatta ctgtgttcta tggtacagca accgctgggt gttcggtgga    1500
   ggaaccaaac tgactgtcct agaggacatc tgcctgccca gatggggctg cctgtgggag    1560
   gaccatcatc accatcatca t                                              1581
<210> 22
   <211> 527
   <212> PRT
   <213> artificial
<220>
   <223> CEA x I2C x SA25
<400> 22
   Gln Ala Val Leu Thr Gln Pro Ala Ser Leu Ser Ala Ser Pro Gly Ala
   1               5                   10                  15
   Ser Ala Ser Leu Thr Cys Thr Leu Arg Arg Gly Ile Asn Val Gly Ala
               20                  25                  30
   Tyr Ser Ile Tyr Trp Tyr Gln Gln Lys Pro Gly Ser Pro Pro Gln Tyr
           35                  40                  45
   Leu Leu Arg Tyr Lys Ser Asp Ser Asp Lys Gln Gln Gly Ser Gly Val
       50                  55                  60
   Ser Ser Arg Phe Ser Ala Ser Lys Asp Ala Ser Ala Asn Ala Gly Ile
   65                  70                  75                  80
   Leu Leu Ile Ser Gly Leu Gln Ser Glu Asp Glu Ala Asp Tyr Tyr Cys
                   85                  90                  95
   Met Ile Trp His Ser Gly Ala Ser Ala Val Phe Gly Gly Gly Thr Lys
               100                 105                 110
   Leu Thr Val Leu Gly Gly Gly Gly Ser Gly Gly Gly Gly Ser Gly Gly
           115                 120                 125
   Gly Gly Ser Glu Val Gln Leu Val Glu Ser Gly Gly Gly Leu Val Gln
       130                 135                 140
   Pro Gly Arg Ser Leu Arg Leu Ser Cys Ala Ala Ser Gly Phe Thr Val
   145                 150                 155                 160
   Ser Ser Tyr Trp Met His Trp Val Arg Gln Ala Pro Gly Lys Gly Leu
                   165                 170                 175
   Glu Trp Val Gly Phe Ile Arg Asn Lys Ala Asn Gly Gly Thr Thr Glu
               180                 185                 190
   Tyr Ala Ala Ser Val Lys Gly Arg Phe Thr Ile Ser Arg Asp Asp Ser
           195                 200                 205
   Lys Asn Thr Leu Tyr Leu Gln Met Asn Ser Leu Arg Ala Glu Asp Thr
       210                 215                 220
   Ala Val Tyr Tyr Cys Ala Arg Asp Arg Gly Leu Arg Phe Tyr Phe Asp
   225                 230                 235                 240
   Tyr Trp Gly Gln Gly Thr Thr Val Thr Val Ser Ser Ser Gly Gly Gly
                   245                 250                 255
   Gly Ser Glu Val Gln Leu Val Glu Ser Gly Gly Gly Leu Val Gln Pro
               260                 265                 270
   Gly Gly Ser Leu Lys Leu Ser Cys Ala Ala Ser Gly Phe Thr Phe Asn
           275                 280                 285
   Lys Tyr Ala Met Asn Trp Val Arg Gln Ala Pro Gly Lys Gly Leu Glu
       290                 295                 300
   Trp Val Ala Arg Ile Arg Ser Lys Tyr Asn Asn Tyr Ala Thr Tyr Tyr
   305                 310                 315                 320
   Ala Asp Ser Val Lys Asp Arg Phe Thr Ile Ser Arg Asp Asp Ser Lys
                   325                 330                 335
   Asn Thr Ala Tyr Leu Gln Met Asn Asn Leu Lys Thr Glu Asp Thr Ala
               340                 345                 350
   Val Tyr Tyr Cys Val Arg His Gly Asn Phe Gly Asn Ser Tyr Ile Ser
           355                 360                 365
   Tyr Trp Ala Tyr Trp Gly Gln Gly Thr Leu Val Thr Val Ser Ser Gly
       370                 375                 380
   Gly Gly Gly Ser Gly Gly Gly Gly Ser Gly Gly Gly Gly Ser Gln Thr
   385                 390                 395                 400
   Val Val Thr Gln Glu Pro Ser Leu Thr Val Ser Pro Gly Gly Thr Val
                   405                 410                 415
   Thr Leu Thr Cys Gly Ser Ser Thr Gly Ala Val Thr Ser Gly Asn Tyr
               420                 425                 430
   Pro Asn Trp Val Gln Gln Lys Pro Gly Gln Ala Pro Arg Gly Leu Ile
           435                 440                 445
   Gly Gly Thr Lys Phe Leu Ala Pro Gly Thr Pro Ala Arg Phe Ser Gly
       450                 455                 460
   Ser Leu Leu Gly Gly Lys Ala Ala Leu Thr Leu Ser Gly Val Gln Pro
   465                 470                 475                 480
   Glu Asp Glu Ala Glu Tyr Tyr Cys Val Leu Trp Tyr Ser Asn Arg Trp
                   485                 490                 495
   Val Phe Gly Gly Gly Thr Lys Leu Thr Val Leu Glu Asp Ile Cys Leu
               500                 505                 510
   Pro Arg Trp Gly Cys Leu Trp Glu Asp His His His His His His
           515                 520                 525
<210> 23
   <211> 1602
   <212> DNA
   <213> artificial
<220>
   <223> SA08 x CEA x I2C
<400> 23
   cagggcctga tcggcgacat ctgcctgccc agatggggct gcctgtgggg cgactccgtg      60
   aaacaggccg tgctgactca gccggcttcc ctctctgcat ctcctggagc atcagccagt     120
   ctcacctgca ccttgcgcag gggcatcaat gttggtgcct acagtatata ctggtaccag     180
   cagaagccag ggagtcctcc ccagtatctc ctgaggtaca aatcagactc agataagcag     240
   cagggctctg gagtctccag ccgcttctct gcatccaaag atgcttcggc caatgcaggg     300
   attttactca tctctgggct ccagtctgag gatgaggctg actattactg tatgatttgg     360
   cacagcggcg cttctgcggt gttcggcgga gggaccaagt tgaccgtcct aggtggtggt     420
   ggttctggcg gcggcggctc cggtggtggt ggttctgagg tgcagctggt cgagtctggg     480
   ggaggcttgg tccagcctgg gaggtccctg agactctcct gtgcagcgtc tggattcacc     540
   gtcagtagct actggatgca ctgggtccgc caagctccag ggaaggggct ggaatgggta     600
   ggtttcatta gaaacaaagc taatggtggg acaacagaat acgccgcgtc tgtgaaaggc     660
   agattcacca tctcaagaga tgattccaag aacacgctgt atcttcaaat gaacagcctg     720
   agagccgagg acacggccgt gtattactgt gcaagagata gggggctacg gttctacttt     780
   gactactggg gccaagggac cacggtcacc gtctcctcat ccggaggtgg tggctccgag     840
   gtgcagctgg tcgagtctgg aggaggattg gtgcagcctg gagggtcatt gaaactctca     900
   tgtgcagcct ctggattcac cttcaataag tacgccatga actgggtccg ccaggctcca     960
   ggaaagggtt tggaatgggt tgctcgcata agaagtaaat ataataatta tgcaacatat    1020
   tatgccgatt cagtgaaaga caggttcacc atctccagag atgattcaaa aaacactgcc    1080
   tatctacaaa tgaacaactt gaagactgag gacactgccg tgtactactg tgtgagacat    1140
   gggaacttcg gtaatagcta catatcctac tgggcttact ggggccaagg gactctggtc    1200
   accgtctcct caggtggtgg tggttctggc ggcggcggct ccggtggtgg tggttctcag    1260
   actgttgtga ctcaggaacc ttcactcacc gtatcacctg gtggaacagt cacactcact    1320
   tgtggctcct cgactggggc tgttacatct ggcaactacc caaactgggt ccaacaaaaa    1380
   ccaggtcagg caccccgtgg tctaataggt gggactaagt tcctcgcccc cggtactcct    1440
   gccagattct caggctccct gcttggaggc aaggctgccc tcaccctctc aggggtacag    1500
   ccagaggatg aggcagaata ttactgtgtt ctatggtaca gcaaccgctg ggtgttcggt    1560
   ggaggaacca aactgactgt cctacatcat caccatcatc at                       1602
<210> 24
   <211> 534
   <212> PRT
   <213> artificial
<220>
   <223> SA08 x CEA x I2C
<400> 24
   Gln Gly Leu Ile Gly Asp Ile Cys Leu Pro Arg Trp Gly Cys Leu Trp
   1               5                   10                  15
   Gly Asp Ser Val Lys Gln Ala Val Leu Thr Gln Pro Ala Ser Leu Ser
               20                  25                  30
   Ala Ser Pro Gly Ala Ser Ala Ser Leu Thr Cys Thr Leu Arg Arg Gly
           35                  40                  45
   Ile Asn Val Gly Ala Tyr Ser Ile Tyr Trp Tyr Gln Gln Lys Pro Gly
       50                  55                  60
   Ser Pro Pro Gln Tyr Leu Leu Arg Tyr Lys Ser Asp Ser Asp Lys Gln
   65                  70                  75                  80
   Gln Gly Ser Gly Val Ser Ser Arg Phe Ser Ala Ser Lys Asp Ala Ser
                   85                  90                  95
   Ala Asn Ala Gly Ile Leu Leu Ile Ser Gly Leu Gln Ser Glu Asp Glu
               100                 105                 110
   Ala Asp Tyr Tyr Cys Met Ile Trp His Ser Gly Ala Ser Ala Val Phe
           115                 120                 125
   Gly Gly Gly Thr Lys Leu Thr Val Leu Gly Gly Gly Gly Ser Gly Gly
       130                 135                 140
   Gly Gly Ser Gly Gly Gly Gly Ser Glu Val Gln Leu Val Glu Ser Gly
   145                 150                 155                 160
   Gly Gly Leu Val Gln Pro Gly Arg Ser Leu Arg Leu Ser Cys Ala Ala
                   165                 170                 175
   Ser Gly Phe Thr Val Ser Ser Tyr Trp Met His Trp Val Arg Gln Ala
               180                 185                 190
   Pro Gly Lys Gly Leu Glu Trp Val Gly Phe Ile Arg Asn Lys Ala Asn
           195                 200                 205
   Gly Gly Thr Thr Glu Tyr Ala Ala Ser Val Lys Gly Arg Phe Thr Ile
   210 215 220
   Ser Arg Asp Asp Ser Lys Asn Thr Leu Tyr Leu Gln Met Asn Ser Leu
   225 230 235 240
   Arg Ala Glu Asp Thr Ala Val Tyr Tyr Cys Ala Arg Asp Arg Gly Leu
   245 250 255
   Arg Phe Tyr Phe Asp Tyr Trp Gly Gln Gly Thr Thr Val Thr Val Ser
   260 265 270
   Ser Ser Gly Gly Gly Gly Ser Glu Val Gln Leu Val Glu Ser Gly Gly
   275 280 285
   Gly Leu Val Gln Pro Gly Gly Ser Leu Lys Leu Ser Cys Ala Ala Ser
   290 295 300
   Gly Phe Thr Phe Asn Lys Tyr Ala Met Asn Trp Val Arg Gln Ala Pro
   305 310 315 320
   Gly Lys Gly Leu Glu Trp Val Ala Arg Ile Arg Ser Lys Tyr Asn Asn
   325 330 335
   Tyr Ala Thr Tyr Tyr Ala Asp Ser Val Lys Asp Arg Phe Thr Ile Ser
   340 345 350
   Arg Asp Asp Ser Lys Asn Thr Ala Tyr Leu Gln Met Asn Asn Leu Lys
   355 360 365
   Thr Glu Asp Thr Ala Val Tyr Tyr Cys Val Arg His Gly Asn Phe Gly
   370 375 380
   Asn Ser Tyr Ile Ser Tyr Trp Ala Tyr Trp Gly Gln Gly Thr Leu Val
   385 390 395 400
   Thr Val Ser Ser Gly Gly Gly Gly Ser Gly Gly Gly Gly Ser Gly Gly
   405 410 415
   Gly Gly Ser Gln Thr Val Val Thr Gln Glu Pro Ser Leu Thr Val Ser
   420 425 430
   Pro Gly Gly Thr Val Thr Leu Thr Cys Gly Ser Ser Thr Gly Ala Val
   435 440 445
   Thr Ser Gly Asn Tyr Pro Asn Trp Val Gln Gln Lys Pro Gly Gln Ala
   450 455 460
   Pro Arg Gly Leu Ile Gly Gly Thr Lys Phe Leu Ala Pro Gly Thr Pro
   465                 470                 475                 480
   Ala Arg Phe Ser Gly Ser Leu Leu Gly Gly Lys Ala Ala Leu Thr Leu
                   485                 490                 495
   Ser Gly Val Gln Pro Glu Asp Glu Ala Glu Tyr Tyr Cys Val Leu Trp
               500                 505                 510
   Tyr Ser Asn Arg Trp Val Phe Gly Gly Gly Thr Lys Leu Thr Val Leu
           515                 520                 525
   His His His His His His
       530
<210> 25
   <211> 1575
   <212> DNA
   <213> artificial
<220>
   <223> SA21 x CEA x I2C H0
<400> 25
   cggctgatcg aggacatctg cctgcccaga tggggctgcc tgtgggagga cgaccaggcc      60
   gtgctgactc agccggcttc cctctctgca tctcctggag catcagccag tctcacctgc     120
   accttgcgca ggggcatcaa tgttggtgcc tacagtatat actggtacca gcagaagcca     180
   gggagtcctc cccagtatct cctgaggtac aaatcagact cagataagca gcagggctct     240
   ggagtctcca gccgcttctc tgcatccaaa gatgcttcgg ccaatgcagg gattttactc     300
   atctctgggc tccagtctga ggatgaggct gactattact gtatgatttg gcacagcggc     360
   gcttctgcgg tgttcggcgg agggaccaag ttgaccgtcc taggtggtgg tggttctggc     420
   ggcggcggct ccggtggtgg tggttctgag gtgcagctgg tcgagtctgg gggaggcttg     480
   gtccagcctg ggaggtccct gagactctcc tgtgcagcgt ctggattcac cgtcagtagc     540
   tactggatgc actgggtccg ccaagctcca gggaaggggc tggaatgggt aggtttcatt     600
   agaaacaaag ctaatggtgg gacaacagaa tacgccgcgt ctgtgaaagg cagattcacc     660
   atctcaagag atgattccaa gaacacgctg tatcttcaaa tgaacagcct gagagccgag     720
   gacacggccg tgtattactg tgcaagagat agggggctac ggttctactt tgactactgg      780
   ggccaaggga ccacggtcac cgtctcctca tccggaggtg gtggctccga ggtgcagctg     840
   gtcgagtctg gaggaggatt ggtgcagcct ggagggtcat tgaaactctc atgtgcagcc     900
   tctggattca ccttcaataa gtacgccatg aactgggtcc gccaggctcc aggaaagggt     960
   ttggaatggg ttgctcgcat aagaagtaaa tataataatt atgcaacata ttatgccgat    1020
   tcagtgaaag acaggttcac catctccaga gatgattcaa aaaacactgc ctatctacaa    1080
   atgaacaact tgaagactga ggacactgcc gtgtactact gtgtgagaca tgggaacttc    1140
   ggtaatagct acatatccta ctgggcttac tggggccaag ggactctggt caccgtctcc    1200
   tcaggtggtg gtggttctgg cggcggcggc tccggtggtg gtggttctca gactgttgtg    1260
   actcaggaac cttcactcac cgtatcacct ggtggaacag tcacactcac ttgtggctcc    1320
   tcgactgggg ctgttacatc tggcaactac ccaaactggg tccaacaaaa accaggtcag    1380
   gcaccccgtg gtctaatagg tgggactaag ttcctcgccc ccggtactcc tgccagattc    1440
   tcaggctccc tgcttggagg caaggctgcc ctcaccctct caggggtaca gccagaggat    1500
   gaggcagaat attactgtgt tctatggtac agcaaccgct gggtgttcgg tggaggaacc    1560
   aaactgactg tccta                                                     1575
<210> 26
   <211> 525
   <212> PRT
   <213> artificial
<220>
   <223> SA21 x CEA x I2C H0
<400> 26
   Arg Leu Ile Glu Asp Ile Cys Leu Pro Arg Trp Gly Cys Leu Trp Glu
   1               5                   10                  15
   Asp Asp Gln Ala Val Leu Thr Gln Pro Ala Ser Leu Ser Ala Ser Pro
               20                  25                  30
   Gly Ala Ser Ala Ser Leu Thr Cys Thr Leu Arg Arg Gly Ile Asn Val
           35                  40                  45
   Gly Ala Tyr Ser Ile Tyr Trp Tyr Gln Gln Lys Pro Gly Ser Pro Pro
       50                  55                  60
   Gln Tyr Leu Leu Arg Tyr Lys Ser Asp Ser Asp Lys Gln Gln Gly Ser
   65                  70                  75                  80
   Gly Val Ser Ser Arg Phe Ser Ala Ser Lys Asp Ala Ser Ala Asn Ala
                   85                  90                  95
   Gly Ile Leu Leu Ile Ser Gly Leu Gln Ser Glu Asp Glu Ala Asp Tyr
               100                 105                 110
   Tyr Cys Met Ile Trp His Ser Gly Ala Ser Ala Val Phe Gly Gly Gly
           115                 120                 125
   Thr Lys Leu Thr Val Leu Gly Gly Gly Gly Ser Gly Gly Gly Gly Ser
       130                 135                 140
   Gly Gly Gly Gly Ser Glu Val Gln Leu Val Glu Ser Gly Gly Gly Leu
   145                 150                 155                 160
   Val Gln Pro Gly Arg Ser Leu Arg Leu Ser Cys Ala Ala Ser Gly Phe
                   165                 170                 175
   Thr Val Ser Ser Tyr Trp Met His Trp Val Arg Gln Ala Pro Gly Lys
               180                 185                 190
   Gly Leu Glu Trp Val Gly Phe Ile Arg Asn Lys Ala Asn Gly Gly Thr
           195                 200                 205
   Thr Glu Tyr Ala Ala Ser Val Lys Gly Arg Phe Thr Ile Ser Arg Asp
       210                 215                 220
   Asp Ser Lys Asn Thr Leu Tyr Leu Gln Met Asn Ser Leu Arg Ala Glu
   225                 230                 235                 240
   Asp Thr Ala Val Tyr Tyr Cys Ala Arg Asp Arg Gly Leu Arg Phe Tyr
                   245                 250                 255
   Phe Asp Tyr Trp Gly Gln Gly Thr Thr Val Thr Val Ser Ser Ser Gly
               260                 265                 270
   Gly Gly Gly Ser Glu Val Gln Leu Val Glu Ser Gly Gly Gly Leu Val
           275                 280                 285
   Gln Pro Gly Gly Ser Leu Lys Leu Ser Cys Ala Ala Ser Gly Phe Thr
       290                 295                 300
   Phe Asn Lys Tyr Ala Met Asn Trp Val Arg Gln Ala Pro Gly Lys Gly
   305                 310                 315                 320
   Leu Glu Trp Val Ala Arg Ile Arg Ser Lys Tyr Asn Asn Tyr Ala Thr
                   325                 330                 335
   Tyr Tyr Ala Asp Ser Val Lys Asp Arg Phe Thr Ile Ser Arg Asp Asp
               340                 345                 350
   Ser Lys Asn Thr Ala Tyr Leu Gln Met Asn Asn Leu Lys Thr Glu Asp
           355                 360                 365
   Thr Ala Val Tyr Tyr Cys Val Arg His Gly Asn Phe Gly Asn Ser Tyr
       370                 375                 380
   Ile Ser Tyr Trp Ala Tyr Trp Gly Gln Gly Thr Leu Val Thr Val Ser
   385                 390                 395                 400
   Ser Gly Gly Gly Gly Ser Gly Gly Gly Gly Ser Gly Gly Gly Gly Ser
                   405                 410                 415
   Gln Thr Val Val Thr Gln Glu Pro Ser Leu Thr Val Ser Pro Gly Gly
               420                 425                 430
   Thr Val Thr Leu Thr Cys Gly Ser Ser Thr Gly Ala Val Thr Ser Gly
           435                 440                 445
   Asn Tyr Pro Asn Trp Val Gln Gln Lys Pro Gly Gln Ala Pro Arg Gly
       450                 455                 460
   Leu Ile Gly Gly Thr Lys Phe Leu Ala Pro Gly Thr Pro Ala Arg Phe
   465                 470                 475                 480
   Ser Gly Ser Leu Leu Gly Gly Lys Ala Ala Leu Thr Leu Ser Gly Val
                   485                 490                 495
   Gln Pro Glu Asp Glu Ala Glu Tyr Tyr Cys Val Leu Trp Tyr Ser Asn
               500                 505                 510
   Arg Trp Val Phe Gly Gly Gly Thr Lys Leu Thr Val Leu
           515                 520                 525
<210> 27
   <211> 1575
   <212> DNA
   <213> artificial
<220>
   <223> CEA x I2C x SA21 HO
<400> 27
   caggccgtgc tgactcagcc ggcttccctc tctgcatctc ctggagcatc agccagtctc      60
   acctgcacct tgcgcagggg catcaatgtt ggtgcctaca gtatatactg gtaccagcag     120
   aagccaggga gtcctcccca gtatctcctg aggtacaaat cagactcaga taagcagcag     180
   ggctctggag tctccagccg cttctctgca tccaaagatg cttcggccaa tgcagggatt     240
   ttactcatct ctgggctcca gtctgaggat gaggctgact attactgtat gatttggcac     300
   agcggcgctt ctgcggtgtt cggcggaggg accaagttga ccgtcctagg tggtggtggt     360
   tctggcggcg gcggctccgg tggtggtggt tctgaggtgc agctggtcga gtctggggga     420
   ggcttggtcc agcctgggag gtccctgaga ctctcctgtg cagcgtctgg attcaccgtc     480
   agtagctact ggatgcactg ggtccgccaa gctccaggga aggggctgga atgggtaggt     540
   ttcattagaa acaaagctaa tggtgggaca acagaatacg ccgcgtctgt gaaaggcaga     600
   ttcaccatct caagagatga ttccaagaac acgctgtatc ttcaaatgaa cagcctgaga     660
   gccgaggaca cggccgtgta ttactgtgca agagataggg ggctacggtt ctactttgac     720
   tactggggcc aagggaccac ggtcaccgtc tcctcatccg gaggtggtgg ctccgaggtg     780
   cagctggtcg agtctggagg aggattggtg cagcctggag ggtcattgaa actctcatgt     840
   gcagcctctg gattcacctt caataagtac gccatgaact gggtccgcca ggctccagga     900
   aagggtttgg aatgggttgc tcgcataaga agtaaatata ataattatgc aacatattat     960
   gccgattcag tgaaagacag gttcaccatc tccagagatg attcaaaaaa cactgcctat    1020
   ctacaaatga acaacttgaa gactgaggac actgccgtgt actactgtgt gagacatggg    1080
   aacttcggta atagctacat atcctactgg gcttactggg gccaagggac tctggtcacc    1140
   gtctcctcag gtggtggtgg ttctggcggc ggcggctccg gtggtggtgg ttctcagact    1200
   gttgtgactc aggaaccttc actcaccgta tcacctggtg gaacagtcac actcacttgt    1260
   ggctcctcga ctggggctgt tacatctggc aactacccaa actgggtcca acaaaaacca    1320
   ggtcaggcac cccgtggtct aataggtggg actaagttcc tcgcccccgg tactcctgcc    1380
   agattctcag gctccctgct tggaggcaag gctgccctca ccctctcagg ggtacagcca    1440
   gaggatgagg cagaatatta ctgtgttcta tggtacagca accgctgggt gttcggtgga    1500
   ggaaccaaac tgactgtcct acggctgatc gaggacatct gcctgcccag atggggctgc    1560
   ctgtgggagg acgac                                                     1575
<210> 28
   <211> 525
   <212> PRT
   <213> artificial
<220>
   <223> CEA x I2C x SA21 H0
<400> 28
   Gln Ala Val Leu Thr Gln Pro Ala Ser Leu Ser Ala Ser Pro Gly Ala
   1               5                   10                  15
   Ser Ala Ser Leu Thr Cys Thr Leu Arg Arg Gly Ile Asn Val Gly Ala
               20                  25                  30
   Tyr Ser Ile Tyr Trp Tyr Gln Gln Lys Pro Gly Ser Pro Pro Gln Tyr
           35                  40                  45
   Leu Leu Arg Tyr Lys Ser Asp Ser Asp Lys Gln Gln Gly Ser Gly Val
       50                  55                  60
   Ser Ser Arg Phe Ser Ala Ser Lys Asp Ala Ser Ala Asn Ala Gly Ile
   65                  70                  75                  80
   Leu Leu Ile Ser Gly Leu Gln Ser Glu Asp Glu Ala Asp Tyr Tyr Cys
                   85                  90                  95
   Met Ile Trp His Ser Gly Ala Ser Ala Val Phe Gly Gly Gly Thr Lys
               100                 105                 110
   Leu Thr Val Leu Gly Gly Gly Gly Ser Gly Gly Gly Gly Ser Gly Gly
           115                 120                 125
   Gly Gly Ser Glu Val Gln Leu Val Glu Ser Gly Gly Gly Leu Val Gln
       130                 135                 140
   Pro Gly Arg Ser Leu Arg Leu Ser Cys Ala Ala Ser Gly Phe Thr Val
   145                 150                 155                 160
   Ser Ser Tyr Trp Met His Trp Val Arg Gln Ala Pro Gly Lys Gly Leu
                   165                 170                 175
   Glu Trp Val Gly Phe Ile Arg Asn Lys Ala Asn Gly Gly Thr Thr Glu
               180                 185                 190
   Tyr Ala Ala Ser Val Lys Gly Arg Phe Thr Ile Ser Arg Asp Asp Ser
           195                 200                 205
   Lys Asn Thr Leu Tyr Leu Gln Met Asn Ser Leu Arg Ala Glu Asp Thr
       210                 215                 220
   Ala Val Tyr Tyr Cys Ala Arg Asp Arg Gly Leu Arg Phe Tyr Phe Asp
   225                 230                 235                 240
   Tyr Trp Gly Gln Gly Thr Thr Val Thr Val Ser Ser Ser Gly Gly Gly
                   245                 250                 255
   Gly Ser Glu Val Gln Leu Val Glu Ser Gly Gly Gly Leu Val Gln Pro
               260                 265                 270
   Gly Gly Ser Leu Lys Leu Ser Cys Ala Ala Ser Gly Phe Thr Phe Asn
           275                 280                 285
   Lys Tyr Ala Met Asn Trp Val Arg Gln Ala Pro Gly Lys Gly Leu Glu
       290                 295                 300
   Trp Val Ala Arg Ile Arg Ser Lys Tyr Asn Asn Tyr Ala Thr Tyr Tyr
   305                 310                 315                 320
   Ala Asp Ser Val Lys Asp Arg Phe Thr Ile Ser Arg Asp Asp Ser Lys
   325 330 335
   Asn Thr Ala Tyr Leu Gln Met Asn Asn Leu Lys Thr Glu Asp Thr Ala
   340 345 350
   Val Tyr Tyr Cys Val Arg His Gly Asn Phe Gly Asn Ser Tyr Ile Ser
   355 360 365
   Tyr Trp Ala Tyr Trp Gly Gln Gly Thr Leu Val Thr Val Ser Ser Gly
   370 375 380
   Gly Gly Gly Ser Gly Gly Gly Gly Ser Gly Gly Gly Gly Ser Gln Thr
   385 390 395 400
   Val Val Thr Gln Glu Pro Ser Leu Thr Val Ser Pro Gly Gly Thr Val
   405 410 415
   Thr Leu Thr Cys Gly Ser Ser Thr Gly Ala Val Thr Ser Gly Asn Tyr
   420 425 430
   Pro Asn Trp Val Gln Gln Lys Pro Gly Gln Ala Pro Arg Gly Leu Ile
   435 440 445
   Gly Gly Thr Lys Phe Leu Ala Pro Gly Thr Pro Ala Arg Phe Ser Gly
   450 455 460
   Ser Leu Leu Gly Gly Lys Ala Ala Leu Thr Leu Ser Gly Val Gln Pro
   465 470 475 480
   Glu Asp Glu Ala Glu Tyr Tyr Cys Val Leu Trp Tyr Ser Asn Arg Trp
   485 490 495
   Val Phe Gly Gly Gly Thr Lys Leu Thr Val Leu Arg Leu Ile Glu Asp
   500 505 510
   Ile Cys Leu Pro Arg Trp Gly Cys Leu Trp Glu Asp Asp
   515 520 525
<210> 29
   <211> 1563
   <212> DNA
   <213> artificial
<220>
   <223> SA25 x CEA x I2C HO
<400> 29
   gaggacatct gcctgcccag atggggctgc ctgtgggagg accaggccgt gctgactcag 60
   ccggcttccc tctctgcatc tcctggagca tcagccagtc tcacctgcac cttgcgcagg 120
   ggcatcaatg ttggtgccta cagtatatac tggtaccagc agaagccagg gagtcctccc 180
   cagtatctcc tgaggtacaa atcagactca gataagcagc agggctctgg agtctccagc 240
   cgcttctctg catccaaaga tgcttcggcc aatgcaggga ttttactcat ctctgggctc 300
   cagtctgagg atgaggctga ctattactgt atgatttggc acagcggcgc ttctgcggtg 360
   ttcggcggag ggaccaagtt gaccgtccta ggtggtggtg gttctggcgg cggcggctcc 420
   ggtggtggtg gttctgaggt gcagctggtc gagtctgggg gaggcttggt ccagcctggg 480
   aggtccctga gactctcctg tgcagcgtct ggattcaccg tcagtagcta ctggatgcac 540
   tgggtccgcc aagctccagg gaaggggctg gaatgggtag gtttcattag aaacaaagct 600
   aatggtggga caacagaata cgccgcgtct gtgaaaggca gattcaccat ctcaagagat 660
   gattccaaga acacgctgta tcttcaaatg aacagcctga gagccgagga cacggccgtg 720
   tattactgtg caagagatag ggggctacgg ttctactttg actactgggg ccaagggacc 780
   acggtcaccg tctcctcatc cggaggtggt ggctccgagg tgcagctggt cgagtctgga 840
   ggaggattgg tgcagcctgg agggtcattg aaactctcat gtgcagcctc tggattcacc 900
   ttcaataagt acgccatgaa ctgggtccgc caggctccag gaaagggttt ggaatgggtt 960
   gctcgcataa gaagtaaata taataattat gcaacatatt atgccgattc agtgaaagac 1020
   aggttcacca tctccagaga tgattcaaaa aacactgcct atctacaaat gaacaacttg 1080
   aagactgagg acactgccgt gtactactgt gtgagacatg ggaacttcgg taatagctac 1140
   atatcctact gggcttactg gggccaaggg actctggtca ccgtctcctc aggtggtggt 1200
   ggttctggcg gcggcggctc cggtggtggt ggttctcaga ctgttgtgac tcaggaacct 1260
   tcactcaccg tatcacctgg tggaacagtc acactcactt gtggctcctc gactggggct 1320
   gttacatctg gcaactaccc aaactgggtc caacaaaaac caggtcaggc accccgtggt 1380
   ctaataggtg ggactaagtt cctcgccccc ggtactcctg ccagattctc aggctccctg 1440
   cttggaggca aggctgccct caccctctca ggggtacagc cagaggatga ggcagaatat 1500
   tactgtgttc tatggtacag caaccgctgg gtgttcggtg gaggaaccaa actgactgtc 1560
   cta 1563
<210> 30
   <211> 521
   <212> PRT
   <213> artificial
<220>
   <223> SA25 x CEA x I2C HO
<400> 30
   Glu Asp Ile Cys Leu Pro Arg Trp Gly Cys Leu Trp Glu Asp Gln Ala
   1               5                   10                  15
   Val Leu Thr Gln Pro Ala Ser Leu Ser Ala Ser Pro Gly Ala Ser Ala
               20                  25                  30
   Ser Leu Thr Cys Thr Leu Arg Arg Gly Ile Asn Val Gly Ala Tyr Ser
           35                  40                  45
   Ile Tyr Trp Tyr Gln Gln Lys Pro Gly Ser Pro Pro Gln Tyr Leu Leu
       50                  55                  60
   Arg Tyr Lys Ser Asp Ser Asp Lys Gln Gln Gly Ser Gly Val Ser Ser
   65                  70                  75                  80
   Arg Phe Ser Ala Ser Lys Asp Ala Ser Ala Asn Ala Gly Ile Leu Leu
                   85                  90                  95
   Ile Ser Gly Leu Gln Ser Glu Asp Glu Ala Asp Tyr Tyr Cys Met Ile
               100                 105                 110
   Trp His Ser Gly Ala Ser Ala Val Phe Gly Gly Gly Thr Lys Leu Thr
           115                 120                 125
   Val Leu Gly Gly Gly Gly Ser Gly Gly Gly Gly Ser Gly Gly Gly Gly
       130                 135                 140
   Ser Glu Val Gln Leu Val Glu Ser Gly Gly Gly Leu Val Gln Pro Gly
   145                 150                 155                 160
   Arg Ser Leu Arg Leu Ser Cys Ala Ala Ser Gly Phe Thr Val Ser Ser
                   165                 170                 175
   Tyr Trp Met His Trp Val Arg Gln Ala Pro Gly Lys Gly Leu Glu Trp
               180                 185                 190
   Val Gly Phe Ile Arg Asn Lys Ala Asn Gly Gly Thr Thr Glu Tyr Ala
           195                 200                 205
   Ala Ser Val Lys Gly Arg Phe Thr Ile Ser Arg Asp Asp Ser Lys Asn
       210                 215                 220
   Thr Leu Tyr Leu Gln Met Asn Ser Leu Arg Ala Glu Asp Thr Ala Val
   225                 230                 235                 240
   Tyr Tyr Cys Ala Arg Asp Arg Gly Leu Arg Phe Tyr Phe Asp Tyr Trp
   245 250 255
   Gly Gln Gly Thr Thr Val Thr Val Ser Ser Ser Gly Gly Gly Gly Ser
   260 265 270
   Glu Val Gln Leu Val Glu Ser Gly Gly Gly Leu Val Gln Pro Gly Gly
   275 280 285
   Ser Leu Lys Leu Ser Cys Ala Ala Ser Gly Phe Thr Phe Asn Lys Tyr
   290 295 300
   Ala Met Asn Trp Val Arg Gln Ala Pro Gly Lys Gly Leu Glu Trp Val
   305 310 315 320
   Ala Arg Ile Arg Ser Lys Tyr Asn Asn Tyr Ala Thr Tyr Tyr Ala Asp
   325 330 335
   Ser Val Lys Asp Arg Phe Thr Ile Ser Arg Asp Asp Ser Lys Asn Thr
   340 345 350
   Ala Tyr Leu Gln Met Asn Asn Leu Lys Thr Glu Asp Thr Ala Val Tyr
   355 360 365
   Tyr Cys Val Arg His Gly Asn Phe Gly Asn Ser Tyr Ile Ser Tyr Trp
   370 375 380
   Ala Tyr Trp Gly Gln Gly Thr Leu Val Thr Val Ser Ser Gly Gly Gly
   385 390 395 400
   Gly Ser Gly Gly Gly Gly Ser Gly Gly Gly Gly Ser Gln Thr Val Val
   405 410 415
   Thr Gln Glu Pro Ser Leu Thr Val Ser Pro Gly Gly Thr Val Thr Leu
   420 425 430
   Thr Cys Gly Ser Ser Thr Gly Ala Val Thr Ser Gly Asn Tyr Pro Asn
   435 440 445
   Trp Val Gln Gln Lys Pro Gly Gln Ala Pro Arg Gly Leu Ile Gly Gly
   450 455 460
   Thr Lys Phe Leu Ala Pro Gly Thr Pro Ala Arg Phe Ser Gly Ser Leu
   465 470 475 480
   Leu Gly Gly Lys Ala Ala Leu Thr Leu Ser Gly Val Gln Pro Glu Asp
   485 490 495
   Glu Ala Glu Tyr Tyr Cys Val Leu Trp Tyr Ser Asn Arg Trp Val Phe
   500 505 510
   Gly Gly Gly Thr Lys Leu Thr Val Leu
   515 520
<210> 31
   <211> 1563
   <212> DNA
   <213> artificial
<220>
   <223> CEA x I2C x SA25 HO
<400> 31
   caggccgtgc tgactcagcc ggcttccctc tctgcatctc ctggagcatc agccagtctc 60
   acctgcacct tgcgcagggg catcaatgtt ggtgcctaca gtatatactg gtaccagcag 120
   aagccaggga gtcctcccca gtatctcctg aggtacaaat cagactcaga taagcagcag 180
   ggctctggag tctccagccg cttctctgca tccaaagatg cttcggccaa tgcagggatt 240
   ttactcatct ctgggctcca gtctgaggat gaggctgact attactgtat gatttggcac 300
   agcggcgctt ctgcggtgtt cggcggaggg accaagttga ccgtcctagg tggtggtggt 360
   tctggcggcg gcggctccgg tggtggtggt tctgaggtgc agctggtcga gtctggggga 420
   ggcttggtcc agcctgggag gtccctgaga ctctcctgtg cagcgtctgg attcaccgtc 480
   agtagctact ggatgcactg ggtccgccaa gctccaggga aggggctgga atgggtaggt 540
   ttcattagaa acaaagctaa tggtgggaca acagaatacg ccgcgtctgt gaaaggcaga 600
   ttcaccatct caagagatga ttccaagaac acgctgtatc ttcaaatgaa cagcctgaga 660
   gccgaggaca cggccgtgta ttactgtgca agagataggg ggctacggtt ctactttgac 720
   tactggggcc aagggaccac ggtcaccgtc tcctcatccg gaggtggtgg ctccgaggtg 780
   cagctggtcg agtctggagg aggattggtg cagcctggag ggtcattgaa actctcatgt 840
   gcagcctctg gattcacctt caataagtac gccatgaact gggtccgcca ggctccagga 900
   aagggtttgg aatgggttgc tcgcataaga agtaaatata ataattatgc aacatattat 960
   gccgattcag tgaaagacag gttcaccatc tccagagatg attcaaaaaa cactgcctat 1020
   ctacaaatga acaacttgaa gactgaggac actgccgtgt actactgtgt gagacatggg 1080
   aacttcggta atagctacat atcctactgg gcttactggg gccaagggac tctggtcacc 1140
   gtctcctcag gtggtggtgg ttctggcggc ggcggctccg gtggtggtgg ttctcagact 1200
   gttgtgactc aggaaccttc actcaccgta tcacctggtg gaacagtcac actcacttgt 1260
   ggctcctcga ctggggctgt tacatctggc aactacccaa actgggtcca acaaaaacca 1320
   ggtcaggcac cccgtggtct aataggtggg actaagttcc tcgcccccgg tactcctgcc 1380
   agattctcag gctccctgct tggaggcaag gctgccctca ccctctcagg ggtacagcca 1440
   gaggatgagg cagaatatta ctgtgttcta tggtacagca accgctgggt gttcggtgga 1500
   ggaaccaaac tgactgtcct agaggacatc tgcctgccca gatggggctg cctgtgggag 1560
   gac 1563
<210> 32
   <211> 521
   <212> PRT
   <213> artificial
<220>
   <223> CEA x I2C x SA25 HO
<400> 32
   Gln Ala Val Leu Thr Gln Pro Ala Ser Leu Ser Ala Ser Pro Gly Ala
   1               5                   10                  15
   Ser Ala Ser Leu Thr Cys Thr Leu Arg Arg Gly Ile Asn Val Gly Ala
               20                  25                  30
   Tyr Ser Ile Tyr Trp Tyr Gln Gln Lys Pro Gly Ser Pro Pro Gln Tyr
           35                  40                  45
   Leu Leu Arg Tyr Lys Ser Asp Ser Asp Lys Gln Gln Gly Ser Gly Val
       50                  55                  60
   Ser Ser Arg Phe Ser Ala Ser Lys Asp Ala Ser Ala Asn Ala Gly Ile
   65                  70                  75                  80
   Leu Leu Ile Ser Gly Leu Gln Ser Glu Asp Glu Ala Asp Tyr Tyr Cys
                   85                  90                  95
   Met Ile Trp His Ser Gly Ala Ser Ala Val Phe Gly Gly Gly Thr Lys
               100                 105                 110
   Leu Thr Val Leu Gly Gly Gly Gly Ser Gly Gly Gly Gly Ser Gly Gly
           115                 120                 125
   Gly Gly Ser Glu Val Gln Leu Val Glu Ser Gly Gly Gly Leu Val Gln
       130                 135                 140
   Pro Gly Arg Ser Leu Arg Leu Ser Cys Ala Ala Ser Gly Phe Thr Val
   145                 150                 155                 160
   Ser Ser Tyr Trp Met His Trp Val Arg Gln Ala Pro Gly Lys Gly Leu
                   165                 170                 175
   Glu Trp Val Gly Phe Ile Arg Asn Lys Ala Asn Gly Gly Thr Thr Glu
               180                 185                 190
   Tyr Ala Ala Ser Val Lys Gly Arg Phe Thr Ile Ser Arg Asp Asp Ser
           195                 200                 205
   Lys Asn Thr Leu Tyr Leu Gln Met Asn Ser Leu Arg Ala Glu Asp Thr
       210                 215                 220
   Ala Val Tyr Tyr Cys Ala Arg Asp Arg Gly Leu Arg Phe Tyr Phe Asp
   225                 230                 235                 240
   Tyr Trp Gly Gln Gly Thr Thr Val Thr Val Ser Ser Ser Gly Gly Gly
                   245                 250                 255
   Gly Ser Glu Val Gln Leu Val Glu Ser Gly Gly Gly Leu Val Gln Pro
               260                 265                 270
   Gly Gly Ser Leu Lys Leu Ser Cys Ala Ala Ser Gly Phe Thr Phe Asn
           275                 280                 285
   Lys Tyr Ala Met Asn Trp Val Arg Gln Ala Pro Gly Lys Gly Leu Glu
       290                 295                 300
   Trp Val Ala Arg Ile Arg Ser Lys Tyr Asn Asn Tyr Ala Thr Tyr Tyr
   305                 310                 315                 320
   Ala Asp Ser Val Lys Asp Arg Phe Thr Ile Ser Arg Asp Asp Ser Lys
                   325                 330                 335
   Asn Thr Ala Tyr Leu Gln Met Asn Asn Leu Lys Thr Glu Asp Thr Ala
               340                 345                 350
   Val Tyr Tyr Cys Val Arg His Gly Asn Phe Gly Asn Ser Tyr Ile Ser
           355                 360                 365
   Tyr Trp Ala Tyr Trp Gly Gln Gly Thr Leu Val Thr Val Ser Ser Gly
       370                 375                 380
   Gly Gly Gly Ser Gly Gly Gly Gly Ser Gly Gly Gly Gly Ser Gln Thr
   385                 390                 395                 400
   Val Val Thr Gln Glu Pro Ser Leu Thr Val Ser Pro Gly Gly Thr Val
                   405                 410                 415
   Thr Leu Thr Cys Gly Ser Ser Thr Gly Ala Val Thr Ser Gly Asn Tyr
               420                 425                 430
   Pro Asn Trp Val Gln Gln Lys Pro Gly Gln Ala Pro Arg Gly Leu Ile
   435 440 445
   Gly Gly Thr Lys Phe Leu Ala Pro Gly Thr Pro Ala Arg Phe Ser Gly
   450 455 460
   Ser Leu Leu Gly Gly Lys Ala Ala Leu Thr Leu Ser Gly Val Gln Pro
   465 470 475 480
   Glu Asp Glu Ala Glu Tyr Tyr Cys Val Leu Trp Tyr Ser Asn Arg Trp
   485 490 495
   Val Phe Gly Gly Gly Thr Lys Leu Thr Val Leu Glu Asp Ile Cys Leu
   500 505 510
   Pro Arg Trp Gly Cys Leu Trp Glu Asp
   515 520
<210> 33
   <211> 1584
   <212> DNA
   <213> artificial
<220>
   <223> SA08 x CEA x I2C H0
<400> 33
   cagggcctga tcggcgacat ctgcctgccc agatggggct gcctgtgggg cgactccgtg      60
   aaacaggccg tgctgactca gccggcttcc ctctctgcat ctcctggagc atcagccagt     120
   ctcacctgca ccttgcgcag gggcatcaat gttggtgcct acagtatata ctggtaccag     180
   cagaagccag ggagtcctcc ccagtatctc ctgaggtaca aatcagactc agataagcag     240
   cagggctctg gagtctccag ccgcttctct gcatccaaag atgcttcggc caatgcaggg     300
   attttactca tctctgggct ccagtctgag gatgaggctg actattactg tatgatttgg     360
   cacagcggcg cttctgcggt gttcggcgga gggaccaagt tgaccgtcct aggtggtggt     420
   ggttctggcg gcggcggctc cggtggtggt ggttctgagg tgcagctggt cgagtctggg     480
   ggaggcttgg tccagcctgg gaggtccctg agactctcct gtgcagcgtc tggattcacc     540
   gtcagtagct actggatgca ctgggtccgc caagctccag ggaaggggct ggaatgggta     600
   ggtttcatta gaaacaaagc taatggtggg acaacagaat acgccgcgtc tgtgaaaggc     660
   agattcacca tctcaagaga tgattccaag aacacgctgt atcttcaaat gaacagcctg     720
   agagccgagg acacggccgt gtattactgt gcaagagata gggggctacg gttctacttt     780
   gactactggg gccaagggac cacggtcacc gtctcctcat ccggaggtgg tggctccgag     840
   gtgcagctgg tcgagtctgg aggaggattg gtgcagcctg gagggtcatt gaaactctca     900
   tgtgcagcct ctggattcac cttcaataag tacgccatga actgggtccg ccaggctcca     960
   ggaaagggtt tggaatgggt tgctcgcata agaagtaaat ataataatta tgcaacatat    1020
   tatgccgatt cagtgaaaga caggttcacc atctccagag atgattcaaa aaacactgcc    1080
   tatctacaaa tgaacaactt gaagactgag gacactgccg tgtactactg tgtgagacat    1140
   gggaacttcg gtaatagcta catatcctac tgggcttact ggggccaagg gactctggtc    1200
   accgtctcct caggtggtgg tggttctggc ggcggcggct ccggtggtgg tggttctcag    1260
   actgttgtga ctcaggaacc ttcactcacc gtatcacctg gtggaacagt cacactcact    1320
   tgtggctcct cgactggggc tgttacatct ggcaactacc caaactgggt ccaacaaaaa    1380
   ccaggtcagg caccccgtgg tctaataggt gggactaagt tcctcgcccc cggtactcct    1440
   gccagattct caggctccct gcttggaggc aaggctgccc tcaccctctc aggggtacag    1500
   ccagaggatg aggcagaata ttactgtgtt ctatggtaca gcaaccgctg ggtgttcggt    1560
   ggaggaacca aactgactgt ccta                                           1584
<210> 34
   <211> 528
   <212> PRT
   <213> artificial
<220>
   <223> SA08 x CEA x I2C H0
<400> 34
   Gln Gly Leu Ile Gly Asp Ile Cys Leu Pro Arg Trp Gly Cys Leu Trp
   1               5                   10                  15
   Gly Asp Ser Val Lys Gln Ala Val Leu Thr Gln Pro Ala Ser Leu Ser
               20                  25                  30
   Ala Ser Pro Gly Ala Ser Ala Ser Leu Thr Cys Thr Leu Arg Arg Gly
           35                  40                  45
   Ile Asn Val Gly Ala Tyr Ser Ile Tyr Trp Tyr Gln Gln Lys Pro Gly
       50                  55                  60
   Ser Pro Pro Gln Tyr Leu Leu Arg Tyr Lys Ser Asp Ser Asp Lys Gln
   65                  70                  75                  80
   Gln Gly Ser Gly Val Ser Ser Arg Phe Ser Ala Ser Lys Asp Ala Ser
                   85                  90                  95
   Ala Asn Ala Gly Ile Leu Leu Ile Ser Gly Leu Gln Ser Glu Asp Glu
               100                 105                 110
   Ala Asp Tyr Tyr Cys Met Ile Trp His Ser Gly Ala Ser Ala Val Phe
           115                 120                 125
   Gly Gly Gly Thr Lys Leu Thr Val Leu Gly Gly Gly Gly Ser Gly Gly
       130                 135                 140
   Gly Gly Ser Gly Gly Gly Gly Ser Glu Val Gln Leu Val Glu Ser Gly
   145                 150                 155                 160
   Gly Gly Leu Val Gln Pro Gly Arg Ser Leu Arg Leu Ser Cys Ala Ala
                   165                 170                 175
   Ser Gly Phe Thr Val Ser Ser Tyr Trp Met His Trp Val Arg Gln Ala
               180                 185                 190
   Pro Gly Lys Gly Leu Glu Trp Val Gly Phe Ile Arg Asn Lys Ala Asn
           195                 200                 205
   Gly Gly Thr Thr Glu Tyr Ala Ala Ser Val Lys Gly Arg Phe Thr Ile
       210                 215                 220
   Ser Arg Asp Asp Ser Lys Asn Thr Leu Tyr Leu Gln Met Asn Ser Leu
   225                 230                 235                 240
   Arg Ala Glu Asp Thr Ala Val Tyr Tyr Cys Ala Arg Asp Arg Gly Leu
                   245                 250                 255
   Arg Phe Tyr Phe Asp Tyr Trp Gly Gln Gly Thr Thr Val Thr Val Ser
               260                 265                 270
   Ser Ser Gly Gly Gly Gly Ser Glu Val Gln Leu Val Glu Ser Gly Gly
           275                 280                 285
   Gly Leu Val Gln Pro Gly Gly Ser Leu Lys Leu Ser Cys Ala Ala Ser
       290                 295                 300
   Gly Phe Thr Phe Asn Lys Tyr Ala Met Asn Trp Val Arg Gln Ala Pro
   305                 310                 315                 320
   Gly Lys Gly Leu Glu Trp Val Ala Arg Ile Arg Ser Lys Tyr Asn Asn
                   325                 330                 335
   Tyr Ala Thr Tyr Tyr Ala Asp Ser Val Lys Asp Arg Phe Thr Ile Ser
               340                 345                 350
   Arg Asp Asp Ser Lys Asn Thr Ala Tyr Leu Gln Met Asn Asn Leu Lys
           355                 360                 365
   Thr Glu Asp Thr Ala Val Tyr Tyr Cys Val Arg His Gly Asn Phe Gly
       370                 375                 380
   Asn Ser Tyr Ile Ser Tyr Trp Ala Tyr Trp Gly Gln Gly Thr Leu Val
   385                 390                 395                 400
   Thr Val Ser Ser Gly Gly Gly Gly Ser Gly Gly Gly Gly Ser Gly Gly
                   405                 410                 415
   Gly Gly Ser Gln Thr Val Val Thr Gln Glu Pro Ser Leu Thr Val Ser
               420                 425                 430
   Pro Gly Gly Thr Val Thr Leu Thr Cys Gly Ser Ser Thr Gly Ala Val
           435                 440                 445
   Thr Ser Gly Asn Tyr Pro Asn Trp Val Gln Gln Lys Pro Gly Gln Ala
       450                 455                 460
   Pro Arg Gly Leu Ile Gly Gly Thr Lys Phe Leu Ala Pro Gly Thr Pro
   465                 470                 475                 480
   Ala Arg Phe Ser Gly Ser Leu Leu Gly Gly Lys Ala Ala Leu Thr Leu
                   485                 490                 495
   Ser Gly Val Gln Pro Glu Asp Glu Ala Glu Tyr Tyr Cys Val Leu Trp
               500                 505                 510
   Tyr Ser Asn Arg Trp Val Phe Gly Gly Gly Thr Lys Leu Thr Val Leu
           515                 520                 525
<210> 35
   <211> 1584
   <212> DNA
   <213> artificial
<220>
   <223> CEA x I2C x SA08 HO
<400> 35
   caggccgtgc tgactcagcc ggcttccctc tctgcatctc ctggagcatc agccagtctc 60
   acctgcacct tgcgcagggg catcaatgtt ggtgcctaca gtatatactg gtaccagcag 120
   aagccaggga gtcctcccca gtatctcctg aggtacaaat cagactcaga taagcagcag 180
   ggctctggag tctccagccg cttctctgca tccaaagatg cttcggccaa tgcagggatt 240
   ttactcatct ctgggctcca gtctgaggat gaggctgact attactgtat gatttggcac 300
   agcggcgctt ctgcggtgtt cggcggaggg accaagttga ccgtcctagg tggtggtggt 360
   tctggcggcg gcggctccgg tggtggtggt tctgaggtgc agctggtcga gtctggggga      420
   ggcttggtcc agcctgggag gtccctgaga ctctcctgtg cagcgtctgg attcaccgtc      480
   agtagctact ggatgcactg ggtccgccaa gctccaggga aggggctgga atgggtaggt      540
   ttcattagaa acaaagctaa tggtgggaca acagaatacg ccgcgtctgt gaaaggcaga      600
   ttcaccatct caagagatga ttccaagaac acgctgtatc ttcaaatgaa cagcctgaga      660
   gccgaggaca cggccgtgta ttactgtgca agagataggg ggctacggtt ctactttgac      720
   tactggggcc aagggaccac ggtcaccgtc tcctcatccg gaggtggtgg ctccgaggtg      780
   cagctggtcg agtctggagg aggattggtg cagcctggag ggtcattgaa actctcatgt      840
   gcagcctctg gattcacctt caataagtac gccatgaact gggtccgcca ggctccagga      900
   aagggtttgg aatgggttgc tcgcataaga agtaaatata ataattatgc aacatattat      960
   gccgattcag tgaaagacag gttcaccatc tccagagatg attcaaaaaa cactgcctat     1020
   ctacaaatga acaacttgaa gactgaggac actgccgtgt actactgtgt gagacatggg     1080
   aacttcggta atagctacat atcctactgg gcttactggg gccaagggac tctggtcacc     1140
   gtctcctcag gtggtggtgg ttctggcggc ggcggctccg gtggtggtgg ttctcagact     1200
   gttgtgactc aggaaccttc actcaccgta tcacctggtg gaacagtcac actcacttgt     1260
   ggctcctcga ctggggctgt tacatctggc aactacccaa actgggtcca acaaaaacca     1320
   ggtcaggcac cccgtggtct aataggtggg actaagttcc tcgcccccgg tactcctgcc     1380
   agattctcag gctccctgct tggaggcaag gctgccctca ccctctcagg ggtacagcca     1440
   gaggatgagg cagaatatta ctgtgttcta tggtacagca accgctgggt gttcggtgga     1500
   ggaaccaaac tgactgtcct acagggcctg atcggcgaca tctgcctgcc cagatggggc     1560
   tgcctgtggg gcgactccgt gaaa                                             1584
<210> 36
   <211> 549
   <212> PRT
   <213> artificial
<220>
   <223> CEA x I2C x SA08 HO
<400> 36
   Gln Gly Leu Ile Gly Asp Ile Cys Leu Pro Arg Trp Gly Cys Leu Trp
   1 5 10 15
   Gly Asp Ser Val Lys Gln Ala Val Leu Thr Gln Pro Ala Ser Leu Ser
   20 25 30
   Ala Ser Pro Gly Ala Ser Ala Ser Leu Thr Cys Thr Leu Arg Arg Gly
   35 40 45
   Ile Asn Val Gly Ala Tyr Ser Ile Tyr Trp Tyr Gln Gln Lys Pro Gly
   50 55 60
   Ser Pro Pro Gln Tyr Leu Leu Arg Tyr Lys Ser Asp Ser Asp Lys Gln
   65 70 75 80
   Gln Gly Ser Gly Val Ser Ser Arg Phe Ser Ala Ser Lys Asp Ala Ser
   85 90 95
   Ala Asn Ala Gly Ile Leu Leu Ile Ser Gly Leu Gln Ser Glu Asp Glu
   100 105 110
   Ala Asp Tyr Tyr Cys Met Ile Trp His Ser Gly Ala Ser Ala Val Phe
   115 120 125
   Gly Gly Gly Thr Lys Leu Thr Val Leu Gly Gly Gly Gly Ser Gly Gly
   130 135 140
   Gly Gly Ser Gly Gly Gly Gly Ser Glu Val Gln Leu Val Glu Ser Gly
   145 150 155 160
   Gly Gly Leu Val Gln Pro Gly Arg Ser Leu Arg Leu Ser Cys Ala Ala
   165 170 175
   Ser Gly Phe Thr Val Ser Ser Tyr Trp Met His Trp Val Arg Gln Ala
   180 185 190
   Pro Gly Lys Gly Leu Glu Trp Val Gly Phe Ile Arg Asn Lys Ala Asn
   195 200 205
   Gly Gly Thr Thr Glu Tyr Ala Ala Ser Val Lys Gly Arg Phe Thr Ile
   210 215 220
   Ser Arg Asp Asp Ser Lys Asn Thr Leu Tyr Leu Gln Met Asn Ser Leu
   225 230 235 240
   Arg Ala Glu Asp Thr Ala Val Tyr Tyr Cys Ala Arg Asp Arg Gly Leu
   245 250 255
   Arg Phe Tyr Phe Asp Tyr Trp Gly Gln Gly Thr Thr Val Thr Val Ser
   260 265 270
   Ser Ser Gly Gly Gly Gly Ser Glu Val Gln Leu Val Glu Ser Gly Gly
   275 280 285
   Gly Leu Val Gln Pro Gly Gly Ser Leu Lys Leu Ser Cys Ala Ala Ser
   290 295 300
   Gly Phe Thr Phe Asn Lys Tyr Ala Met Asn Trp Val Arg Gln Ala Pro
   305 310 315 320
   Gly Lys Gly Leu Glu Trp Val Ala Arg Ile Arg Ser Lys Tyr Asn Asn
   325 330 335
   Tyr Ala Thr Tyr Tyr Ala Asp Ser Val Lys Asp Arg Phe Thr Ile Ser
   340 345 350
   Arg Asp Asp Ser Lys Asn Thr Ala Tyr Leu Gln Met Asn Asn Leu Lys
   355 360 365
   Thr Glu Asp Thr Ala Val Tyr Tyr Cys Val Arg His Gly Asn Phe Gly
   370 375 380
   Asn Ser Tyr Ile Ser Tyr Trp Ala Tyr Trp Gly Gln Gly Thr Leu Val
   385 390 395 400
   Thr Val Ser Ser Gly Gly Gly Gly Ser Gly Gly Gly Gly Ser Gly Gly
   405 410 415
   Gly Gly Ser Gln Thr Val Val Thr Gln Glu Pro Ser Leu Thr Val Ser
   420 425 430
   Pro Gly Gly Thr Val Thr Leu Thr Cys Gly Ser Ser Thr Gly Ala Val
   435 440 445
   Thr Ser Gly Asn Tyr Pro Asn Trp Val Gln Gln Lys Pro Gly Gln Ala
   450 455 460
   Pro Arg Gly Leu Ile Gly Gly Thr Lys Phe Leu Ala Pro Gly Thr Pro
   465 470 475 480
   Ala Arg Phe Ser Gly Ser Leu Leu Gly Gly Lys Ala Ala Leu Thr Leu
   485 490 495
   Ser Gly Val Gln Pro Glu Asp Glu Ala Glu Tyr Tyr Cys Val Leu Trp
   500 505 510
   Tyr Ser Asn Arg Trp Val Phe Gly Gly Gly Thr Lys Leu Thr Val Leu
   515 520 525
   Gln Gly Leu Ile Gly Asp Ile Cys Leu Pro Arg Trp Gly Cys Leu Trp
   530 535 540
   Gly Asp Ser Val Lys
   545
<210> 37
   <211> 5
   <212> PRT
   <213> artificial
<220>
   <223> N-terminus of human CD3
<400> 37
   Gln Asp Gly Asn Glu
   1 5